# EUROPEAN PATENT APPLICATION

(11) **EP 4 245 757 A1**
(43) Date of publication of application: **20.09.2023**
(21) Application number: 21891258.2
(22) Date of filing: 15.11.2021
(51) Int. Cl.: C07D 471/04, A61K 31/437, A61P 35/00

(54) **CRYSTAL FORM OF FREE BASE OF INHIBITOR CONTAINING BICYCLIC RING DERIVATIVE AND PREPARATION METHOD AND APPLICATION OF CRYSTAL FORM**

(30) Priority: 13.11.2020 CN 202011271725; 11.11.2021 CN 202111333586
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: DONG, Hua, Shanghai 201203 (CN); YANG, Long, Shanghai 201203 (CN); GUO, Linsong, Shanghai 201203 (CN)
(74) Representative: Hoffmann Eitle
(86) International application number: PCT/CN2021/130725
(87) International publication number: WO 2022/100738

(57) **Abstract**

The present invention relates to a crystal form of a free base of an inhibitor containing a bicyclic ring derivative and a preparation method and an application of the crystal form, and in particular to a crystal form of a compound represented by general formula (I), a preparation method and a pharmaceutical composition containing a therapeutically effective amount of the compound crystal form, and an application of the crystal form used as an RET inhibitor in treatment of diseases such as cancer, inflammation, chronic liver diseases, diabetes, cardiovascular diseases and AIDS.

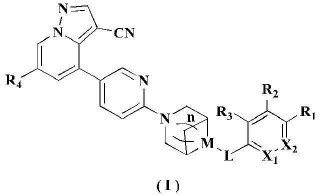

## Description

This application claims the priorities of Chinese patent application 2020112717256 filed on November 13, 2020 and Chinese patent application 202111333586 filed on November 11, 2021. The contents of the Chinese patent applications are incorporated herein by reference in their entireties.

### TECHNICAL FIELD

The present disclosure belongs to the field of medical biology, specifically related to a crystal form of a free base of an inhibitor containing a bicyclic ring derivative and a preparation method and an application of the crystal form.

### BACKGROUND

RET (rearranged during transfection) protein is encoded by the proto-oncogene RET located on chromosome 10, and is a receptor tyrosine kinase that consists of an extracellular domain, a transmembrane domain and an intracellular kinase domain. RET ligands are glial-cell-line derived neurotrophic factor (GDNF) family ligands (GFLs) such as GDNF, neuroturin (NRTN), artemin (ARTN), persephin (PSPN), and the activation of the receptor also requires the combined effect of the co-receptor GFRα family, GFLs and GFRα form a dimer binding to RET and recruiting it to the cholesterol-rich membrane region. The RET protein undergoes dimerization and autophosphorylation, thereby activating downstream RAS-MAPK and PI3K-AKT, PKC and other signal pathways. RET plays an important role in the development of the kidney and enteric nervous system during embryonic development; it is also important for the homeostasis of neuroendocrine, hematopoietic and male germ cells and other tissues.

The disorder of RET protein function has led to the occurrence of many diseases. The lack of RET protein function during developmental processes can lead to a series of congenital diseases such as Hirschsprung disease (HSCR), congenital kidney and urinary tract malformations (CAKUT), etc. The activating mutations of RET protein, including point mutations and RET protein fusion caused by chromosome rearrangement, are also related to the occurrence of many diseases. RET fusion mainly occurs in 1 to 2% of non-small cell lung cancer (NSCLC) patients and 5 to 10% of papillary thyroid carcinoma, while RET mutations mainly occur in 60% of medullary thyroid carcinoma, and the activating mutations of RET protein are found in many other tumors such as breast cancer, gastric cancer, bowel cancer, and chronic bone marrow myelomonocytic leukemia.

Although there is a large clinical need, the current treatment for RET targets is still extremely limited, unlike ALK, EGFR and other targeted drugs that have achieved excellent efficacy in the clinic, there are still no approved targeted drugs for RET targets. At present, multi-kinase inhibitors (MKI) such as vandetinib and cabozantinib are mostly used in clinical drugs, these multi-kinase inhibitors have the disadvantages of high side effects and poor efficacy due to poor selectivity, and they cannot overcome the drug resistance problems that occur during treatment.

The demand for RET targeted drugs has attracted a large number of domestic and foreign pharmaceutical companies to develop RET specific targeted drugs, wherein the more prominent ones are Loxo Oncology's LOXO-292, which has entered clinical phase VII, and Blueprint's BLU-667, which has also entered clinical phase I. Both of these targeted drugs have shown very good efficacy and safety in preclinical trials for patients with RET activating mutations, as well as overcoming possible drug resistance mutations in preclinical activity screening, which is expected to bring more treatment options for cancers with RET activating mutations in the future.

There are currently no specific targeted drugs for RET targets, and there is a large clinical demand. RET inhibitors with higher selectivity, better activity, better safety, and the ability to overcome drug-resistant mutations have the potential to treat a variety of cancers and have broad market prospects.

The PCT patent application (application No.: PCT/CN2020/090142) of Jiangsu Hansoh Pharmaceutical Group Co., Ltd. disclosed structures of a series of inhibitor containing bicyclic derivative. In the subsequent research and development, in order to simplify the process preparation method, improve the stability and bioavailability, etc., the present disclosure has carried out a comprehensive study on the free base of the above substances, and is committed to obtaining a stable crystal form suitable for later drug development with simple operation, easy storage and high bioavailability.

### CONTENT OF THE PRESENT INVENTION

All the contents involved in the patent application PCT/CN2020/090142 are incorporated in the present disclosure by citation.

The object of the present disclosure is to provide a crystal form of a compound represented by general formula (I): 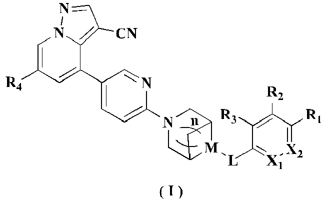 wherein:
L is selected from -CH₂- or -NHC(O)-;
M is selected from CR or N;
R is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
X₁ is selected from CRₐ or N;
X₂ is selected from CR_{b} or N;
Rₐ and R_{b} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
R₁, R₂ and R₃ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
R₄ is selected from C₂₋₆ alkenyl, C₂₋₆ alkynyl, 3-8-membered heterocyclyl, - O(CH₂)ₘR₅, -(CH₂)ₘR₅, -NR₆(CH₂)ₘR₅, -NR₆C(O)R₅, -CH=CH-(CH₂)ₘR₅, -O(CH₂)ₘS(O)R₅, -O(CH₂)ₘS(O)₂Rs or -O(CH₂)ₘS(O)(=NH)R₅, and the C₂₋₆ alkenyl, the C₂₋₆ alkynyl, and the 3-8-membered heterocyclyl are optionally further substituted by one or more substituents selected from deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-12-membered heterocyclyl, C₆₋₁₀ aryl and 5-12-membered heteroaryl;
R₅ and R₆ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl, and the C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl are optionally further substituted by one or more substituents selected from deuterium, halogen, amino, nitro, hydroxyl, cyano, oxo, thio, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl, 5-12-membered heteroaryl, =NH, -(CH₂)ₜR₇, -(CH₂)ₜOR₇ and -(CH₂)ₜC(O)NR₇R₈;
R₇ and R₈ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl, and the C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl are optionally further substituted by one or more substituents selected from deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl;
n is 0, 1, 2 or 3;
m is 0, 1, 2 or 3; and
t is 0, 1, 2 or 3.

In a further preferred embodiment of the present disclosure, M is selected from CR or N;
R is selected from hydrogen, amino or C₁₋₃ alkyl;
preferably hydrogen, amino or methyl;
X₁ is selected from CRₐ or N;
X₂ is selected from CR_{b} or N;
Rₐ and R_{b} are each independently selected from hydrogen, deuterium, halogen, C₁₋₃ alkyl or C₁₋₃ alkoxy;
preferably hydrogen, fluorine, chlorine, methyl or methoxy;
R₁, R₂ and R₃ are each independently selected from hydrogen, deuterium, halogen, C₁₋₃ alkyl or C₁₋₃ alkoxy;
preferably hydrogen, fluorine, chlorine, methyl or methoxy.

In a further preferred embodiment of the present disclosure, R₄ is selected from C₂₋₃ alkynyl, C₂₋₃ alkenyl, 3-8-membered heterocyclyl containing a nitrogen or oxygen atom, - O(CH₂)ₘR₅, -(CH₂)ₘR₅, -NR₆(CH₂)ₘR₅, -NHC(O)R₅, -CH=CH-(CH₂)ₘR₅, -O(CH₂)ₘS(O)R₅, - O(CH₂)ₘS(O)₂R₅ or -O(CH₂)ₘS(O)(=NH)R₅, and the 3-8-membered heterocyclyl containing the nitrogen or oxygen atom is optionally further substituted by one or more substituents selected from deuterium, fluorine, chlorine, bromine, amino, hydroxyl, cyano, C₁₋₃ alkyl, C₁₋₃ hydroxyalkyl or C₁₋₃ alkoxy;
preferably vinyl, ethynyl, oxetanyl, azetidinyl, pyrrolidinyl, piperidinyl, 2-azaspiro[3.3]heptanyl, -OCH₂R₅, -O(CH₂)₂R₅, -(CH₂)₂R₅, -NR₆(CH₂)ₘR₅, -NHC(O)R₅, - CH=CH-(CH₂)ₘR₅, -O(CH₂)ₘS(O)R₅, -O(CH₂)ₘS(O)₂R₅ or -O(CH₂)ₘS(O)(=NH)R₅, and the vinyl, ethynyl, oxetanyl, azetidinyl, pyrrolidinyl, piperidinyl and 2-azaspiro[3.3]heptanyl are optionally further substituted by one or more substituents selected from deuterium, hydroxyl, cyano, methyl, hydroxyethyl, 2-hydroxyisopropyl, 2-aminoisopropyl;
R₅ is selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₆ cycloalkyl or 3-8-membered heterocyclyl, and the C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₃ cycloalkyl and 3-8-membered heterocyclyl are optionally further substituted by one or more substituents selected from deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₆ cycloalkyl, 3-8-membered heterocyclyl, -CH₂R₇, -CH₂OR₇ and -C(O)NR₇R₈;
preferably methyl, ethyl, isopropyl, cyclopropyl, cyclobutyl, cyclopentyl, ethynyl, oxetanyl, thietanyl, azetidinyl, tetrahydropyranyl, bicyclo[1.1.1]pentanyl or tetrahydro-2*H-*thiopyran, and the methyl, ethyl, isopropyl, cyclopropyl, cyclobutyl, cyclopentyl, oxetanyl, thietanyl, azetidinyl, tetrahydropyranyl, bicyclo[1.1.1]pentanyl and tetrahydro-2H-thiopyran are optionally further substituted by one or more substituents selected from deuterium, hydroxyl, amino, cyano, fluorine, chlorine, bromine, methoxy, ethynyl, cyclopropyl, hydroxyethyl, oxo, =NH, -CH₂OCH₃, -C(O)NH₂ and -C(O)NHCH₃;
R₆ is selected from hydrogen, deuterium, halogen, amino, hydroxyl, or C₁₋₃ alkyl;
preferably hydrogen, deuterium or methyl;
R₇ and R₈ are each independently selected from hydrogen, deuterium, halogen, amino, hydroxyl, or C₁₋₃ alkyl;
preferably hydrogen, deuterium or methyl;
n is 0, 1, 2 or 3; and
m is 0, 1, 2 or 3.

In a further preferred embodiment of the present disclosure, the compound is further represented by general formula (II): 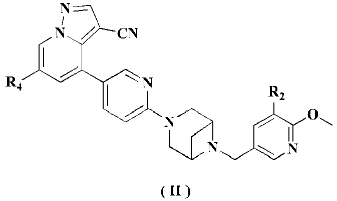
R₂ is selected from hydrogen, fluorine or chlorine;
R₄ is selected from -O(CH₂)ₘR₅, -(CH₂)ₘR₅ or -NR₆(CH₂)ₘR₅,
R₅ is selected from C₂₋₃ alkynyl, optionally further substituted by one or more substituents selected from deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl or C₃₋₆ cycloalkyl.

In a further preferred embodiment of the present disclosure, the compound is 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*] pyridine-3-carbonitrile, wherein the crystal formis a crystal form I, a crystal form II, a crystal form III, a crystal form IV or a crystal form V

Most preferably, in a further preferred embodiment of the present disclosure, the crystal form I of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63.

The crystal form I has an X-ray powder diffraction pattern having a diffraction peak at 2θ angle of 5.0±0.2°; or having a diffraction peak at 9.6±0.2°; or having a diffraction peak at 15.0±0.2°; or having a diffraction peak at 17.2±0.2°; or having a diffraction peak at 22.2±0.2°; or having a diffraction peak at 19.4±0.2°; or having a diffraction peak at 30.2±0.2°; or having a diffraction peak at 8.2±0.2°; or having a diffraction peak at 25.1±0.2°; or having a diffraction peak at 26.9±0.2°; preferably comprising any 2-5, or 3-5, or 3-6, or 3-8, or 5-8, or 6-8 of the above diffraction peaks; more preferably comprising any 6, 7, or 8 of the diffraction peaks,
the X-ray powder diffraction pattern of the crystal form I has a diffraction peak at 2θ angle of 5.0±0.2°; or has a diffraction peak at 8.2±0.2°; or has a diffraction peak at 9.6±0.2°; or has a diffraction peak at 12.9±0.2°; or has a diffraction peak at 15.0±0.2°; or has a diffraction peak at 17.2±0.2°; or has a diffraction peak at 15.4±0.2°; or has a diffraction peak at 16.6±0.2°; or has a diffraction peak at 17.7±0.2°; or has a diffraction peak at 18.5±0.2°; preferably comprises any 2-5, or 3-5, or 3-6, or 3-8, or 5-8, or 6-8 of the above diffraction peaks; more preferably comprises any 6, 7, or 8 of the diffraction peaks;
as a specific example, the X-ray powder diffraction pattern of the crystal form I comprises at least one or more diffraction peaks at 2θ angles of 5.0±0.2°, 9.6±0.2°, 15.0±0.2°, preferably comprises two of the above diffraction peaks, more preferably comprises three of the diffraction peaks; optionally, further comprises at least one diffraction peak at 2θ angle of 17.2±0.2°, 22.2±0.2°, 19.4±0.2°, 30.2±0.2° or 8.2±0.2°, preferably comprises 2, 3, 4 or 5 of the above diffraction peaks;
comprises diffraction peaks at 2θ angles of 5.0±0.2°, 9.6±0.2°, 15.0±0.2°, 17.2±0.2°, 22.2±0.2° and 19.4±0.2°;
or, comprises diffraction peaks at 2θ angles of 5.0±0.2°, 9.6±0.2°, 15.0±0.2°, 17.2±0.2°, 22.2±0.2° and 8.2±0.2°;
or, comprises diffraction peaks at 2θ angles of 5.0±0.2°, 9.6±0.2°, 15.0±0.2°, 17.2±0.2°, 22.2±0.2°, 19.4±0.2° and 8.2±0.2°;
the X-ray powder diffraction pattern of the crystal form I comprises at least one or more diffraction peaks at 2θ angles of 5.0±0.2°, 8.2±0.2°, 9.6±0.2°, preferably comprises two of the above diffraction peaks, more preferably comprises three of the diffraction peaks; optionally, further comprises at least one diffraction peak at 2θ angle of 12.9±0.2°, 15.0±0.2°, 17.2±0.2°, 15.4±0.2° or 16.6±0.2°, preferably comprises 2, 3, 4 or 5 of the above diffraction peaks;
comprises diffraction peaks at 2θ angles of 5.0±0.2°, 8.2±0.2°, 9.6±0.2°, 12.9±0.2°, 15.0±0.2° and 17.2±0.2°;
or, comprises diffraction peaks at 2θ angles of 8.2±0.2°, 9.6±0.2°, 12.9±0.2°, 15.0±0.2°, 17.2±0.2° and 15.4±0.2°;
or, comprises diffraction peaks at 2θ angles of 9.6±0.2°, 12.9±0.2°, 15.0±0.2°, 17.2±0.2°, 15.4±0.2° and 16.6±0.2°;
or, comprises diffraction peaks at 2θ angles of 5.0±0.2°, 9.6±0.2°, 12.9±0.2°, 15.0±0.2°, 17.2±0.2° and 15.4±0.2°;
or, comprises diffraction peaks at 2θ angles of 8.2±0.2°, 12.9±0.2°, 15.0±0.2°, 17.2±0.2°, 15.4±0.2° and 16.6±0.2°;
preferably, the X-ray powder diffraction pattern of the crystal form I optionally also comprises one or more diffraction peaks at 2θ angles of 10.0±0.2°, 25.4±0.2°, 26.9±0.2°, 13.0±0.2°, 12.9±0.2° or 20.0±0.2°; preferably comprises at least any 2-3, or 4-5, or 6-7 of the above diffraction peaks; more preferably comprises any 2, 3, 4, 5, 6 or 7 of the diffraction peaks;
comprises diffraction peaks at 2θ angles of 5.0±0.2°, 9.6±0.2°, 15.0±0.2°, 17.2±0.2°, 22.2±0.2°, 19.4±0.2°, 26.9±0.2° and 10.0±0.2°;
or, comprises diffraction peaks at 2θ angles of 5.0±0.2°, 9.6±0.2°, 15.0±0.2°, 17.2±0.2°, 22.2±0.2°, 19.4±0.2°, 26.9±0.2° and 25.4±0.2°,
preferably, the X-ray powder diffraction pattern of the crystal form I optionally also comprises one or more diffraction peaks at 2θ angles of 15.4±0.2°, 16.6±0.2°, 17.7±0.2°, 18.5±0.2°, 19.3±0.2° or 24.0±0.2°; preferably comprises at least any 2-3, or 4-5, or 6 of the above diffraction peaks; further preferably comprises any 2, 3, 4, 5 or 6 of the diffraction peaks;
comprises diffraction peaks at 2θ angles of 5.0±0.2°, 8.2±0.2°, 9.6±0.2°, 15.4±0.2°, 16.6±0.2°, 17.7±0.2°, 18.5±0.2° and 19.3±0.2°;
or, comprises diffraction peaks at 2θ angles of 5.0±0.2°, 8.2±0.2°, 9.6±0.2°, 16.6±0.2°, 17.7±0.2°, 18.5±0.2°, 19.3±0.2° and 24.0±0.2°;
further preferably, the X-ray powder diffraction pattern of the crystal form I comprises one or more diffraction peaks at 2θ angles of 5.0±0.2°, 9.6±0.2°, 15.0±0.2°, 17.2±0.2°, 22.2±0.2°, 19.4±0.2°, 30.2±0.2°, 25.1±0.2°, 10.0±0.2°, 25.1±0.2°, 25.4±0.2°, 26.9±0.2°, 13.0±0.2°, 12.9±0.2° or 20.0±0.2°; preferably, comprises any 4, 5, 6, 8 or 10 of the above diffraction peaks;
the X-ray powder diffraction pattern of the crystal form I has diffraction peaks at following 2θ angles of:
   5.0±0.2°, 9.6±0.2°, 15.0±0.2° and 17.2±0.2°;
   or, 5.0±0.2°, 9.6±0.2°, 15.0±0.2° and 22.2±0.2°;
   or, 5.0±0.2°, 9.6±0.2°, 17.2±0.2° and 22.2±0.2°;
   or, 5.0±0.2°, 15.0±0.2°, 17.2±0.2° and 22.2±0.2°;
   or, 9.6±0.2°, 15.0±0.2°, 17.2±0.2° and 22.2±0.2°;
   or, 5.0±0.2°, 9.6±0.2°, 15.0±0.2°, 17.2±0.2°, 22.2±0.2° and 25.1±0.2°;
   or, 8.2±0.2°, 9.6±0.2°, 15.0±0.2°, 17.2±0.2°, 19.4±0.2°, 22.2±0.2°, 25.1±0.2° and 25.4±0.2°;
   or, 5.0±0.2°, 10.0±0.2°, 15.0±0.2°, 17.2±0.2°, 19.4±0.2°, 22.2±0.2°, 25.1±0.2° and 25.4±0.2°;
further preferably, the X-ray powder diffraction pattern of the crystal form I comprises one or more diffraction peaks at 2θ angles of 5.0±0.2°, 8.2±0.2°, 9.6±0.2°, 12.9±0.2°, 15.0±0.2°, 17.2±0.2°, 15.4±0.2°, 16.6±0.2°, 17.7±0.2°, 18.5±0.2°, 19.3±0.2° or 24.0±0.2°; preferably, comprises any 4, 5, 6, 8 or 10 of the above diffraction peaks;
the X-ray powder diffraction pattern of the crystal form I has diffraction peaks at following 2θ angles of:
   5.0±0.2°, 8.2±0.2° and 9.6±0.2°;
   or, 5.0±0.2°, 8.2±0.2°, 9.6±0.2° and 12.9±0.2°;
   or, 8.2±0.2°, 9.6±0.2°, 12.9±0.2° and 15.0±0.2°;
   or, 9.6±0.2°, 12.9±0.2°, 15.0±0.2° and 17.2±0.2°;
   or, 12.9±0.2°, 15.0±0.2°, 17.2±0.2° and 15.4±0.2°;
   or, 15.0±0.2°, 17.2±0.2°, 15.4±0.2° and 16.6±0.2°;
   or, 5.0±0.2°, 8.2±0.2°, 9.6±0.2°, 12.9±0.2°, 15.0±0.2 and 17.2±0.2°;
   or, 8.2±0.2°, 9.6±0.2°, 12.9±0.2°, 15.0±0.2°, 17.2±0.2° and 15.4±0.2°;
   or, 5.0±0.2°, 8.2±0.2°, 9.6±0.2°, 12.9±0.2°, 15.0±0.2°, 17.2±0.2°, 15.4±0.2° and 16.6±0.2°;
   or, 8.2±0.2°, 9.6±0.2°, 12.9±0.2°, 15.0±0.2°, 17.2±0.2°, 15.4±0.2°, 16.6±0.2° and 17.7±0.2°;
   or, 5.0±0.2°, 8.2±0.2°, 9.6±0.2°, 12.9±0.2°, 15.0±0.2°, 17.2±0.2°, 15.4±0.2°, 16.6±0.2°, 17.7±0.2° and 18.5±0.2°;
   or, 8.2±0.2°, 9.6±0.2°, 12.9±0.2°, 15.0±0.2°, 17.2±0.2°, 15.4±0.2°, 16.6±0.2°, 17.7±0.2°, 18.5±0.2° and 19.3±0.2°;
   using Cu-Kα radiation, the X-ray characteristic diffraction peaks represented by 2θ angles and interplanar spacing d values are shown in Table 1.

**Table 1**

| No. | XRPD X-ray diffraction data of the crystal form I of the compound of embodiment 63 | | | | | |
|---|---|---|---|---|---|---|
| | 2θ (±0.2°) | d value | Peak height | Proportion (I%) | Area | Proportion (I%) |
| 1 | 4.997 | 17.6685 | 30424 | 100 | 115645 | 100 |
| 2 | 8.218 | 10.7493 | 598 | 2 | 2298 | 2 |
| 3 | 9.574 | 9.2298 | 4345 | 14.3 | 17682 | 15.3 |
| 4 | 9.987 | 8.8495 | 433 | 1.4 | 2548 | 2.2 |
| 5 | 12.879 | 6.8683 | 313 | 1 | 1618 | 1.4 |
| 6 | 13.007 | 6.8009 | 320 | 1.1 | 1506 | 1.3 |
| 7 | 14.997 | 5.9027 | 3532 | 11.6 | 14167 | 12.3 |
| 8 | 17.201 | 5.1508 | 1921 | 6.3 | 13121 | 11.3 |
| 9 | 19.373 | 4.578 | 1575 | 5.2 | 7520 | 6.5 |
| 10 | 20.038 | 4.4275 | 312 | 1 | 1331 | 1.2 |
| 11 | 22.212 | 3.9989 | 1682 | 5.5 | 8985 | 7.8 |
| 12 | 25.109 | 3.5436 | 574 | 1.9 | 2876 | 2.5 |
| 13 | 25.352 | 3.5103 | 411 | 1.4 | 2286 | 2 |
| 14 | 26.874 | 3.3147 | 514 | 1.7 | 2428 | 2.1 |
| 15 | 30.236 | 2.9534 | 677 | 2.2 | 2993 | 2.6 |

The crystal form I of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63 of the present disclosure has an X-ray powder diffraction pattern basically as shown in FIG. 1; a DSC pattern basically as shown in FIG. 2; where the crystal form I has a melting point of 216.8 to 218.4°C; and a TGA pattern basically as shown in FIG. 3.

In a further preferred embodiment of the present disclosure, the crystal form II of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63.

The crystal form II has an X-ray powder diffraction pattern comprising at least one or more diffraction peaks at 2θ angles of 4.8±0.2°, 17.7±0.2°, 16.6±0.2°, preferably comprising two of the above diffraction peaks, more preferably comprising three of the diffraction peaks; optionally, further comprising at least one diffraction peak at 2θ angle of 9.4±0.2°, 18.4±0.2°, 19.1±0.2°, 17.0±0.2° or 18.6±0.2°, preferably comprising 2, 3, 4 or 5 of the above diffraction peaks;
or, comprising diffraction peaks at 2θ angles of 4.8±0.2°, 17.7±0.2°, 16.6±0.2°, 9.4±0.2°, 18.4±0.2° and 19.1±0.2°;
or, comprising diffraction peaks at 2θ angles of 4.8±0.2°, 17.7±0.2°, 16.6±0.2°, 9.4±0.2°, 18.4±0.2° and 17.0±0.2°;
or, comprising diffraction peaks at 2θ angles of 4.8±0.2°, 17.7±0.2°, 16.6±0.2°, 9.4±0.2°, 18.4±0.2° and 18.6±0.2°;
or, comprising diffraction peaks at 2θ angles of 4.8±0.2°, 17.7±0.2°, 9.4±0.2°, 18.4±0.2°, 19.1±0.2° and 17.0±0.2°;
or, comprising diffraction peaks at 2θ angles of 4.8±0.2°, 16.6±0.2°, 9.4±0.2°, 18.4±0.2°, 19.1±0.2° and 17.0±0.2°;
preferably, the X-ray powder diffraction pattern of the crystal form II optionally also comprises at least one or more diffraction peaks at 2θ angles of 4.5±0.2°, 15.4±0.2°, 14.6±0.2°, 24.0±0.2°, 21.5±0.2°, 20.5±0.2° or 18.0±0.2°; preferably comprises at least any 2-3, or 4-5, or 6-7 of the above diffraction peaks; further preferably, comprises any 2, 3, 4, 5, 6, 7 of the diffraction peaks;
comprises diffraction peaks at 2θ angles of 4.8±0.2°, 17.7±0.2°, 16.6±0.2°, 9.4±0.2°, 18.4±0.2°, 19.1±0.2°, 4.5±0.2° and 15.4±0.2°;
or, comprises diffraction peaks at 2θ angles of 4.8±0.2°, 17.7±0.2°, 16.6±0.2°, 9.4±0.2°, 18.4±0.2°, 19.1±0.2°, 4.5±0.2° and 14.6±0.2°;
further preferably, the X-ray powder diffraction pattern of the crystal form II comprises one or more diffraction peaks at 2θ angles of 4.8±0.2°, 17.7±0.2°, 16.6±0.2°, 9.4±0.2°, 18.4±0.2°, 19.1±0.2°, 17.0±0.2°, 18.6±0.2°, 4.5±0.2°, 15.4±0.2°, 14.6±0.2°, 24.0±0.2°, 21.5±0.2°, 20.5±0.2° or 18.0±0.2°; preferably, comprises any 4, 5, 6, 8 or 10 of the above diffraction peaks;
the X-ray powder diffraction pattern of the crystal form II has diffraction peaks at following 2θ angles of:
   4.8±0.2°, 17.7±0.2°, 16.6±0.2° and 9.4±0.2°;
   4.8±0.2°, 17.7±0.2°, 16.6±0.2° and 18.4±0.2°;
   4.8±0.2°, 17.7±0.2°, 16.6±0.2°, 9.4±0.2°, 18.4±0.2° and 20.5±0.2°;
   4.8±0.2°, 17.7±0.2°, 16.6±0.2°, 9.4±0.2°, 18.4±0.2°, 19.1±0.2°, 17.0±0.2° and 18.6±0.2°;
   4.8±0.2°, 17.7±0.2°, 16.6±0.2°, 9.4±0.2°, 18.4±0.2°, 19.1±0.2°, 17.0±0.2°, 18.6±0.2°, 4.5±0.2°, 14.6±0.2°;
   further preferably, the X-ray powder diffraction pattern of the crystal form II comprises one or more diffraction peaks at 2θ angles of 4.8±0.2°, 17.7±0.2°, 16.6±0.2°, 9.4±0.2°, 18.4±0.2°, 19.1±0.2°, 17.0±0.2°, 18.6±0.2°, 4.5±0.2°, 15.4±0.2°, 14.6±0.2°, 24.0±0.2°, 21.5±0.2°, 20.5±0.2°, 18.0±0.2, 26.0±0.2°, 22.0±0.2°, 14.8±0.2°, 28.8±0.2° or 25.4±0.2°; preferably, comprises any 6, 8, 10, 11 or 12 of the above diffraction peaks;
   using Cu-Kα radiation, the X-ray characteristic diffraction peaks represented by 2θ angles and interplanar spacing d values are shown in Table 2.

**Table 2**

| No. | XRPD X-ray diffraction data of the crystal form II of the compound of embodiment 63 | | | | | |
|---|---|---|---|---|---|---|
| | 2θ (±0.2 °) | d value | Peak height | Proportion (I%) | Area | Proportion (I%) |
| 1 | 4.504 | 19.6038 | 493 | 24 | 4333 | 33.4 |
| 2 | 4.792 | 18.4264 | 2058 | 100 | 12984 | 100 |
| 3 | 7.992 | 11.0529 | 134 | 6.5 | 803 | 6.2 |
| 4 | 9.389 | 9.4118 | 812 | 39.5 | 5836 | 44.9 |
| 5 | 12.722 | 6.9526 | 127 | 6.2 | 1300 | 10 |
| 6 | 14.583 | 6.069 | 431 | 20.9 | 2970 | 22.9 |
| 7 | 14.801 | 5.9803 | 222 | 10.8 | 2546 | 19.6 |
| 8 | 15.353 | 5.7664 | 474 | 23 | 3106 | 23.9 |
| 9 | 16.567 | 5.3466 | 854 | 41.5 | 6411 | 49.4 |
| 10 | 16.993 | 5.2135 | 511 | 24.8 | 4718 | 36.3 |
| 11 | 17.662 | 5.0174 | 1010 | 49.1 | 6824 | 52.6 |
| 12 | 18.009 | 4.9215 | 206 | 10 | 1872 | 14.4 |
| 13 | 18.354 | 4.8299 | 557 | 27.1 | 5343 | 41.2 |
| 14 | 18.576 | 4.7725 | 504 | 24.5 | 4212 | 32.4 |
| 15 | 19.143 | 4.6325 | 554 | 26.9 | 3823 | 29.4 |
| 16 | 19.895 | 4.4591 | 131 | 6.4 | 1118 | 8.6 |
| 17 | 20.545 | 4.3194 | 261 | 12.7 | 2750 | 21.2 |
| 18 | 21.48 | 4.1334 | 270 | 13.1 | 2122 | 16.3 |
| 19 | 22.042 | 4.0292 | 288 | 14 | 2991 | 23 |
| 20 | 22.326 | 3.9787 | 133 | 6.5 | 1318 | 10.2 |
| 21 | 22.835 | 3.8912 | 108 | 5.2 | 1136 | 8.7 |
| 22 | 23.951 | 3.7124 | 415 | 20.2 | 3602 | 27.7 |
| 23 | 25.086 | 3.5469 | 146 | 7.1 | 2093 | 16.1 |
| 24 | 25.447 | 3.4974 | 216 | 10.5 | 3291 | 25.3 |
| 25 | 25.996 | 3.4247 | 292 | 14.2 | 4449 | 34.3 |
| 26 | 26.624 | 3.3453 | 189 | 9.2 | 1048 | 8.1 |
| 27 | 26.975 | 3.3026 | 157 | 7.6 | 1198 | 9.2 |
| 28 | 28.834 | 3.0937 | 223 | 10.8 | 2373 | 18.3 |
| 29 | 29.12 | 3.0641 | 110 | 5.3 | 1307 | 10.1 |

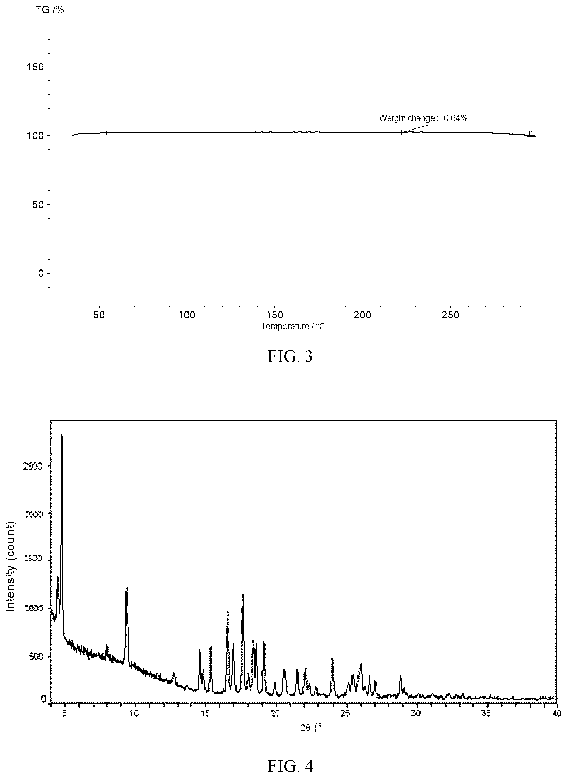

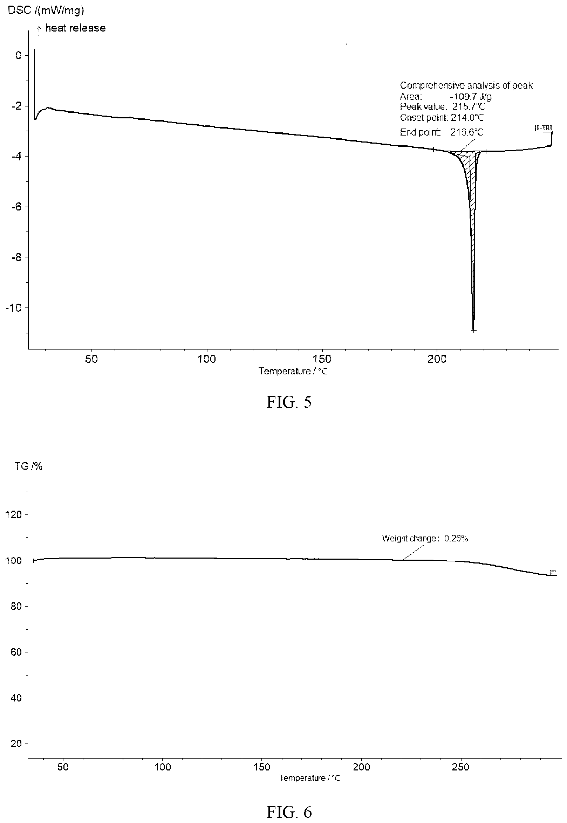

The crystal form II of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63 of the present disclosure has an X-ray powder diffraction pattern basically as shown in FIG. 4; a DSC pattern basically as shown in FIG. 5; a TGA pattern basically as shown in FIG. 6.

In a further preferred embodiment of the present disclosure, the crystal form III of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63.

The crystal form III has an X-ray powder diffraction pattern having a diffraction peak at 2θ angle of 4.6±0.2°; or having a diffraction peak at 15.2±0.2°; or having a diffraction peak at 9.9±0.2°; or having a diffraction peak at 16.7±0.2°; or having a diffraction peak at 18.2±0.2°; or having a diffraction peak at 17.9±0.2°; or having a diffraction peak at 25.5±0.2°; or having a diffraction peak at 15.0±0.2°; or having a diffraction peak at 19.5±0.2°; or having a diffraction peak at 23.6±0.2°; preferably comprising any 2-5, or 3-5, or 3-6, or 3-8, or 5-8, or 6-8 of the above diffraction peaks; more preferably comprising any 6, 7, or 8 of the diffraction peaks;
the X-ray powder diffraction pattern of the crystal form III comprises at least one or more diffraction peaks at 2θ angles of 4.6±0.2°, 15.2±0.2° or 9.9±0.2°; preferably comprises two of the above diffraction peaks, more preferably comprises three of the diffraction peaks; optionally, further also comprises at least one diffraction peak at 2θ angle of 16.7±0.2°, 18.2±0.2°, 17.9±0.2°, 25.5±0.2° or 15.0±0.2°, preferably comprises 2, 3, 4 or 5 of the above diffraction peaks;
or, comprises diffraction peaks at 2θ angles of 4.6±0.2°, 15.2±0.2°, 9.9±0.2°, 16.7±0.2°, 18.2±0.2° and 17.9±0.2°;
or, comprises diffraction peaks at 2θ angles of 4.6±0.2°, 15.2±0.2°, 9.9±0.2°, 16.7±0.2°, 18.2±0.2° and 25.5±0.2°;
or, comprises diffraction peaks at 2θ angles of 4.6±0.2°, 15.2±0.2°, 9.9±0.2°, 16.7±0.2°, 18.2±0.2° and 15.0±0.2°;
or, comprises diffraction peaks at 2θ angles of 4.6±0.2°, 15.2±0.2°, 9.9±0.2°, 16.7±0.2°, 18.2±0.2° and 25.5±0.2°;
or, comprises diffraction peaks at 2θ angles of 4.6±0.2°, 15.2±0.2°, 9.9±0.2°, 18.2±0.2° 17.9±0.2° and 15.0±0.2°;
the X-ray powder diffraction pattern of the crystal form III optionally also comprises one or more diffraction peaks at 2θ angles of 19.5±0.2°, 23.6±0.2°, 26.1±0.2°, 22.0±0.2°, 20.2±0.2°, 21.1±0.2° or 27.4±0.2°; preferably comprises at least any 2-3, or 4-5, or 6-7 of the above diffraction peaks; further preferably, comprises any 2, 3, 4, 5, 6, 7 of the diffraction peaks;
comprises diffraction peaks at 2θ angles of 4.6±0.2°, 15.2±0.2°, 9.9±0.2°, 16.7±0.2°, 18.2±0.2°, 17.9±0.2°, 19.5±0.2° and 23.6±0.2°;
or, comprises diffraction peaks at 2θ angles of 4.6±0.2°, 15.2±0.2°, 9.9±0.2°, 16.7±0.2°, 18.2±0.2°, 17.9±0.2°, 19.5±0.2° and 26.1±0.2°;
the X-ray powder diffraction pattern of the crystal form III comprises one or more diffraction peaks at 2θ angles of 4.6±0.2°, 15.2±0.2°, 9.9±0.2°, 16.7±0.2°, 18.2±0.2°, 17.9±0.2°, 25.5±0.2°, 15.0±0.2°, 19.5±0.2°, 23.6±0.2°, 26.1±0.2°, 22.0±0.2°, 20.2±0.2°, 21.1±0.2° or 27.4±0.2°; preferably, comprises any 4, 5, 6, 8 or 10 of the above diffraction peaks;
the X-ray powder diffraction pattern of the crystal form III comprises diffraction peaks at following 2θ angles of:
   4.6±0.2°, 15.2±0.2°, 9.9±0.2° and 16.7±0.2°;
   or, 4.6±0.2°, 15.2±0.2°, 9.9±0.2° and 18.2±0.2°;
   or, 4.6±0.2°, 15.2±0.2°, 16.7±0.2° and 18.2±0.2°;
   or, 4.6±0.2°, 9.9±0.2°, 16.7±0.2° and 18.2±0.2°;
   or, 15.2±0.2°, 9.9±0.2°, 16.7±0.2° and 18.2±0.2°;
   4.6±0.2°, 15.2±0.2°, 9.9±0.2°, 16.7±0.2°, 18.2±0.2° and 17.9±0.2°;
   4.6±0.2°, 15.2±0.2°, 9.9±0.2°, 16.7±0.2°, 18.2±0.2°, 17.9±0.2°, 26.1±0.2° and 22.0±0.2°;
   4.6±0.2°, 15.2±0.2°, 9.9±0.2°, 16.7±0.2°, 18.2±0.2°, 17.9±0.2°, 25.5±0.2°, 15.0±0.2°, 19.5±0.2° and 23.6±0.2°;
   using Cu-Kα radiation, the X-ray characteristic diffraction peaks represented by 2θ angles and interplanar spacing d values are shown in Table 3.

**Table 3**

| No. | XRPD X-ray diffraction data of the crystal form III of the compound of embodiment 63 | | | | | |
|---|---|---|---|---|---|---|
| | 2θ (±0.2°) | d value | Peak height | Proportion (I%) | Area | Proportion (I%) |
| 1 | 4.588 | 19.2419 | 1334 | 100 | 10972 | 100 |
| 2 | 8.435 | 10.4741 | 88 | 6.6 | 614 | 5.6 |
| 3 | 9.922 | 8.9076 | 414 | 31 | 6208 | 56.6 |
| 4 | 14.971 | 5.9129 | 202 | 15.1 | 6637 | 60.5 |
| 5 | 15.23 | 5.8127 | 501 | 37.6 | 7112 | 64.8 |
| 6 | 16.691 | 5.3071 | 361 | 27.1 | 4827 | 44 |
| 7 | 17.947 | 4.9383 | 273 | 20.5 | 5505 | 50.2 |
| 8 | 18.23 | 4.8623 | 360 | 27 | 7587 | 69.1 |
| 9 | 19.53 | 4.5416 | 192 | 14.4 | 2413 | 22 |
| 10 | 20.2 | 4.3924 | 142 | 10.6 | 872 | 7.9 |
| 11 | 21.07 | 4.2129 | 139 | 10.4 | 1962 | 17.9 |
| 12 | 21.962 | 4.0438 | 151 | 11.3 | 2037 | 18.6 |
| 13 | 23.561 | 3.7729 | 175 | 13.1 | 2656 | 24.2 |
| 14 | 25.472 | 3.494 | 230 | 17.2 | 4657 | 42.4 |
| 15 | 26.132 | 3.4072 | 174 | 13 | 3629 | 33.1 |
| 16 | 27.351 | 3.258 | 121 | 9.1 | 1308 | 11.9 |
| 17 | 28.227 | 3.1589 | 84 | 6.3 | 1896 | 17.3 |

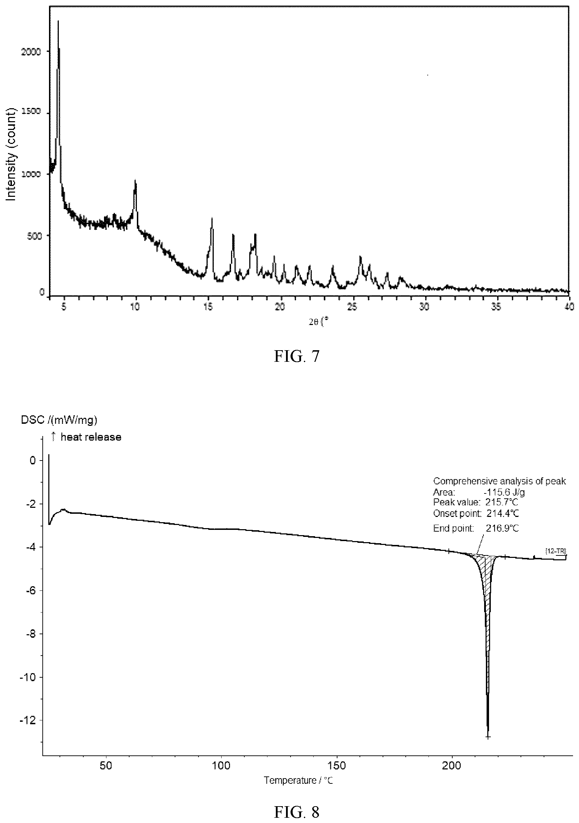

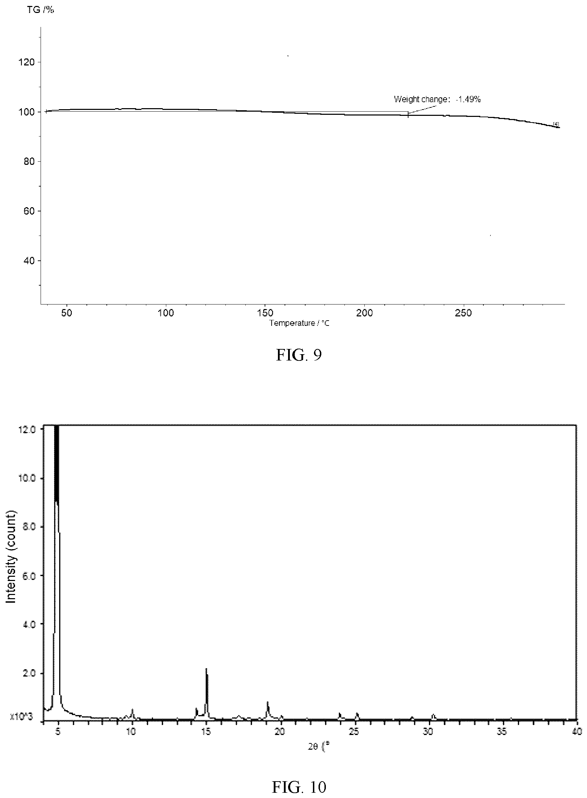

The crystal form III of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63 of the present disclosure has an X-ray powder diffraction pattern basically as shown in FIG. 7; a DSC pattern basically as shown in FIG. 8; a TGA pattern basically as shown in FIG. 9.

In a further preferred embodiment of the present disclosure, the crystal form IV of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile shown in embodiment 63.

The crystal form IV has an X-ray powder diffraction pattern having a diffraction peak at 2θ angle of 5.0±0.2°; or having a diffraction peak at 4.8±0.2°; or having a diffraction peak at 15.0±0.2°; or having a diffraction peak at 19.1±0.2°; or having a diffraction peak at 14.3±0.2°; or having a diffraction peak at 10.0±0.2°; or having a diffraction peak at 23.9±0.2°; or having a diffraction peak at 25.1±0.2°; or having a diffraction peak at 30.3±0.2°; or having a diffraction peak at 9.6±0.2°; preferably comprises any 2-5, or 3-5, or 3-6, or 3-8, or 5-8, or 6-8 of the above diffraction peaks; more preferably comprises any 6, 7, or 8 of the diffraction peaks;
the X-ray powder diffraction pattern of the crystal form IV comprises at least one or more diffraction peaks at 2θ angles of 5.0±0.2°, 4.8±0.2° or 15.0±0.2°, preferably comprises two of the above diffraction peaks, more preferably comprises three of the diffraction peaks; optionally, further also comprises at least one diffraction peak at 2θ angle of 19.1±0.2°, 14.3±0.2°, 10.0±0.2°, 23.9±0.2° or 25.1±0.2°, preferably comprises 2, 3, 4 or 5 of the above diffraction peaks;
or, comprises diffraction peaks at 2θ angles of 5.0±0.2°, 4.8±0.2°, 15.0±0.2°, 19.1±0.2°, 14.3±0.2° and 10.0±0.2°;
or, comprises diffraction peaks at 2θ angles of 5.0±0.2°, 4.8±0.2°, 15.0±0.2°, 19.1±0.2°, 14.3±0.2° and 23.9±0.2°;
the X-ray powder diffraction pattern of the crystal form IV optionally also comprises one or more diffraction peaks at 2θ angles of 30.3±0.2° or 9.6±0.2°; preferably comprises at least any 2-3, or 4-5, or 6-7 of the above diffraction peaks; further preferably, comprises any 2, 3, 4, 5, 6, 7 of the diffraction peaks;
comprises diffraction peaks at 2θ angles of 5.0±0.2°, 4.8±0.2°, 15.0±0.2°, 19.1±0.2°, 14.3±0.2°, 10.0±0.2°, 23.9±0.2° and 30.3±0.2°;
or, comprises diffraction peaks at 2θ angles of 5.0±0.2°, 4.8±0.2°, 15.0±0.2°, 19.1±0.2°, 14.3±0.2°, 10.0±0.2°, 23.9±0.2° and 9.6±0.2°;
the X-ray powder diffraction pattern of the crystal form IV comprises one or more diffraction peaks at 2θ angles of 5.0±0.2°, 4.8±0.2°, 15.0±0.2°, 19.1±0.2°, 14.3±0.2°, 10.0±0.2°, 23.9±0.2°, 25.1±0.2°, 30.3±0.2° or 9.6±0.2°; preferably, comprises any 4, 5, 6, 8 or 10 of the above diffraction peaks;
the X-ray powder diffraction pattern of the crystal form IV has diffraction peaks at following 2θ angles of:
   comprises diffraction peaks at 2θ angles of 5.0±0.2°, 4.8±0.2°, 15.0±0.2° and 19.1±0.2°;
   or, 5.0±0.2°, 4.8±0.2°, 15.0±0.2° and 14.3±0.2°;
   or, 5.0±0.2°, 4.8±0.2°, 15.0±0.2°, 19.1±0.2°, 23.9±0.2° and 25.1±0.2°;
   or, 5.0±0.2°, 4.8±0.2°, 15.0±0.2°, 19.1±0.2°, 14.3±0.2°, 10.0±0.2°, 23.9±0.2° and 25.1±0.2°;
   or, 5.0±0.2°, 4.8±0.2°, 15.0±0.2°, 19.1±0.2°, 14.3±0.2°, 10.0±0.2°, 23.9±0.2°, 25.1±0.2° and 30.3±0.2°;
   or, 5.0±0.2°, 4.8±0.2°, 15.0±0.2°, 19.1±0.2°, 14.3±0.2°, 10.0±0.2°, 23.9±0.2°, 25.1±0.2°, 30.3±0.2° and 9.6±0.2°;
   using Cu-Kα radiation, the X-ray characteristic diffraction peaks represented by 2θ angles and interplanar spacing d values are shown in Table 4.

**Table 4**

| No. | XRPD X-ray diffraction data of the crystal form IV of the compound of embodiment 63 | | | | | |
|---|---|---|---|---|---|---|
| | 2θ (±0.2°) | d value | Peak height | Proportion (I%) | Area | Proportion (I%) |
| 1 | 4.79 | 18.4329 | 18504 | 76.6 | 217525 | 100 |
| 2 | 4.992 | 17.6863 | 24146 | 100 | 145528 | 66.9 |
| 3 | 9.592 | 9.2128 | 139 | 0.6 | 1687 | 0.8 |
| 4 | 9.983 | 8.8531 | 392 | 1.6 | 2644 | 1.2 |
| 5 | 14.306 | 6.1858 | 444 | 1.8 | 4841 | 2.2 |
| 6 | 14.993 | 5.9042 | 2086 | 8.6 | 17268 | 7.9 |
| 7 | 19.109 | 4.6406 | 673 | 2.8 | 6105 | 2.8 |
| 8 | 23.949 | 3.7126 | 259 | 1.1 | 2259 | 1 |
| 9 | 25.109 | 3.5437 | 268 | 1.1 | 2045 | 0.9 |
| 10 | 30.271 | 2.9501 | 230 | 1 | 2439 | 1.1 |

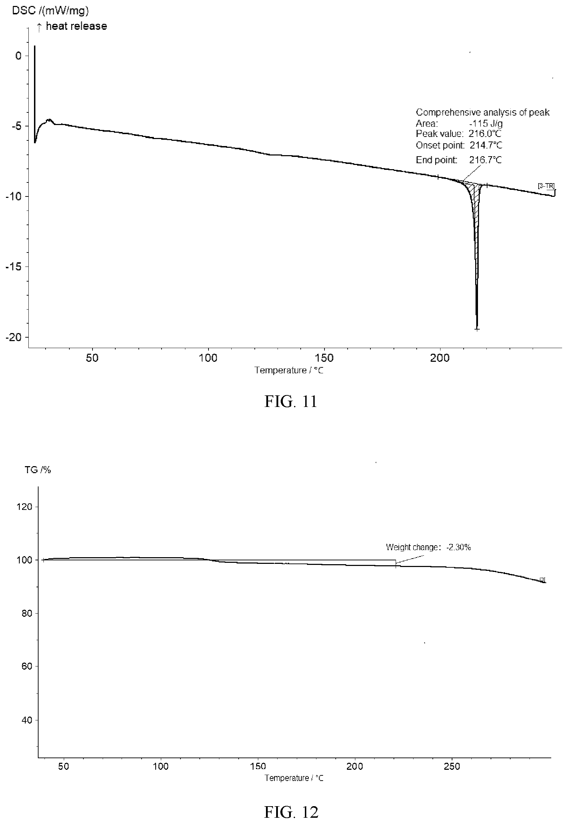

The crystal form IV of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63 of the present disclosure has an X-ray powder diffraction pattern basically as shown in FIG. 10; a DSC pattern basically as shown in FIG. 11; a TGA pattern basically as shown in FIG. 12.

In a further preferred embodiment of the present disclosure, the crystal form V of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63.

The crystal form V has an X-ray powder diffraction pattern having a diffraction peak at 2θ angle of 4.7±0.2°; or having a diffraction peak at 18.2±0.2°; or having a diffraction peak at 10. 1±0.2°; or having a diffraction peak at 18.8±0.2°; or having a diffraction peak at 15.6±0.2°; or having a diffraction peak at 17.0±0.2°; or having a diffraction peak at 21.8±0.2°; or having a diffraction peak at 14.7±0.2°; or having a diffraction peak at 19.3±0.2°; or having a diffraction peak at 25.8±0.2°; preferably comprising any 2-5, or 3-5, or 3-6, or 3-8, or 5-8, or 6-8 of the above diffraction peaks; more preferably comprising any 6, 7, or 8 of the diffraction peaks;
the X-ray powder diffraction pattern of the crystal form V comprises at least one or more diffraction peaks at 2θ angles of 4.7±0.2°, 18.2±0.2° or 10.1±0.2°; preferably comprises two of the above diffraction peaks, more preferably comprises three of the diffraction peaks; optionally, further also comprises at least one diffraction peak at 2θ angle of 18.8±0.2°, 15.6±0.2°, 17.0±0.2°, 21.8±0.2° or 14.7±0.2°, preferably comprises 2, 3, 4 or 5 of the above diffraction peaks;
or, comprises diffraction peaks at 2θ angles of 4.7±0.2°, 18.2±0.2°, 10.1±0.2°, 18.8±0.2, 15.6±0.2° and 17.0±0.2°;
or, comprises diffraction peaks at 2θ angles of 4.7±0.2°, 18.2±0.2°, 10.1±0.2°, 18.8±0.2, 15.6±0.2° and 21.8±0.2°;
or, comprises diffraction peaks at 2θ angles of 4.7±0.2°, 18.2±0.2°, 10.1±0.2°, 18.8±0.2, 15.6±0.2° and 14.7±0.2°.
the X-ray powder diffraction pattern of the crystal form V optionally also comprises one or more diffraction peaks at 2θ angles of 19.3±0.2°, 25.8±0.2°, 15.2±0.2°, 17.8±0.2°, 20.4±0.2°, 23.5±0.2° or 25.6±0.2°; preferably comprises at least any 2-3, or 4-5, or 6-7 of the above diffraction peaks; further preferably, comprises any 2, 3, 4, 5, 6, 7 of the diffraction peaks;
comprises diffraction peaks at 2θ angles of 4.7±0.2°, 18.2±0.2°, 10. 1±0.2°, 18.8±0.2°, 15.6±0.2°, 17.0±0.2°, 19.3±0.2° and 25.8±0.2°;
or, comprises diffraction peaks at 2θ angles of 4.7±0.2°, 18.2±0.2°, 10.1±0.2°, 18.8±0.2°, 15.6±0.2°, 17.0±0.2°, 19.3±0.2° and 15.2±0.2°.
The X-ray powder diffraction pattern of the crystal form V comprises one or more diffraction peaks at 2θ angles of 4.7±0.2°, 18.2±0.2°, 10.1±0.2°, 18.8±0.2°, 15.6±0.2°, 17.0±0.2°, 21.8±0.2°, 14.7±0.2°, 19.3±0.2°, 25.8±0.2°, 15.2±0.2°, 17.8±0.2°, 20.4±0.2°, 23.5±0.2° or 25.6±0.2°; preferably, comprises any 4, 5, 6, 8 or 10 of the above diffraction peaks;
the X-ray powder diffraction pattern of the crystal form V has diffraction peaks at following 2θ angles of:
   comprises diffraction peaks at 2θ angles of 4.7±0.2°, 18.2±0.2°, 10.1±0.2° and 18.8±0.2°;
   or, comprises diffraction peaks at 2θ angles of 4.7±0.2°, 10.1±0.2°, 18.8±0.2°, 15.6±0.2°, 17.0±0.2° and 21.8±0.2°;
   or, comprises diffraction peaks at 2θ angles of 4.7±0.2°, 18.2±0.2°, 10.1±0.2°, 18.8±0.2°, 15.6±0.2°, 17.0±0.2°, 21.8±0.2° and 14.7±0.2°;
   or, comprises diffraction peaks at 2θ angles of 4.7±0.2°, 18.2±0.2°, 10.1±0.2°, 18.8±0.2°, 15.6±0.2°, 17.0±0.2°, 21.8±0.2°, 14.7±0.2°, 19.3±0.2°, and 15.2±0.2°.
   Using Cu-Kα radiation, the X-ray characteristic diffraction peaks represented by 2θ angles and interplanar spacing d values are shown in Table 5.

**Table 5**

| No. | XRPD X-ray diffraction data of the crystal form V of the compound of embodiment 63 | | | | | |
|---|---|---|---|---|---|---|
| | 2θ (±0.2°) | d value | Peak height | Proportion (I%) | Area | Proportion (I%) |
| 1 | 4.67 | 18.9077 | 7168 | 100 | 60818 | 100 |
| 2 | 10.127 | 8.7276 | 298 | 4.2 | 2287 | 3.8 |
| 3 | 14.053 | 6.2969 | 77 | 1.1 | 663 | 1.1 |
| 4 | 14.748 | 6.0017 | 161 | 2.2 | 1656 | 2.7 |
| 5 | 15.249 | 5.8054 | 114 | 1.6 | 1464 | 2.4 |
| 6 | 15.559 | 5.6905 | 200 | 2.8 | 1204 | 2 |
| 7 | 16.992 | 5.2137 | 173 | 2.4 | 1751 | 2.9 |
| 8 | 17.766 | 4.9882 | 109 | 1.5 | 1166 | 1.9 |
| 9 | 18.23 | 4.8623 | 305 | 4.3 | 2516 | 4.1 |
| 10 | 18.777 | 4.7219 | 302 | 4.2 | 4255 | 7 |
| 11 | 19.289 | 4.5977 | 147 | 2.1 | 1504 | 2.5 |
| 12 | 20.36 | 4.3582 | 106 | 1.5 | 1256 | 2.1 |
| 13 | 20.788 | 4.2695 | 80 | 1.1 | 922 | 1.5 |
| 14 | 21.841 | 4.0659 | 173 | 2.4 | 1357 | 2.2 |
| 15 | 23.521 | 3.7791 | 99 | 1.4 | 2240 | 3.7 |
| 16 | 25.574 | 3.4803 | 86 | 1.2 | 1614 | 2.7 |
| 17 | 25.835 | 3.4457 | 146 | 2 | 2713 | 4.5 |

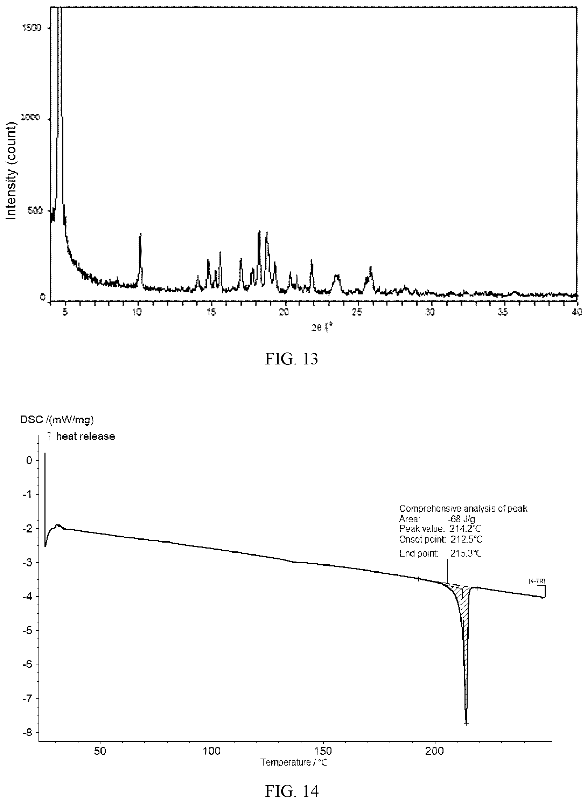

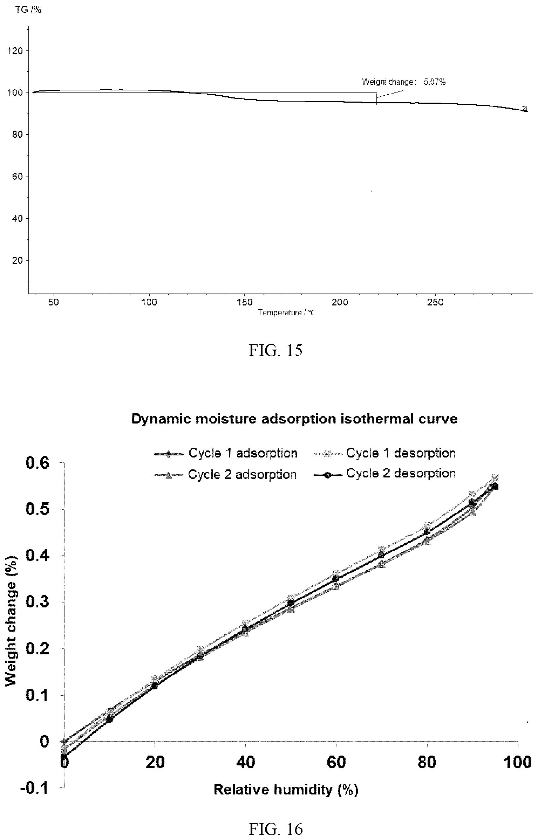

The crystal form V of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile shown in embodiment 63 of the present disclosure has an X-ray powder diffraction pattern basically as shown in FIG. 13; a DSC pattern basically as shown in FIG. 13; a TGA pattern basically as shown in FIG. 15.

For the crystal forms described in the present disclosure, positions of diffraction peaks with relative peak intensity of top ten in the X-ray powder diffraction patterns of the crystal forms and diffraction peaks at corresponding positions in the corresponding figures have a 2θ error of ±0.2° to ±0.5°, preferably ±0.2° to ±0.3°, most preferably ±0.2°.

In a preferred embodiment of the present disclosure, for the crystal form of any one of the compound represented by general formula (I), the crystal form is a solvent-containing or solvent-free,wherein the solvent is selected from one or more of water, methanol, acetone, ethyl acetate, acetonitrile, ethanol, 88% acetone, tetrahydrofuran, 2-methyl-tetrahydrofuran, dichloromethane, 1,4-dioxane, benzene, toluene, isopropanol, *n*-butanol, isobutanol, *N,N-*dimethylformamide, *N,N*-dimethylacetamide, *N-*methylpyrrolidone, dimethyl sulfoxide, *n-*propanol, *tert*-butanol, 2-butanone, 3-pentanone, *n*-heptane, heptane, ethyl formate, isopropyl acetate, cyclohexane, methyl *tert*-butyl ether or diisopropyl ether.

In a preferred embodiment of the present disclosure, for the crystal form of any one of the compound represented by general formula (I), the number of the solvent is 0.2-3, preferably 0.2, 0.5, 1, 1.5, 2, 2.5 or 3, more preferably 0.5, 1, 2 or 3.

In a preferred embodiment of the present disclosure, the crystal form of the compound represented by general formula (I) is a non-solvent crystal form, preferably an anhydrous crystal form.

In a preferred embodiment of the present disclosure, the crystal form of the compound represented by general formula (I) is a hydrate crystal form, the number of water is 0.2-3, preferably 0.2, 0.5, 1, 1.5, 2, 2.5 or 3, more preferably 0.5, 1, 2 or 3.

In a more preferred embodiment of the present disclosure, the crystal form of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile is a non-solvent compound , preferably an anhydrate or a hydrate.

In a more preferred embodiment of the present disclosure, the number of water in the hydrate crystal form of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile is 0.2-3, preferably 0.2, 0.5, 1, 1.5, 2, 2.5 or 3, more preferably 0.5, 1, 2 or 3.

It is well known to those skilled in the art that XRPD may produce certain displacement and strength deviation due to the detection method, conditions and instruments. As a specific example of the crystal form of the present disclosure, an XRPD thereof is shown in pattern X, but ordinary technicians understand that when the key characteristic peak displacement has a 2θ deviation of±0.5, especially around±0.2, the crystal forms can be identified as the same crystal form.

The present disclosure also provides a method for preparing the crystal form of the compound represented by general formula (I), specifically comprising the following steps:
1) weighing an appropriate amount of a free base and suspending with a poor solvent, preferably with a suspension density of 50 to 200 mg/mL;
2) shaking a suspension obtained above, preferably at a temperature of 0 to 50°C and preferably for a time of 4 hours to 10 days;
3) centrifuging the above suspension rapidly, removing a supernatant, and drying a remaining solid to constant weight in a 50°C vacuum drying oven to obtain a target product;
wherein,
the poor solvent is selected from methanol, acetone, ethyl acetate, tetrahydrofuran, acetonitrile, ethanol, 88% acetone, 2-methyl-tetrahydrofuran, dichloromethane, 1,4-dioxane, methyl *tert*-butyl ether, *n*-heptane, benzene, toluene, chlorobenzene, isopropanol, *n*-butanol, isobutanol, *N,N-*dimethylformamide, *N,N*-dimethylacetamide, dimethyl sulfoxide, *n*-propanol, ethyl formate, isopropyl acetate, *tert*-butanol, 2-butanone or 3-pentanone; preferably acetonitrile, acetone, ethyl acetate, methanol, ethanol.

The present disclosure also provides a method for preparing the crystal form of the compound represented by general formula (I), specifically comprising the following steps:
1) weighing an appropriate amount of a free base and dissolving with a good solvent;
2) optionally, adding an anti-solvent to a solution obtained above;
3) stirring until a solid is precipitated, preferably at a crystallization temperature of 0 to 50°C;
4) optionally, centrifuging the above suspension rapidly;
5) removing a supernatant, and drying a remaining solid to obtain a target product;
wherein,
the good solvent is selected from methanol, acetone, ethyl acetate, tetrahydrofuran, acetonitrile, ethanol, 88% acetone, 2-methyl-tetrahydrofuran, dichloromethane, 1,4-dioxane, benzene, toluene, chlorobenzene, isopropanol, *n*-butanol, isobutanol, *N,N-*dimethylformamide, *N,N*-dimethylacetamide, dimethyl sulfoxide, *n*-propanol, ethyl formate, isopropyl acetate, *tert-*butanol, 2-butanone or 3-pentanone; preferably dichloromethane, tetrahydrofuran, 2-methyl-tetrahydrofuran, 1,4-dioxane, dimethyl sulfoxide, acetonitrile, 2-butanone.

The poor solvent is selected from methanol, ethanol, ethyl acetate, acetone, isopropanol, toluene, *n*-heptane, water, isopropyl acetate, cyclohexane, methyl *tert*-butyl ether, diisopropyl ether; preferably water, *n*-heptane, cyclohexane and methyl *tert*-butyl ether.

The purpose of the present disclosure is also to provide a pharmaceutical composition comprising a therapeutically effective amount of the crystal form of the compound represented by general formula (I), and one or more pharmaceutically acceptable carriers or excipients.

The purpose of the present disclosure is also to provide a use of the crystal form of the compound 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*] pyridine-3-carbonitrile represented by general formula (I), and a pharmaceutical composition thereof in the manufacture of a medicament of a RET inhibitor.

The purpose of the present disclosure is to provide a use of the crystal form of the compound represented by general formula (I) and the pharmaceutical composition in the manufacture of a medicament for the treatment and/or prevention of a tumor, preferably, the tumor is selected from non-small cell lung cancer, fibrosarcoma, pancreatic tumor, medullary thyroid carcinoma, papillary thyroid tumor, soft tissue sarcoma, highly solid tumor, breast tumor and colon tumor.

The crystal form may be identified by powder X-ray diffraction spectrum. However, those skilled in the art should be understood that peak intensity and/or peak condition of powder X-ray diffraction may be different due to different experimental conditions, such as different diffraction test conditions and/or preferred orientation. At the same time, the measured 2θ value may have an error of about ±0.2, and individual peaks may have an error of about ±0.3 or ±0.4 due to the different accuracy of different instruments. However, it is known that the relative intensity value of the peak is more dependent on some properties of the measured sample than the position of the peak, such as the size of the crystal in the sample, the orientation effect of the crystal and the purity of the material being analyzed, therefore, the deviation of the peak intensity shown in the range of about±20% or more is possible.

### Detailed description of the invention

Unless otherwise stated, the terms used in the description and claims have the following meanings.

The term "alkyl" refers to a saturated aliphatic hydrocarbon group, which is a straight or branched chain group containing 1 to 20 carbon atoms, preferably alkyl containing 1 to 8 carbon atoms, more preferably alkyl containing 1 to 6 carbon atoms, most preferably alkyl containing 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers. More preferably lower alkyl containing 1 to 6 carbon atoms, non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, when substituted, the substituents may be substituted at any available attachment point, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate, preferably alkyl substituted by methyl, ethyl, isopropyl, *tert*-butyl, haloalkyl, deuterated alkyl, alkoxy-substituted alkyl and hydroxyl-substituted alkyl.

The term "cycloalkyl" refers to a saturated or partially unsaturated monocyclic or polycyclic cyclic hydrocarbon substituent, a cycloalkyl ring contains 3 to 20 carbon atoms, preferably 3 to 12 carbon atoms, more preferably 3 to 8 carbon atoms, most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctanyl, etc.; polycylic cycloalkyl includes spiro, fused and bridged cycloalkyl, preferably cyclopropyl, cyclobutyl, cyclohexyl, cyclopentyl and cycloheptyl.

The term "heterocyclyl" refers to a saturated or partially unsaturated monocyclic or polycyclic cyclic hydrocarbon substituent comprising 3 to 20 ring atoms, wherein one or more of the ring atoms are heteroatoms selected from nitrogen, oxygen or S(O)ₘ (wherein m is an integer of 0 to 2), but not including the ring part of -O-O-, -O-S- or -S-S-, and the remaining ring atoms are carbon. It preferably contains 3 to 12 ring atoms, wherein 1 to 4 ring atoms are heteroatoms; more preferably contains 3 to 8 ring atoms; most preferably contains 3 to 6 ring atoms. Non-limiting examples of monocyclic heterocyclyl include oxetanyl, thietanyl, pyrrolidinyl, pyrrolidonyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, etc., preferably oxetanyl, pyrrolidonyl, tetrahydrofuranyl, pyrazolidinyl, morpholinyl, piperazinyl and pyranyl. Polycyclic heterocyclyl includes spiro, fused and bridged heterocyclyl; the spiro, fused and bridged heterocyclyl are optionally connected to other groups through a single bond, or further connected in parallel with other cycloalkyl, heterocyclyl, aryl and heteroaryl through any two or more of ring atoms.

The heterocyclyl may be optionally substituted or unsubstituted, when substituted, the substituent is preferably one or more of the following groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylic ester group.

"Haloalkyl" refers to alkyl substituted by one or more halogens, wherein the alkyl is as defined above.

"Haloalkoxy" refers to alkoxy substituted by one or more halogens, wherein the alkoxy is as defined above.

"Hydroxyalkyl" refers to alkyl substituted by one or more hydroxyl, wherein the alkyl is as defined above.

"Alkenyl" refers to chain alkenyl, also known as olefinic group, wherein the alkenyl may be further substituted with other related groups, for example: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Alknyl" refers to (CH=C-), wherein the alknyl may be further substituted by other related groups, for example: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Hydroxyl" refers to the -OH group.

"Halogen" refers to fluorine, chlorine, bromine or iodine.

"Amino" refers to -NH₂.

"Cyano" refers to -CN.

"Nitro" refers to -NO₂.

"Carboxyl" refers to -C(O)OH.

"THF" refers to tetrahydrofuran.

"EtOAc" refers to ethyl acetate.

"MeOH" refers to methanol.

"DMF" refers to *N*,*N*-dimethylformamide.

"DIPEA" refers to diisopropylethylamine.

"TFA" refers to trifluoroacetic acid.

"MeCN" refers to acetonitrile.

"DMA" refers to *N,N-*dimethylacetamide.

"Et₂O" refers to diethyl ether.

"DCE" refers to 1,2 dichloroethane.

"DIPEA" refers to *N,N-*diisopropylethylamine.

"NBS" refers to *N*-bromosuccinimide.

"NIS" refers to *N*-iodosuccinimide.

"Cbz-Cl" refers to benzyl chloroformate.

"Pd₂(dba)₃" refers to tris(dibenzylideneacetone)dipalladium.

"Dppf" refers to 1,1'-bis(diphenylphosphino)ferrocene.

"HATU" refers to 2-(7-aza-1*H*-benzotriazole-1-yl)-1,1,3,3-tetramethyluronium hexafluorophosphate.

"KHMDS" refers to potassium hexamethyldisilazide.

"LiHMDS" refers to lithium bistrimethylsilylamide.

"MeLi" refers to methyl lithium.

"*n*-BuLi" refers to *n*-butyl lithium.

"NaBH(OAc)₃" refers to sodium triacetoxyborohydride.

"DMAP" refers to 4-dimethylaminopyridine.

"SEM-Cl" refers to 2-chloromethyl 2-(trimethylsilyl)ethyl ether.

"Xantphos" refers to 4,5-bis(diphenylphosphino)-9,9-dimethylxanthene.

"DCM" refers to dichloromethane.

"X is selected from A, B, or C", "X is selected from A, B and C", "X is A, B or C", "X is A, B and C" and other terms all express the same meaning, which means that X can be any one or more of A, B, and C.

The hydrogen atom described in the present disclosure can be replaced by its isotope deuterium, and any hydrogen atom in the embodiment compounds of the present disclosure can also be replaced by a deuterium atom.

"Optional" or "optionally" refers to that the event or environment described later can but does not have to occur, and the description includes occasions where the event or environment occurs or does not occur. For example, "heterocyclic group optionally substituted by alkyl" refers to that alkyl may but does not have to be present, and the description includes the case where the heterocyclic group is substituted by alkyl and the case where the heterocyclic group is not substituted by alkyl.

"Substituted" refers to one or more hydrogen atoms in the group, preferably up to 5, more preferably 1 to 3 hydrogen atoms, independently substituted by a corresponding number of substituents. It goes without saying that the substituents are only in their possible chemical positions, and those skilled in the art can determine (by experiment or theory) possible or impossible substitutions without too much effort. For example, amino or hydroxyl having free hydrogen may be unstable when combined with a carbon atom having an unsaturated (e.g., olefinic) bond.

"Pharmaceutical composition" refers to a mixture comprising one or more of the compounds described herein or the physiologically/pharmaceutically acceptable salt or prodrug thereof and other chemical components, and the other component is, for example, physiological/pharmaceutically acceptable carrier and excipient. The purpose of the pharmaceutical composition is to promote the administration to the organism, facilitate the absorption of the active ingredient and then exert the biological activity.

"Pharmaceutically acceptable salt" refers to a salt of the compound of the present disclosure, which is safe and effective when used in mammals, and has due biological activity.

### BRIEF DESCRIPTION OF THE DRAWINGS

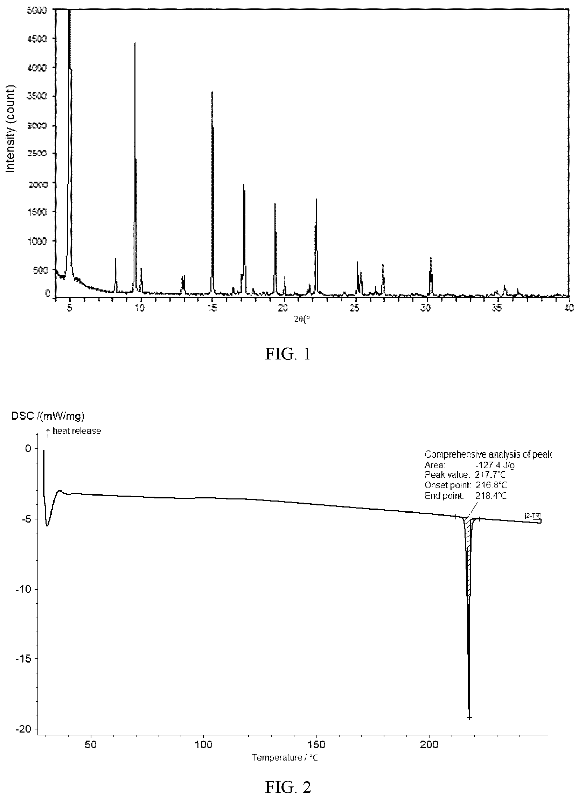

FIG. 1 is an XRPD pattern of a crystal form I of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 2 is a DSC pattern of the crystal form I of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 3 is a TGA pattern of the crystal form I of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 4 is an XRPD pattern of a crystal form II of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 5 is a DSC pattern of the crystal form II of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 6 is a TGA pattern of the crystal form II of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 7 is an XRPD pattern of a crystal form III of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 8 is a DSC pattern of the crystal form III of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 9 is a TGA pattern of the crystal form III of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 10 is an XRPD pattern of a crystal form IV of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 11 is a DSC pattern of the crystal form IV of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 12 is a TGA pattern of the crystal form IV of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 13 is an XRPD pattern of a crystal form V of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 14 is a DSC pattern of the crystal form V of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 15 is a TGA pattern of the crystal form V of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.
FIG. 16 is a DVS pattern of the crystal form I of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.

### DETAILED DESCRIPTION OF THE PREFERRED EMBODIMENT

The following embodiments will further describe the present disclosure, but these embodiments do not limit the scope of the present disclosure.

### 1. Preparation of compounds

### Embodiment

The structures of the compounds of the present disclosure were determined by nuclear magnetic resonance (NMR) or/and liquid chromatography-mass spectrometry (LC-MS). NMR chemical shift (δ) was given in units of parts per million (ppm). NMR was determined using a Bruker AVANCE-400 NMR instrument with deuterated dimethyl sulfoxide (DMSO-*d₆*), deuterated methanol (CD₃OD) and deuterated chloroform (CDCl₃) as solvents and tetramethylsilane (TMS) as internal standard.

Liquid chromatography-mass spectrometry LC-MS was determined with an Agilent 1200 Infinity Series mass spectrometer. HPLC determinations were performed using an Agilent 1200DAD high pressure liquid chromatograph (Sunfire C18 150 × 4.6 mm column) and a Waters 2695-2996 high pressure liquid chromatograph (Gimini C18 150 × 4.6 mm column).

Yantai Huanghai HSGF254 or Qingdao GF254 silica gel plate was used as thin-layer chromatography silica gel plate, the specification of TLC was 0.15 mm to 0.20 mm, and the specification of thin-layer chromatography separation and purification products was 0.4 m to 0.5 mm. Generally, 200-300 mesh Yantai Huanghai silica gel was used as a carrier for column chromatography.

The starting materials in the embodiments of the present disclosure are known and commercially available, or can be synthesized by using or following methods known in the art.

Unless otherwise specified, all reactions of the present disclosure were carried out under continuous magnetic stirring under dry nitrogen or argon atmosphere, the solvent is a dry solvent, and the unit of the reaction temperature was degrees Celsius.

### Embodiment 1

### 4-(6-(6-((6-Methoxypyridin-3-yl)methyl)-3,6-diazabicyclo[3.1.1]heptan-3-yl)pyridin-3-yl)-6-(2-(methylsulfinyl)ethoxy)pyrazolo[1,5-a]pyridine-3-carbonitrile

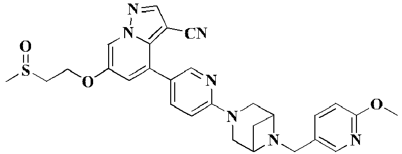

### Step 1: 2-(methylthio)ethan-1-ol

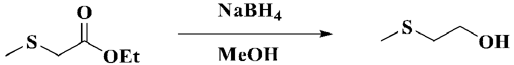

Ethyl 2-(methylthio)acetate (500 mg, 3.7 mmol) was dissolved in 20 mL of MeOH, and NaBH₄ (562 mg, 14.8 mmol) was added thereto at 0°C, and the mixture was stirred at room temperature for 0.5 hour. 10 mL of NH₄Cl solution was added thereto, and the mixture was extracted with ethyl acetate (20 mL*3). The organic phase was washed with water and saturated brine, dried over anhydrous sodium sulfate. The residue was filtered and evaporated to dryness by rotary evaporation to obtain 2-(methylthio)ethan-1-ol (240 mg, colorless liquid, yield: 70%).

### Step 2: 4-bromo-6-(2-(methylthio)ethoxy)pyrazolo[1,5-a]pyridine-3-carbonitrile

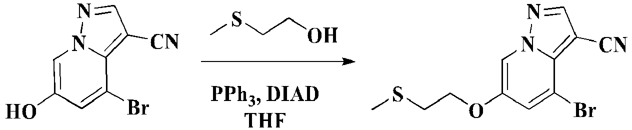

4-Bromo-6-hydroxypyrazolo[1,5-*a*]pyridine-3-carbonitrile (100 mg, 0.42 mmol) was dissolved in 10 mL of THF; 2-(methylthio)ethan-1-ol (16 mg, 0.5 mmol), triphenylphosphine (165 mg, 0.63 mmol) and DIAD (127 mg, 0.63 mmol) were added thereto and stirred at room temperature overnight. 10 mL of water was added thereto, and the mixture was extracted with ethyl acetate (20 mL*3). The organic phase was washed with water and saturated brine, dried over anhydrous sodium sulfate. The residue was filtered and evaporated to dryness by rotary evaporation, and the crude product was separated by column chromatography (eluted with dichloromethane/methanol = 10/1) to obtain 4-bromo-6-(2-(methylthio)ethoxy)pyrazolo[1,5-*a*]pyridine-3-carbonitrile (69 mg, white solid, yield: 53%).

MS m/z (ESI): 311.9 [M+H]⁺.

### Step 3: 4-bromo-6-(2-(methylsulfinyl)ethoxy)pyrazolo[1,5-a]pyridine-3-carbonitrile

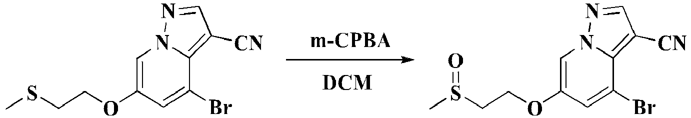

4-Bromo-6-(2-(methylthio)ethoxy)pyrazolo[1,5-*a*]pyridine-3-carbonitrile (200 mg, 0.64 mmol) was dissolved in 20 mL of DCM; 3-chloroperbenzoic acid (110 mg, 0.64 mmol) was added thereto, and the mixture was stirred at room temperature for 4 hours, 10 mL of water was added thereto and the mixture was extracted with ethyl acetate (20 mL*3). The organic phase was washed with water and saturated brine, dried over anhydrous sodium sulfate. The residue was filtered and evaporated to dryness by rotary evaporation, and the crude product was separated by column chromatography (eluted with dichloromethane/methanol = 10/1) to obtain 4-bromo-6-(2-(methylsulfinyl)ethoxy)pyrazolo[1,5-*a*]pyridine-3-carbonitrile (189 mg, white solid, yield: 90%).

MS m/z (ESI): 327.9 [M+H]⁺.

### Step 4: tert-butyl 3-(5-bromopyridin-2-yl)-3,6-diazabicyclo[3.1.1]heptane-6-carboxylate

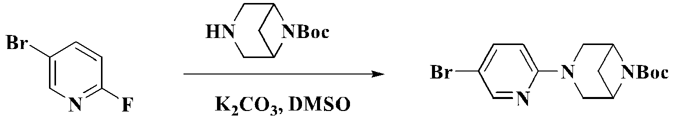

5-Bromo-2-fluoropyridine (500 mg, 2.5 mmol) was dissolved in 20 mL of DMSO; and tert-butyl 3,6-diazabicyclo[3.1.1]heptane-6-carboxylate (489 mg, 2.8 mmol), potassium carbonate (1.7 g, 12.5 mmol) were added thereto, and the mixture was stirred at 90°C overnight. 10 mL of water was added thereto, and the mixture was extracted with ethyl acetate (20 mL*3). The organic phase was washed with water and saturated brine, dried over anhydrous sodium sulfate. The residue was filtered, evaporated to dryness by rotary evaporation, and the crude product was separated by column chromatography (eluted with petroleum ether/ethyl acetate = 1/1) to obtain *tert*-butyl 3-(5-bromopyridin-2-yl)-3,6-diazabicyclo[3.1.1]heptane-6-carboxylate (650 mg, white solid, yield: 3%).

MS m/z (ESI): 354.0 [M+H]⁺.

### Step 5: 3-(5-bromopyridin-2-yl)-3,6-diazabicyclo[3.1.1]heptane

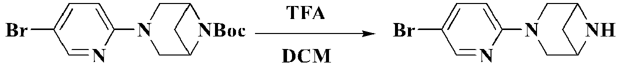

*tert*-Butyl 3-(5-bromopyridin-2-yl)-3,6-diazabicyclo[3.1.1]heptane-6-carboxylate (100 mg, 0.28 mmol) was dissolved in 3 mL of DCM, and 1 mL of TFA was added thereto, and the mixture was stirred at room temperature for 2 hours. The mixture was evaporated to dryness by rotary evaporation, sodium bicarbonate aqueous solution was added thereto to adjust the pH to basic, and extracted with ethyl acetate (20 mL*3). The organic phase was washed with water and saturated brine, dried over anhydrous sodium sulfate. The residue was filtered, evaporated to dryness by rotary evaporation to obtain 3-(5-bromopyridin-2-yl)-3,6-diazabicyclo[3.1.1]heptane (70 mg, white solid, yield: 99%).

MS m/z (ESI): 254.0 [M+H]⁺.

### Step 6: 3-(5-bromopyridin-2-yl)-6-((6-methoxypyridin-3-yl)methyl)-3,6-diazabicyclo[3.1.1]heptane

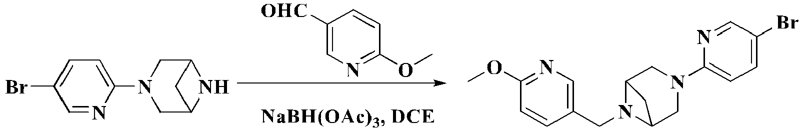

3-(5-Bromopyridin-2-yl)-3,6-diazabicyclo[3.1.1]heptane (70 mg, 0.28 mmol) and 6-methoxynicotinaldehyde (113 mg, 0.83 mmol) were dissolved in 10 mL of DCE, NaBH(OAc)₃ (176 mg, 0.83 mmol) was added thereto, and the mixture was stirred at room temperature overnight. 10 mL of water was added thereto, and the mixture was extracted with ethyl acetate (20 mL*3). The organic phase was washed with water and saturated brine, dried over anhydrous sodium sulfate. The residue was filtered and evaporated to dryness by rotary evaporation, and the crude product was separated by column chromatography (eluted with dichloromethane/methanol = 10/1) to obtain 3-(5-bromopyridin-2-yl)-6-((6-methoxypyridin-3-yl)methyl)-3,6-diazabicyclo[3.1.1]heptane (60 mg, white solid, yield: 57%).

MS m/z (ESI): 375.0 [M+H]⁺.

### Step 7: 6-(2-(methylsulfinyl)ethoxy)-4-(4,4,5,5-tetramethyl-1,3,2-dioxaborolan-2-yl) pyrazolo[1,5-a]pyridine-3-carbonitrile

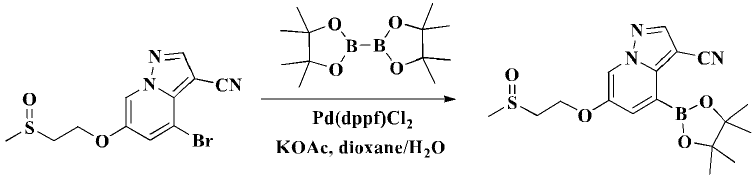

4-Bromo-6-(2-(methylsulfinyl)ethoxy)pyrazolo[1,5-a]pyridine-3-carbonitrile (200 mg, 0.6 mmol), bis(pinacolato)diboron (232 mg, 0.91 mmol), Pd(dppf)Cl₂ (44 mg, 0.06 mmol) and KOAc (176 mg, 1.8 mmol) were dissolved in dioxane/H₂O (20 mL, v/v = 10:1), and the mixture was stirred at 90°C overnight under nitrogen atmosphere. 10 mL of water was added thereto, and the mixture was extracted with ethyl acetate (20 mL*3). The organic phase was washed with water and saturated brine, dried over anhydrous sodium sulfate. The residue was filtered and evaporated to dryness by rotary evaporation, and the crude product was separated by column chromatography (eluted with dichloromethane/methanol = 10/1) to obtain 6-(2-(methylsulfinyl)ethoxy)-4-(4,4,5,5-tetramethyl-1,3,2-dioxaborolan-2-yl)pyrazolo[1,5-a]pyridine-3-carbonitrile (117 mg, white solid, yield: 52%).

MS m/z (ESI): 376.1 [M+H]⁺.

### Step 8: 4-(6-(6-((6-methoxypyridin-3-yl)methyl)-3,6-diazabicyclo[3.1.1]heptan-3-yl)pyridin-3-yl)-6-(2-(methylsulfinyl)ethoxy)pyrazolo[1,5-a]pyridine-3-carbonitrile

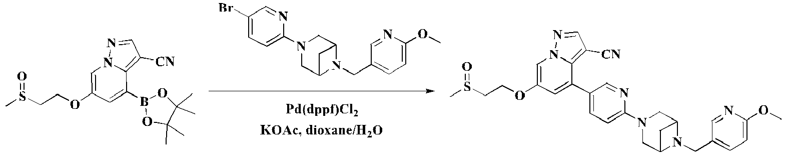

6-(2-(Methylsulfinyl)ethoxy)-4-(4,4,5,5-tetramethyl-1,3,2-dioxaborolan-2-yl)pyrazolo[1,5-a]pyridine-3-carbonitrile (60 mg, 0.16 mmol), 3-(5-bromopyridin-2-yl)-6-((6-methoxypyridin-3-yl)methyl)-3,6-diazabicyclo[3.1.1]heptane (72 mg, 0.19 mmol), Pd(dppf)Cl₂ (15 mg, 0.02 mmol) and KOAc (44 mg, 0.5 mmol) were dissolved in dioxane/H₂O (20 mL, v/v = 10:1), and the mixture was stirred at 90°C overnight under nitrogen atmosphere. 10 mL of water was added thereto, and the mixture was extracted with ethyl acetate (20 mL*3). The organic phase was washed with water and saturated brine, dried over anhydrous sodium sulfate. The residue was filtered and evaporated to dryness by rotary evaporation, the crude product was prepared by prep-HPLC to obtain a product of 4-(6-(6-((6-methoxypyridin-3-yl)methyl)-3,6-diazabicyclo[3 .1.1]heptan-3-yl)pyridin-3-yl)-6-(2-(methylsulfinyl)ethoxy)pyrazolo[1,5-*a*]pyridine-3-carbonitrile (41 mg, white solid, yield: 48%).

MS m/z (ESI): 544.2 [M+H]⁺.

¹H NMR (400 MHz, DMSO) δ 8.82 (d, J = 1.9 Hz, 1H), 8.62 (s, 1H), 8.41 (d, J = 2.3 Hz, 1H), 8.07 (d, J = 1.9 Hz, 1H), 7.85 (dd, J = 8.8, 2.4 Hz, 1H), 7.68 (dd, J = 8.5, 2.2 Hz, 1H), 7.32 (d, J = 2.0 Hz, 1H), 6.78 (t, J = 9.4 Hz, 2H), 4.62 - 4.44 (m, 2H), 3.82 (s, 3H), 3.73 (d, J = 11.6 Hz, 2H), 3.67 (d, J = 5.7 Hz, 2H), 3.58 - 3.52 (m, 2H), 3.50 (s, 2H), 3.13 (dt, J = 13.6, 4.4 Hz, 1H), 2.67 (s, 3H), 2.59 - 2.52 (m, 2H), 1.59 (d, J = 8.5 Hz, 1H).

### Embodiment 2

### 4-(6-(6-((6-Methoxypyridin-3-yl)methyl)-3,6-diazabicyclo[3.1.1]heptan-3-yl)pyridin-3-yl)-6-(2-(methylsulfonyl)ethoxy)pyrazolo[1,5-a]pyridine-3-carbonitrile

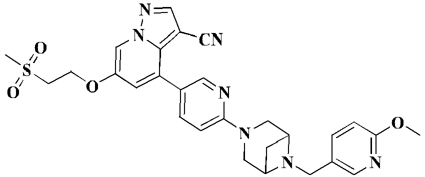

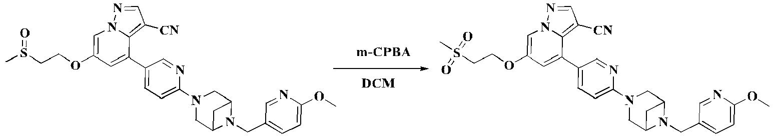

4-(6-(6-((6-Methoxypyridin-3-yl)methyl)-3,6-diazabicyclo[3.1.1]heptan-3-yl)pyridin-3-yl)-6-(2-(methylsulfonyl)ethoxy)pyrazolo[1,5-*a*]pyridine-3-carbonitrile (45 mg, white solid, yield: 70%) was obtained by using 4-(6-(6-((6-methoxypyridin-3-yl)methyl)-3,6-diazabicyclo[3.1.1]heptan-3-yl)pyridin-3-yl)-6-(2-(methylsulfinyl)ethoxy)pyrazolo[1,5-*a*]pyridine-3-carbonitrile as raw material with reference to step 3 of embodiment 1.

MS m/z (ESI): 560.2 [M+H]⁺.

### Embodiment 3

### 4-(6-(6-((6-Methoxypyridin-3-yl)methyl)-3,6-diazabicyclo[3.1.1]heptan-3-yl)pyridin-3-yl)-6-(2-(S-methylsulfonimidoyl)ethoxy)pyrazolo[1,5-a]pyridine-3-carbonitrile

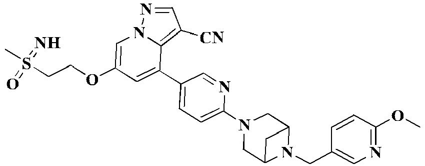

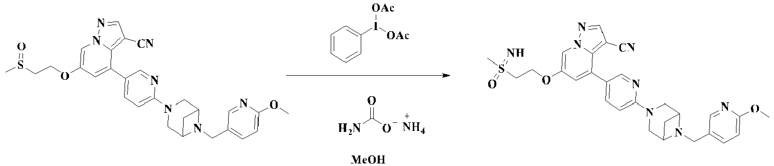

4-(6-(6-((6-Methoxypyridin-3-yl)methyl)-3,6-diazabicyclo[3.1.1]heptan-3-yl)pyridin-3-yl)-6-(2-(methylsulfonyl)ethoxy)pyrazolo[1,5-a]pyridine-3-carbonitrile (60 mg, 0.11 mmol) was dissolved in 5 mL of methanol; and ammonium carbamate (17 mg, 0.22 mmol) and iodobenzene diacetate (70 mg, 0.22 mmol) were added thereto, and the mixture was reacted at room temperature for 2 hours. 10 mL of water was added thereto, and the mixture was extracted with ethyl acetate (10 mL*3). The organic phase was washed with saturated brine, dried over anhydrous sodium sulfate. The residue was filtered and evaporated to dryness by rotary evaporation, the crude product was prepared by prep-HPLC to obtain a product of 4-(6-(6-((6-methoxypyridin-3-yl)methyl)-3,6-diazabicyclo[3.1.1]heptan-3-yl)pyridin-3-yl)-6-(2-(*S*-methylsulfonimidoyl)ethoxy)pyrazolo[1,5-*a*]pyridine-3-carbonitrile (25 mg, white solid, yield: 45%).

MS m/z (ESI): 559.2 [M+H]⁺.

### Embodiment 4

### 4-(6-(6-((6-Methoxypyridin-3-yl)methyl)-3,6-diazabicyclo[3.1.1]heptan-3-yl)pyridin-3-yl)-6-(3-(methylsulfinyl)propoxy)pyrazolo[1,5-a]pyridine-3-carbonitrile

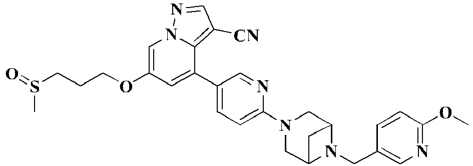

The title compound (35 mg, white solid, 40%) was obtained by using ethyl 3-(methylthio)propionate as raw material with reference to embodiment 1.

MS m/z (ESI): 558.2 [M+H]⁺.

### Embodiment 5

### 4-(6-(6-((6-Methoxypyridin-3-yl)methyl)-3,6-diazabicyclo[3.1.1]heptan-3-yl)pyridin-3-yl)-6-(3-(methylsulfonyl)propoxy)pyrazolo[1,5-a]pyridine-3-carbonitrile

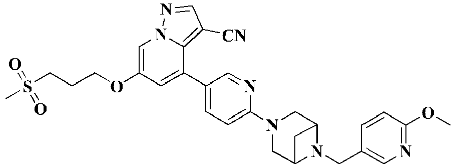

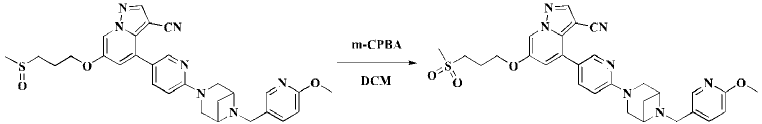

4-(6-(6-((6-Methoxypyridin-3-yl)methyl)-3,6-diazabicyclo[3.1.1]heptan-3-yl)pyridin-3-yl)-6-(3-(methylsulfonyl)propoxy)pyrazolo[1,5-*a*]pyridine-3-carbonitrile (32 mg, white solid, 56%) was obtained by using 4-(6-(6-((6-methoxypyridin-3-yl)methyl)-3,6-diazabicyclo[3.1.1]heptan-3-yl)pyridin-3-yl)-6-(3-(methylsulfinyl)propoxy)pyrazolo[1,5-a]pyridine-3-carbonitrile as raw material with reference to embodiment 2.

MS m/z (ESI): 574.2 [M+H]⁺.

### Embodiment 6

### 4-(6-(6-((6-Methoxypyridin-3-yl)methyl)-3,6-diazabicyclo[3.1.1]heptan-3-yl)pyridin-3-yl)-6-(3-(S-methylsulfonimidoyl)propoxy)pyrazolo[1,5-a]pyridine-3-carbonitrile

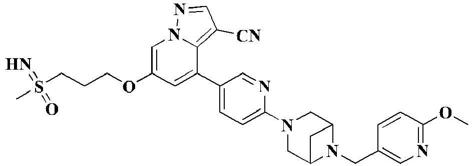

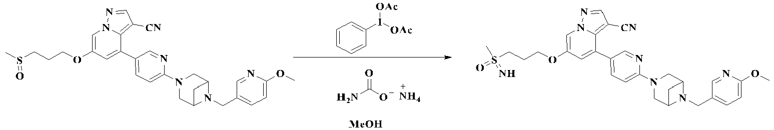

4-(6-(6-((6-Methoxypyridin-3-yl)methyl)-3,6-diazabicyclo[3.1.1]heptan-3-yl)pyridin-3-yl)-6-(3-(*S*-methylsulfonimidoyl)propoxy)pyrazolo[1,5-*a*]pyridine-3-carbonitrile (28 mg, white solid, 44%) was obtained by using 4-(6-(6-((6-methoxypyridin-3-yl)methyl)-3,6-diazabicyclo[3.1.1]heptan-3-yl)pyridin-3-yl)-6-(3-(methylsulfinyl)propoxy)pyrazolo[1,5-a]pyridine-3-carbonitrile as raw material with reference to embodiment 3.

MS m/z (ESI): 573.2 [M+H]⁺.

### Embodiment 7

### 6-(3-(2-Hydroxypropan-2-yl)azetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

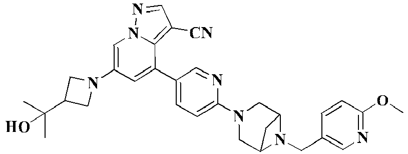

### Step 1: 6-bromo-3-cyanopyrazolo[1,5-a]pyridin-4-yl trifluoromethanesulfonate

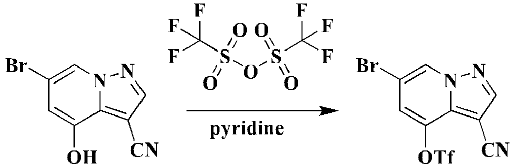

6-Bromo-4-hydroxypyrazolo[1,5-*a*]pyridine-3-carbonitrile, dichloromethane and pyridine were added to a 25 mL single-neck flask sequentially, after the reaction mixture was stirred for 2 minutes, trifluoromethanesulfonic anhydride was slowly added dropwise thereto. The reaction mixture was stirred at room temperature for 12 hours, the reaction mixture was concentrated, dissolved in ethyl acetate and then washed with saturated brine, and the organic phase was dried over anhydrous sodium sulfate, filtered and evaporated to dryness by rotary evaporation. The crude product was separated by column chromatography to obtain a product of 6-bromo-3-cyanopyrazolo[1,5-*a*]pyridin-4-yl trifluoromethanesulfonate.

MS m/z (ESI): 370.0[M+H]⁺, 372.0[M+H+2]⁺ .

### Step 2: 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

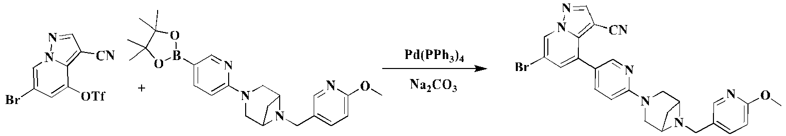

6-Bromo-3-cyanopyrazolo[1,5-*a*]pyridin-4-yl trifluoromethanesulfonate, 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane, tetrakis(triphenylphosphine)palladium, sodium carbonate, and dioxane and water were added to a 25 mL three-necked flask sequentially, and the reaction mixture was replaced with nitrogen 5 times. The reaction mixture was heated to 85°C under nitrogen atmosphere, stirred for 5 hours and then cooled to room temperature; the reaction mixture was concentrated, dissolved in ethyl acetate and then washed with saturated brine, and the organic phase was dried over anhydrous sodium sulfate, filtered and evaporated to dryness by rotary evaporation. The crude product was purified by prep-HPLC to obtain 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile in step 2 of embodiment 7.

MS m/z (ESI): 516.1[M+H]⁺, 518.1[M+H+2]⁺ .

### Step 3: 6-(3-(2-hydroxypropan-2-yl)azetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

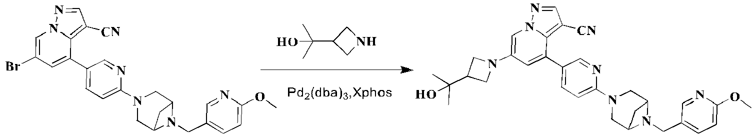

A mixture of 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (80 mg, 0.15 mmol), 2-(azetidin-3-yl)propan-2-ol (36 mg, 0.30 mmol), tris(dibenzylideneacetone)dipalladium (7 mg, 0.0075 mmol), 2-dicyclohexylphosphino-2',4',6'-triisopropylbiphenyl (4 mg, 0.0075 mmol), cesium carbonate (146 mg, 0.45 mmol) and toluene (4 mL) was replaced with nitrogen, then stirred at 130°C for 2 hours under microwave irradiation. After the reaction was completed, the reaction mixture was cooled to room temperature, concentrated, dissolved in ethyl acetate (20 mL) and washed with saturated brine (15 mL); the organic phase was dried over anhydrous sodium sulfate, filtered, evaporated to dryness by rotary evaporation, and then purified by preparative chromatography (18 mg, white solid, yield: 21%).

MS m/z (ESI): 551.2[M+H]⁺ .

¹H NMR (400 MHz, MeOD) δ 8.32 (d, *J* = 2.1 Hz, 1H), 8.24 (s, 1H), 8.08 (s, 1H), 7.85 - 7.79 (m, 2H), 7.71 (dd, *J* = 8.5, 2.3 Hz, 1H), 6.92 (d, *J* = 1.7 Hz, 1H), 6.86 (d, *J* = 8.8 Hz, 1H), 6.78 (d, *J* = 8.5 Hz, 1H), 3.97 (t, *J* = 7.8 Hz, 2H), 3.92 - 3.84 (m, 7H), 3.78 (d, *J* = 5.6 Hz, 2H), 3.65 (s, 1H), 3.62 (s, 3H), 2.95 - 2.81 (m, 1H), 2.70 (d, *J* = 7.0 Hz, 1H), 1.70 (d, *J* = 8.8 Hz, 1H), 1.21 (s, 6H).

### Embodiment 8

### 6-(3-Hydroxy-3-methylbut-1-yn-1-yl)-4-(6-(6-((6-methoxypyridin-3-yl)methyl)-3,6-diazabicyclo[3.1.1] heptan-3-yl)pyridin-3-yl)pyrazolo [1,5-a]pyridine-3-carbonitrile

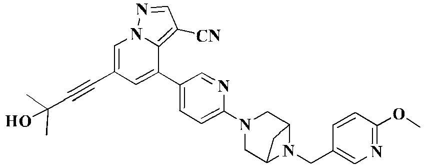

### Step 1: 6-bromo-4-(4,4,5,5-tetramethyl-1,3,2-dioxaborolan-2-yl)pyrazolo[1,5-a]pyridine-3-carbonitrile

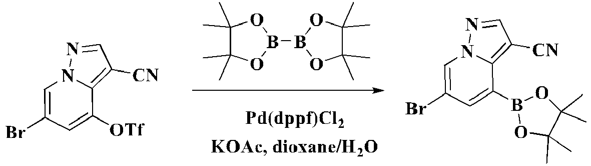

6-Bromo-4-(4,4,5,5-tetramethyl-1,3,2-dioxaborolan-2-yl)pyrazolo[1,5-*a*]pyridine-3-carbonitrile (620 mg, white solid, 56%) was obtained by using 6-bromo-3-cyanopyrazolo[1,5-*a*]pyridin-4-yl trifluoromethanesulfonate as raw material with reference to step 7 of embodiment **1**.

MS m/z (ESI): 348.0 [M+H]⁺.

### Step 2: 6-(3-hydroxy-3-methylbut-1-yn-1-yl)-4-(4,4,5,5-tetramethyl-1,3,2-dioxaborolan-2-yl)pyrazolo[1,5-a]pyridine-3-carbonitrile

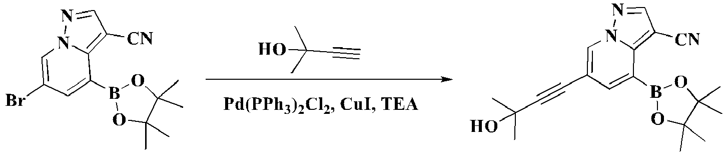

6-Bromo-4-(4,4,5,5-tetramethyl-1,3,2-dioxaborolan-2-yl)pyrazolo[1,5-*a*]pyridine-3-carbonitrile (300 mg, 0.86 mmol) was dissolved in 20 mL of triethylamine, then 2-methylbut-3-yn-2-ol (108 mg, 1.3 mmol), Pd₂(PPh₃)₂Cl₂ (120 mg, 0.17 mmol) and CuI (17 mg, 0.09 mmol) were added thereto, and the reaction was carried out at 65°C overnight under nitrogen atmosphere. 10 mL of ammonium chloride aqueous solution was added, and the mixture was extracted with ethyl acetate (20 mL*3). The organic phase was washed with saturated brine, dried over anhydrous sodium sulfate. The residue was filtered and evaporated to dryness by rotary evaporation, and the crude product was separated by column chromatography (eluted with dichloromethane/methanol = 10/1) to obtain 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile (197 mg, white solid, yield: 65%).

MS m/z (ESI): 352.1 [M+H]⁺.

### Step 3: 6-(3-hydroxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

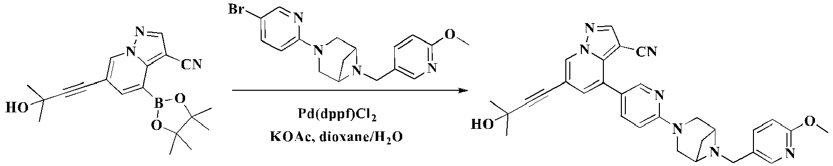

6-(3-Hydroxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (25 mg, white solid, 43%) was obtained by using 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile and 3-(5-bromopyridin-2-yl)-6-((6-methoxypyridin-3-yl)methyl)-3,6-diazabicyclo[3.1.1]heptane as raw materials with reference to the step 8 of embodiment **1**.

MS m/z (ESI): 520.2 [M+H]⁺.

¹H NMR (400 MHz, MeOD) δ 8.83 (s, 1H), 8.47 (s, 1H), 8.35 (d, J = 2.1 Hz, 1H), 8.09 (s, 1H), 7.84 (dd, J = 8.8, 2.3 Hz, 1H), 7.72 (dd, J = 8.4, 2.1 Hz, 1H), 7.41 (s, 1H), 6.88 (d, J = 8.9 Hz, 1H), 6.78 (d, J = 8.5 Hz, 1H), 3.91 (s, 1H), 3.88 (s, 4H), 3.79 (d, J = 5.7 Hz, 2H), 3.66 (s, 1H), 3.63 (s, 3H), 2.74 - 2.62 (m, 1H), 1.70 (d, J = 8.9 Hz, 1H), 1.59 (s, 6H).

### Embodiment 9

### 6-(2-Cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

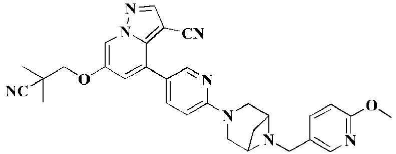

### Step 1: 4-bromo-6-(2-cyano-2-methylpropoxy)pyrazolo[1,5-a]pyridine-3-carbonitrile

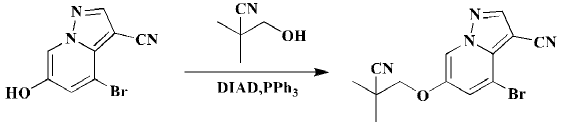

4-Bromo-6-hydroxypyrazolo[1,5-a]pyridine-3-carbonitrile (200 mg, 0.84 mmol) and 3-hydroxy-2,2-dimethylpropanenitrile (83 mg, 0.84 mmol) were dissolved in 5 mL of anhydrous tetrahydrofuran solution, and then triphenylphosphine (330 mg, 1.26 mmol) and diisopropyl azodicarboxylate (202 mg, 1 mmol) were added thereto, and the reaction mixture was stirred at 0°C for 12 hours under nitrogen atmosphere. The reaction mixture was concentrated, dissolved in ethyl acetate (10 mL), washed three times with water (5 mL*3), and the organic phase was concentrated and then separated by column chromatography (dichloromethane/methanol: 30/1) and purified to obtain a product of 4-bromo-6-(2-cyano-2-methylpropoxy)pyrazolo[1,5-*a*]pyridine-3-carbonitrile (180 mg, yellow solid, yield: 67.1%).

MS m/z (ESI):319.0 [M+H]⁺. 321.0 [M+H+2]⁺.

### Step 2: 6-(2-cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

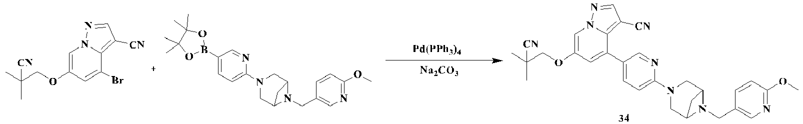

The product 6-(2-cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-bromo-6-(2-cyano-2-methylpropoxy)pyrazolo[1,5-*a*]pyridine-3-carbonitrile as raw material with reference to step 2 of embodiment 7.

MS m/z (ESI):535.2[M+H]⁺ .

¹H NMR (400 MHz, Chloroform-d) δ 8.43 (d, J = 2.5 Hz, 1H), 8.23 (s, 1H), 8.16 (d, J = 2.1 Hz, 1H), 8.13 (d, J = 2.4 Hz, 1H), 7.81-7.75 (m, 2H), 7.19 (d, J = 2.1 Hz, 1H), 6.75 (d, J = 8.5 Hz, 1H), 6.70 (d, J = 8.8 Hz, 1H), 3.97 (s, 3H), 3.96 - 3.86 (m, 6H), 3.78 - 3.64 (m, 4H), 2.94 - 2.80 (m, 1H), 1.78 - 1.72 (m, 1H), 1.55 (s, 6H).

### Embodiment 10

### 4-(6-(6-((6-Methoxypyridin-3-yl)methyl)-3,6-diazabicyclo[3.1.1]heptan-3-yl)pyridin-3-yl)-6-(2-(methylsulfonyl)ethyl)pyrazolo[1,5-a]pyridine-3-carbonitrile

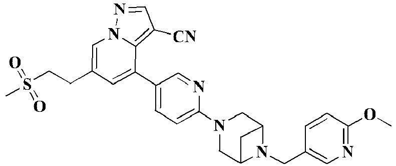

### Step 1: 3-bromo-5-(2-(methylsulfonyl)ethyl)pyridine

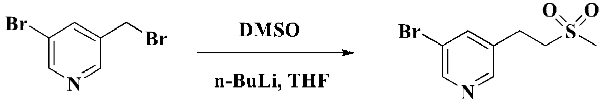

DMSO (780 mg, 10 mmol) was dissolved in 10 mL of THF, *n*-BuLi (4 mL, 10 mmol) was added thereto at -78°C, and the mixture was stirred at -78°C for 0.5 hour; 3-bromo-5-(bromomethyl)pyridine (500 mg, 2 mmol) was added thereto at -78°C, the temperature was slowly raised to room temperature and the mixture was stirred for 2 hours, 10 mL of ammonium chloride aqueous solution was added thereto, and the mixture was extracted with ethyl acetate (20 mL*3). The organic phase was washed with saturated brine, dried over anhydrous sodium sulfate. The residue was filtered and evaporated to dryness by rotary evaporation, and the crude product was separated by column chromatography (eluted with dichloromethane/methanol = 10/1) to obtain 3-bromo-5-(2-(methylsulfonyl)ethyl)pyridine (252 mg, yield: 48%).

MS m/z (ESI): 263.9 [M+H]⁺.

### Step 2: 4-bromo-6-(2-(methylsulfonyl)ethyl)pyrazolo[1,5-a]pyridine-3-carbonitrile

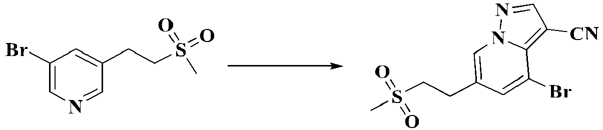

1.8-Diazabicyclo[5.4.0]undec-7-ene was added to a solution of 3-bromo-5-(2-(methylsulfonyl)ethyl)pyridine and 2-chloroacrylonitrile in dichloromethane in batches, then the mixture was heated to room temperature and stirred for 24 hours; methyl *tert*-butyl ether was added to the reaction mixture, and the mixture was slurried at room temperature for 15 minutes, then filtered and the filter cake was dried to obtain 4-bromo-6-(2-(methylsulfonyl)ethyl)pyrazolo[1,5-*a*]pyridine-3-carbonitrile (350 mg, white solid).

MS m/z (ESI): 327.9 [M+H]⁺.

### Step 3: 6-(2-(methylsulfonyl)ethyl)-4-(4,4,5,5-tetramethyl-1,3,2-dioxaborolan-2-yl)pyrazolo[1,5-a]pyridine-3-carbonitrile

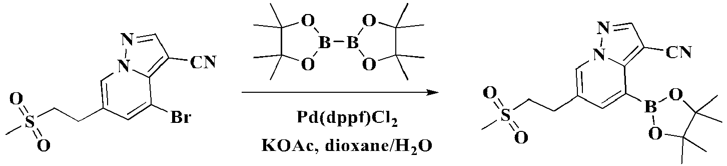

6-(2-(Methylsulfonyl)ethyl)-4-(4,4,5,5-tetramethyl-1,3,2-dioxaborolan-2-yl)pyrazolo[1,5-*a*]pyridine-3-carbonitrile (230 mg, white solid, 68%) was obtained by using 4-bromo-6-(2-(methylsulfonyl)ethyl)pyrazolo[1,5-*a*]pyridine-3-carbonitrile as raw material with reference to step 7 of embodiment **1**.

MS m/z (ESI): 376.1 [M+H]⁺.

### Step 4: 4-(6-(6-((6-methoxypyridin-3-yl)methyl)-3,6-diazabicyclo[3.1.1]heptan-3-yl)pyridin-3-yl)-6-(2-(methylsulfonyl)ethyl)pyrazolo[1,5-a]pyridine-3-carbonitrile

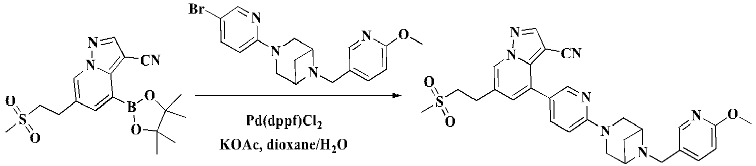

4-(6-(6-((6-Methoxypyridin-3-yl)methyl)-3,6-diazabicyclo[3.1.1]heptan-3-yl)pyridin-3-yl)-6-(2-(methylsulfonyl)ethyl)pyrazolo[1,5-*a*]pyridine-3-carbonitrile (30 mg, white solid, 48%) was obtained by using 6-(2-(methylsulfonyl)ethyl)-4-(4,4,5,5-tetramethyl-1,3,2-dioxaborolan-2-yl)pyrazolo[1,5-*a*]pyridine-3-carbonitrile as raw material with reference to step 8 of embodiment **1.**

MS m/z (ESI): 544.2 [M+H]⁺.

### Embodiment 11

### 6-(3-Hydroxy-3-methylbutyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

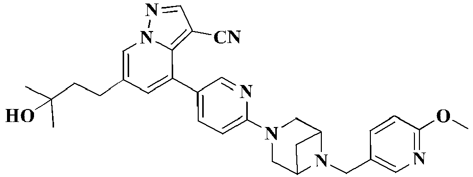

The product 6-(3-hydroxy-3-methylbutyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (30 mg, white solid) was obtained with reference to embodiment 10.

MS m/z (ESI): 524.2 [M+H]⁺.

### Embodiment 12

### 6-((1-Imino-1-hydroxy-1l6-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

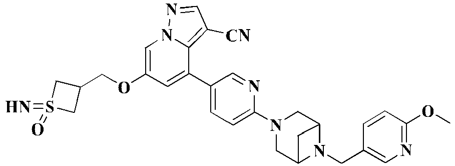

### Step 1: thietan-3-ylmethanol

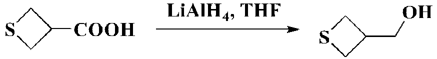

Thietane-3-carboxylic acid (500 mg, 4.2 mmol) was dissolved in 10 mL of THF, LiAlH₄ (8.4 mL, 8.4 mmol) was added thereto at -78°C; and the mixture was stirred at -78°C for 2 hours, the temperature was slowly raised to room temperature and the mixture was stirred for 2 hours, then 10 mL of ammonium chloride aqueous solution was added thereto, and the mixture was extracted with ethyl acetate (20 mL*3). The organic phase was washed with saturated brine and dried over anhydrous sodium sulfate. The residue was filtered and evaporated to dryness by rotary evaporation to obtain a crude product of thietan-3-ylmethanol (314 mg, yield: 72%).

### Step 2: 4-bromo-6-(thietan-3-ylmethoxy)pyrazolo[1,5-a]pyridine-3-carbonitrile

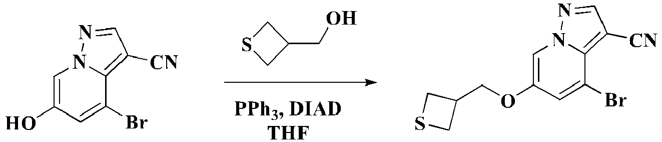

4-Bromo-6-(thietan-3-ylmethoxy)pyrazolo[1,5-a]pyridine-3-carbonitrile (210 mg, white solid, 67%) was obtained by using 4-bromo-6-hydroxypyrazolo[1,5-a]pyridine-3-carbonitrile and thietan-3-ylmethanol as raw materials with reference to step 2 of embodiment **1.**

MS m/z (ESI): 323.9 [M+H]⁺.

### Step 3: 4-bromo-6-((1-hydroxythietan-3-yl)methoxy)pyrazolo[1,5-a]pyridine-3-carbonitrile

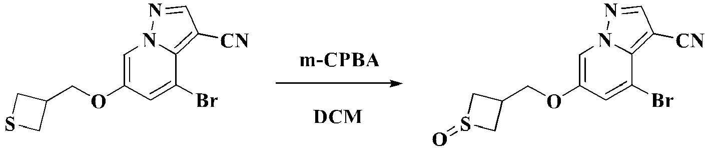

4-Bromo-6-((1-hydroxythietan-3-yl)methoxy)pyrazolo[1,5-*a*]pyridine-3-carbonitrile (180 mg, white solid, 85%) was obtained by using 4-bromo-6-(thietan-3-ylmethoxy)pyrazolo[1,5-*a*]pyridine-3-carbonitrile as raw material with reference to step 3 of embodiment 1.

MS m/z (ESI): 339.9 [M+H]⁺.

### Step 4: 4-bromo-6-((1-imino-1-hydroxy-116-thietan-3-yl)methoxy)pyrazolo[1,5-a]pyridine-3-carbonitrile

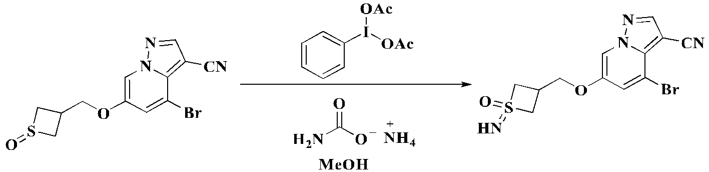

4-Bromo-6-((1-imino-1-hydroxy-116-thietan-3-yl)methoxy)pyrazolo[1,5-a]pyridine-3-carbonitrile (150 mg, white solid, 79%) was obtained by using 4-bromo-6-((1-hydroxythietan-3-yl)methoxy)pyrazolo[1,5-*a*]pyridine-3-carbonitrile as raw material with reference to embodiment **3.**

MS m/z (ESI): 354.9 [M+H]⁺.

### Step 5: 6-((1-imino-1-hydroxy-116-thietan-3-yl)methoxy)-4-(4,4,5,5-tetramethyl-1,3,2-dioxaborolan-2-yl)pyrazolo[1,5-a]pyridine-3-carbonitrile

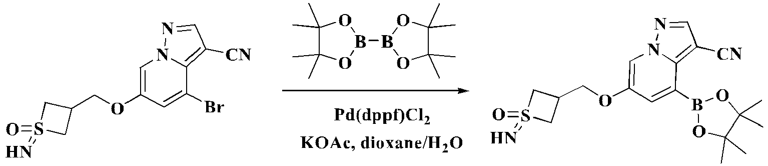

6-((1-Imino-1-hydroxy-1l6-thietan-3-yl)methoxy)-4-(4,4,5,5-tetramethyl-1,3,2-dioxaborolan-2-yl)pyrazolo[1,5-*a*]pyridine-3-carbonitrile (96 mg, white solid, 64%) was obtained by using 4-bromo-6-((1-imino-1-hydroxy-116-thietan-3-yl)methoxy)pyrazolo[1,5-a]pyridine-3-carbonitrile as raw material with reference to step 7 of embodiment **1.**

MS m/z (ESI): 403.1 [M+H]⁺.

### Step 6: 6-((1-imino-1-hydroxy-116-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

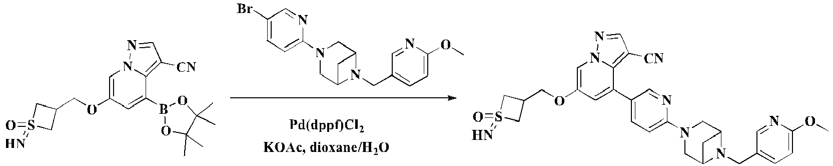

6-((1-Imino-1-hydroxy-116-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (30 mg, white solid, 34%) was obtained by using 6-((1-imino-1-hydroxy-1l6-thietan-3-yl)methoxy)-4-(4,4,5,5-tetramethyl-1,3,2-dioxaborolan-2-yl)pyrazolo[1,5-*a*]pyridine-3-carbonitrile as raw material with reference to step 8 of embodiment **1.**

MS m/z (ESI): 571.2 [M+H]⁺.

¹H NMR (400 MHz, MeOD) δ 8.53 (s, 1H), 8.38 (s, 1H), 8.36 (s, 1H), 8.19 (s, 1H), 7.87 (d, *J* = 8.8 Hz, 1H), 7.77 (d, *J* = 8.7 Hz, 1H), 7.32 (s, 1H), 6.90 (d, *J* = 8.7 Hz, 1H), 6.83 (d, *J* = 8.7 Hz, 1H), 4.43 - 4.22 (m, 4H), 4.21 - 4.05 (m, 4H), 4.06 - 3.94 (m, 5H), 3.91 (s, 3H), 3.67 - 3.59 (m, 1H), 3.22 - 3.11 (m, 1H), 2.25 - 1.88 (m, 2H).

### Embodiment 13

### 6-(2-(1-Imino-1-hydroxy-1l6-thietan-3-yl)ethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

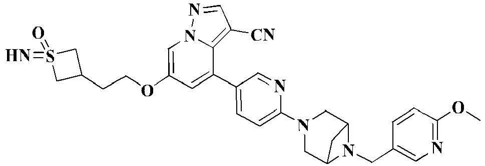

6-(2-(1-Imino-1-hydroxy-1l6-thietan-3-yl)ethoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (35 mg, white solid) was obtained by using 2-(thietan-3-yl)ethan-1-ol as raw material with reference to embodiment **12.**

MS m/z (ESI): 585.2 [M+H]⁺.

### Embodiment 14

### 4-(6-(6-((6-Methoxypyridin-3-yl)methyl)-3,6-diazabicyclo[3.1.1]heptan-3-yl)pyridin-3-yl)-6-(2-(1-hydroxytetrahydro-2H-thiopyran-4-yl)ethoxy)pyrazolo[1,5-a]pyridine-3-carbonitrile

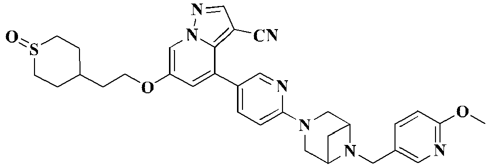

4-(6-(6-((6-Methoxypyridin-3-yl)methyl)-3,6-diazabicyclo[3.1.1]heptan-3-yl)pyridin-3-yl)-6-(2-(1-hydroxytetrahydro-2*H*-thiopyran-4-yl)ethoxy)pyrazolo[1,5-*a*]pyridine-3-carbonitrile (29 mg, white solid) was obtained by using 2-(tetrahydro-2*H-*thiopyran-4-yl)ethan-1-ol as raw material with reference to embodiment **12.**

MS m/z (ESI): 598.2 [M+H]⁺.

### Embodiment 15

### 6-(2-(1-Imino-1-hydroxyhexahydro-1l6-thiopyran-4-yl)ethoxy)-4-(6-(6-((6-methoxypyridin-3-yl)methyl)-3,6-diazabicyclo [3.1.1] heptan-3-yl)pyridin-3-yl)pyrazolo[1,5-a]pyridine-3-carbonitrile

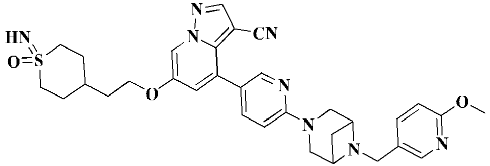

6-(2-(1-Imino-1-hydroxyhexahydro-116-thiopyran-4-yl)ethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (18 mg, white solid) was obtained by using 4-(6-(6-((6-methoxypyridin-3-yl)methyl)-3,6-diazabicyclo[3.1.1]heptan-3-yl)pyridin-3-yl)-6-(2-(1-hydroxytetrahydro-2*H*-thiopyran-4-yl)ethoxy)pyrazolo[1,5-*a*]pyridine-3-carbonitrile as raw material with reference to embodiment **3.**

MS m/z (ESI): 613.2 [M+H]⁺.

### Embodiment 16

### 6-((1-Imino-1-hydroxyhexahydro-116-thiopyran-4-yl)oxo)-4-(6-(6-((6-methoxypyridin-3-yl)methyl)-3,6-diazabicyclo [3.1.1] heptan-3-yl)pyridin-3-yl)pyrazolo[1,5-a]pyridine-3-carbonitrile

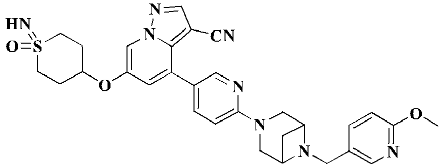

### Step 1: preparation of 4-bromo-6-((tetrahydro-2H-thiopyran-4-yl)oxo)pyrazolo[1,5-a]pyridine-3-carbonitrile

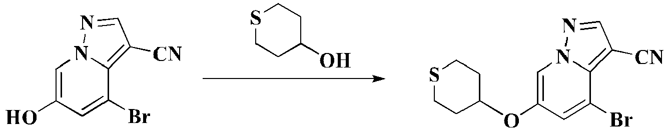

4-Bromo-6-hydroxypyrazolo[1,5-a]pyridine-3-carbonitrile (10 g, 42.0 mmol) was dissolved in tetrahydrofuran (100 mL), and tetrahydro-2*H*-thiopyran-4-ol (6 g, 50.4 mmol) and triphenylphosphine (22 g, 84.0 mmol) were added thereto. DEAD (14.6 g, 84.0 mmol) was slowly added dropwise to the reaction mixture. The reaction was stirred at room temperature overnight. Water was added to quench the reaction, and then the mixture was extracted with ethyl acetate. The organic phase was dried and then evaporated to dryness by rotary evaporation. The crude product was purified by column chromatography to obtain 4-bromo-6-((tetrahydro-2*H*-thiopyran-4-yl)oxo)pyrazolo[1,5-*a*]pyridine-3-carbonitrile (7 g, yield: 49%).

MS m/z (ESI): 337.9[M+H]⁺.

### Step 2: preparation of 4-bromo-6-((1-imino-1-hydroxyhexahydro-116-thiopyran-4-yl)oxo)pyrazolo[1,5-a]pyridine-3-carbonitrile

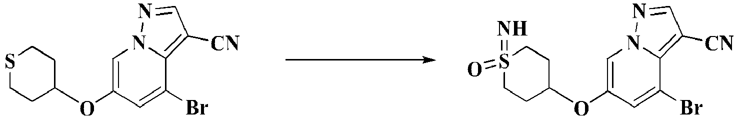

4-Bromo-6-((tetrahydro-2*H*-thiopyran-4-yl)oxo)pyrazolo[1,5-*a*]pyridine-3-carbonitrile (3 g, 8.9 mmol) was dissolved in methanol (40 mL), and ammonium carbonate (1.6 g, 16.9 mmol) and (diacetoxyiodo)benzene (5.7 g, 17.8 mmol) were added thereto. The reaction was stirred at room temperature overnight. The reaction mixture was evaporated to dryness by rotary evaporation. The crude product was purified by column chromatography to obtain 4-bromo-6-((1-imino-1-hydroxyhexahydro-1l6-thiopyran-4-yl)oxo)pyrazolo[1,5-*a*]pyridine-3-carbonitrile (350 mg, yield: 11%).

MS m/z (ESI): 368.9[M+H]⁺.

### Step 3: preparation of 6-((1-imino-1-hydroxyhexahydro-1l6-thiopyran-4-yl)oxo)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

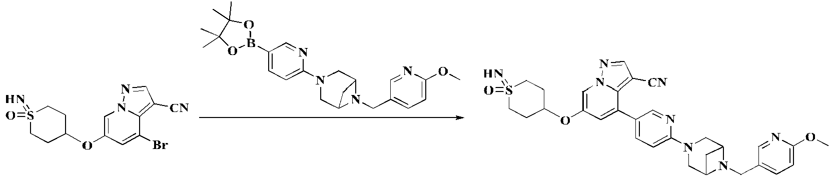

6-((1-Imino-1-hydroxyhexahydro-116-thiopyran-4-yl)oxo)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (35 mg, yield: 36%) was obtained by using 4-bromo-6-((1-imino-1-hydroxyhexahydro-116-thiopyran-4-yl)oxo)pyrazolo[1,5-*a*]pyridine-3-carbonitrile and 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane as raw materials with reference to step 8 of embodiment 1.

MS m/z (ESI): 585.2[M+H]⁺.

¹H NMR (400 MHz, DMSO-d6) δ 8.89 (dd, J = 7.6, 2.0 Hz, 1H), 8.62 (s, 1H), 8.42 (t, J = 2.4 Hz, 1H), 8.07 (s, 1H), 7.85 (d, J = 8.6 Hz, 1H), 7.72 - 7.60 (m, 1H), 7.51 - 7.40 (m, 1H), 6.78 (t, J = 9.4 Hz, 2H), 4.90 - 4.78 (m, 1H), 3.82 (s, 3H), 3.79 - 3.61 (m, 4H), 3.60 - 3.45 (m, 4H), 3.27 - 3.13 (m, 2H), 3.13 - 2.98 (m, 2H), 2.65 - 2.53 (m, 1H),2.31 - 2.14 (m, 3H), 2.13 - 1.92 (m, 1H), 1.59 (d, J = 8.4 Hz, 1H), 0.84 - 0.69 (m, 1H).

### Embodiment 17

### 6-(3-Amino-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

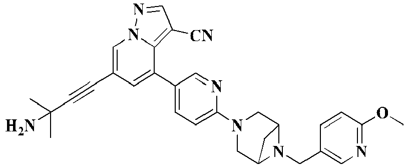

6-(3-Amino-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 2-methylbut-3-yn-2-amine as raw material with reference to embodiment 7.

MS m/z (ESI): 519.2 [M+H]⁺.

¹H NMR (400 MHz, Methanol-d₄) δ 8.82 (d, J = 1.3 Hz, 1H), 8.46 (s, 1H), 8.34 (d, J = 2.4 Hz, 1H), 8.09 (d, J = 2.3 Hz, 1H), 7.84 (dd, J = 8.9, 2.5 Hz, 1H), 7.72 (dd, J = 8.5, 2.5 Hz, 1H), 7.41 (d, J = 1.4 Hz, 1H), 6.88 (d, J = 8.9 Hz, 1H), 6.78 (d, J = 8.5 Hz, 1H), 4.01 - 3.83 (m, 5H), 3.84 - 3.73 (m, 2H), 3.72 - 3.56 (m, 4H), 2.78 - 2.65 (m, 1H), 1.71 (d, J = 9.0 Hz, 1H), 1.52 (s, 6H).

### Embodiment 18

### 4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

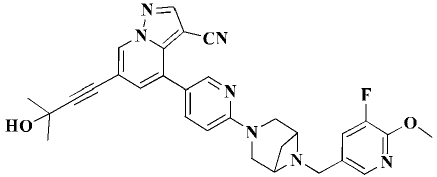

### Step 1: 6-bromo-3-cyanopyrazolo[1,5-a]pyridin-4-yl trifluoromethanesulfonate

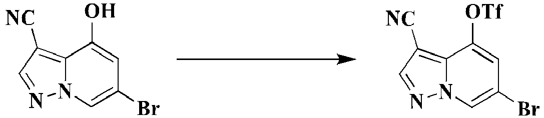

6-Bromo-4-hydroxypyrazolo[1,5-*a*]pyridine-3-carbonitrile (5 g, 21 mmol) and triethylamine (4.2 g, 42 mmol) were dissolved in dichloromethane (500 mL), and trifluoromethanesulfonic anhydride (8.9 g, 31.5 mmol) was added thereto under an ice bath; then the mixture was stirred at room temperature for 12 hours, 100 mL of water was added thereto, and the mixture was extracted with ethyl acetate (80 mL*3). The organic phase was washed with saturated brine, dried over anhydrous sodium sulfate, filtered and evaporated to dryness by rotary evaporation, and the crude product was separated by column chromatography (petroleum ether/ethyl acetate = 1/1) to obtain 6-bromo-3-cyanopyrazolo[1,5-*a*]pyridin-4-yl trifluoromethanesulfonate (5 g, white solid, yield: 64%).

¹H NMR (400 MHz, DMSO) δ 9.60 (d, *J* = 0.9 Hz, 1H), 8.85 (s, 1H), 8.23 (s, 1H).

### Step 2: 6-bromo-4-(6-fluoropyridin-3-yl)pyrazolo[1,5-a]pyridine-3-carbonitrile

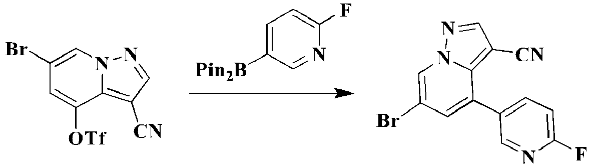

1,1'-Bis(diphenylphosphino)ferrocene-palladium(II)dichloride dichloromethane complex (494 mg, 0.68 mmol) was added to a mixture solution of 6-bromo-3-cyanopyrazolo[1,5-*a*]pyridin-4-yl trifluoromethanesulfonate (5 g, 13.51 mmol), 2-fluoro-5-(4,4,5,5-tetramethyl-1,3,2-dioxaborolan-2-yl)pyridine (3.3 g, 14.86 mmol), potassium carbonate (3.7 g, 27 mmol) and dioxane (100 mL), the mixture was replaced with nitrogen three times and then stirred for 16 hours at 70°C under nitrogen atmosphere. After the reaction was completed, the reaction mixture was cooled and filtered, the filtrate was concentrated under reduced pressure to dryness and separated by column chromatography (dichloromethane/methanol = 10:1) to obtain a white solid of 6-bromo-4-(6-fluoropyridin-3-yl)pyrazolo[1,5-*a*]pyridine-3-carbonitrile (3 g, yield: 70%).

¹H NMR (400 MHz, DMSO) δ 9.49 (d, *J* = 1.3 Hz, 1H), 8.73 (s, 1H), 8.51 (d, *J* = 2.0 Hz, 1H), 8.27 (td, *J* = 8.2, 2.5 Hz, 1H), 7.86 (d, *J* = 1.2 Hz, 1H), 7.40 (dd, *J* = 8.5, 2.5 Hz, 1H).

MS m/z (ESI): 317.0 [M+H]⁺.

### Step 3: tert-butyl 3-(5-(6-bromo-3-cyanopyrazolo[1,5-a]pyridin-4-yl)pyridin-2-yl)-3,6-diazabicyclo[3.1.1]heptane-6-carboxylate

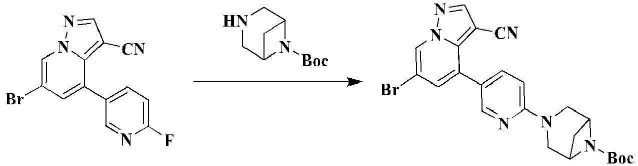

*tert*-Butyl 3-(5-(6-bromo-3-cyanopyrazolo[1,5-*a*]pyridin-4-yl)pyridin-2-yl)-3,6-diazabicyclo[3.1.1]heptane-6-carboxylate was obtained by using 6-bromo-4-(6-fluoropyridin-3-yl)pyrazolo[1,5-*a*]pyridine-3-carbonitrile as raw material with reference to step 7 of embodiment 11.

MS m/z (ESI):495.1 [M+H]⁺.

### Step 4: 4-(6-(3,6-diazabicyclo[3.1.1]heptan-3-yl)pyridin-3-yl)-6-bromopyrazolo[1,5-a]pyridine-3 -carbonitrile

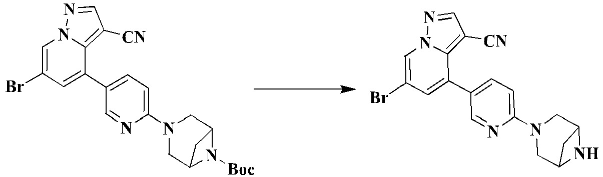

4-(6-(3,6-Diazabicyclo[3.1.1]heptan-3-yl)pyridin-3-yl)-6-bromopyrazolo[1,5-*a*]pyridine-3-carbonitrile was obtained by using tert-butyl 3-(5-(6-bromo-3-cyanopyrazolo[1,5-*a*]pyridin-4-yl)pyridin-2-yl)-3,6-diazabicyclo[3.1.1]heptane-6-carboxylate as raw material with reference to step 8 of embodiment 11.

MS m/z (ESI): 395.1 [M+H]⁺.

### Step 5: 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

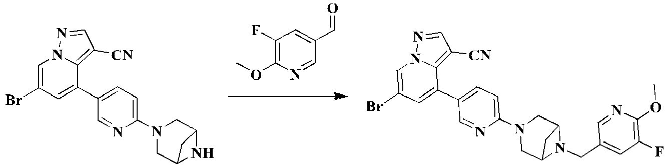

6-Bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile was obtained by using 4-(6-(3,6-diazabicyclo[3.1.1]heptan-3-yl)pyridin-3-yl)-6-bromopyrazolo[1,5-*a*]pyridine-3-carbonitrile and 5-fluoro-6-methoxynicotinaldehyde as raw materials with reference to step 9 of embodiment 11.

MS m/z (ESI):534.1 [M+H]⁺.

### Step 6: 4-(6-(6-((5-fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

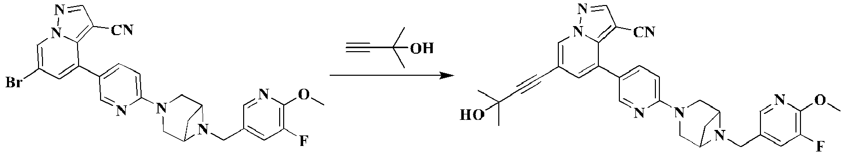

4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-*a*]pyridine-3-carbonitrile was obtained by using 2-methylbut-3-yn-2-ol and 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile as raw materials with reference to step 2 of embodiment 8.

MS m/z (ESI):538.2 [M+H]⁺.

¹H NMR (400 MHz, CDCl₃) δ 8.61 (s, 1H), 8.40 (d, J = 2.2 Hz, 1H), 8.31 (s, 1H), 7.86 (s, 1H), 7.76 (dd, J = 8.8, 2.4 Hz, 1H), 7.47 (d, J = 11.1 Hz, 1H), 7.30 (s, 1H), 6.68 (d, J = 8.7 Hz, 1H), 4.01 (s, 3H), 3.80 (s, 4H), 3.59 (s, 4H), 2.78 - 2.53 (m, 2H), 1.65 (s, 6H).

### Embodiment 19

### 6-(3-Cyclopropyl-3-hydroxyprop-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

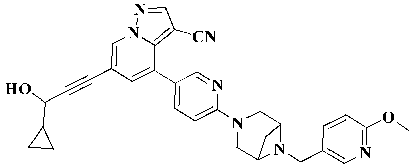

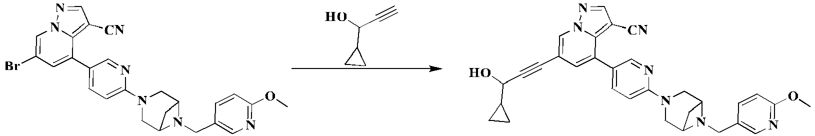

6-Bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (50 mg, 0.1 mmol) was dissolved in N,N-dimethylformamide (2 mL), then 1-cyclopropylprop-2-yn-1-ol (47 mg, 0.5 mmol), triethylamine (50 mg, 0.5 mmol), Pd₂(PPh₃)₂Cl₂ (7 mg, 0.01 mmol) and CuI (1 mg, 0.01 mmol) were added thereto, the reaction was carried out at 65°C overnight under nitrogen atmosphere. 5 mL of ammonium chloride aqueous solution was added thereto, and the mixture was extracted with ethyl acetate (3 mL*3). The organic phase was washed with saturated brine, dried over anhydrous sodium sulfate. The residue was filtered and evaporated to dryness by rotary evaporation, and the crude product was separated by column chromatography (eluted with dichloromethane/methanol = 10/1) to obtain 6-(3-cyclopropyl-3-hydroxyprop-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (15 mg, white solid, yield: 29%).

MS m/z (ESI): 532.2 [M+H]⁺.

### Embodiment 20

### 6-((1-Cyanocyclopentyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

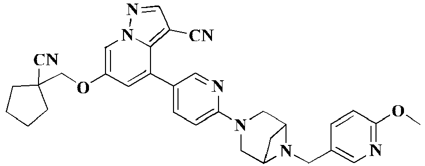

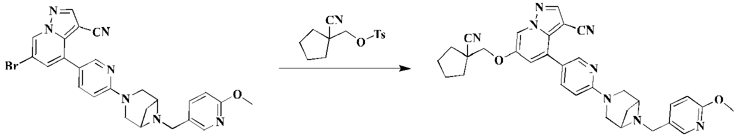

6-((1-Cyanocyclopentyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopentyl)methyl 4-methylbenzenesulfonate as raw materials with reference to step 2 of embodiment 27.

MS m/z (ESI): 561.2 [M+H]⁺.

### Embodiment 21

### 6-((S)-3-Hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

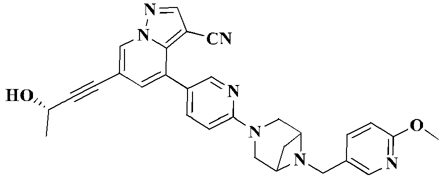

6-((*S*)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (*S*)-but-3-yn-2-ol as raw materials with reference to step 2 of embodiment 20.

MS m/z (ESI): 506.2 [M+H]⁺.

¹H NMR (400 MHz, Methanol-d4) δ 8.85 (s, 1H), 8.47 (s, 1H), 8.35 (d, J = 2.5 Hz, 1H), 8.08 (d, J = 2.5 Hz, 1H), 7.84 (dd, J = 8.9, 2.6 Hz, 1H), 7.72 (dd, J = 8.5, 2.5 Hz, 1H), 7.42 (s, 1H), 6.88 (d, J = 8.9 Hz, 1H), 6.78 (d, J = 8.6 Hz, 1H), 4.72 (q, J = 6.6 Hz, 1H), 3.96 - 3.84 (m, 5H), 3.83 - 3.74 (m, 2H), 3.69 - 3.56 (m, 4H), 2.76 - 2.64 (m, 1H), 1.70 (d, J = 8.9 Hz, 1H), 1.51 (d, J = 6.6 Hz, 3H).

### Embodiment 22

### 6-((R)-3-Hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

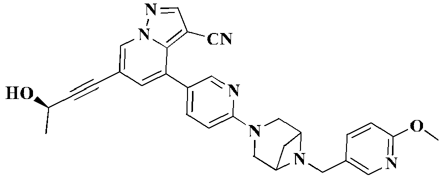

6-((*R*)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (*S*)-but-3-yn-2-ol as raw materials with reference to step 2 of embodiment 20.

MS m/z (ESI): 506.2 [M+H]⁺.

¹H NMR (400 MHz, Methanol-d₄) δ 8.85 (d, J = 1.4 Hz, 1H), 8.47 (s, 1H), 8.35 (d, J = 2.5 Hz, 1H), 8.09 (s, 1H), 7.84 (dd, J = 8.8, 2.5 Hz, 1H), 7.72 (dd, J = 8.5, 2.5 Hz, 1H), 7.42 (d, J = 1.4 Hz, 1H), 6.88 (d, J = 8.9 Hz, 1H), 6.78 (d, J = 8.5 Hz, 1H), 4.75 - 4.70 (m, 1H), 3.92 - 3.85 (m, 5H), 3.84 - 3.75 (m, 2H), 3.68 - 3.58 (m, 4H), 2.74 - 2.67 (m, 1H), 1.71 (d, J = 9.0 Hz, 1H), 1.51 (d, J = 6.7 Hz, 3H).

### Embodiment 23

### 6-(3-Amino-3-methylbut-1-yn-1-yl)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

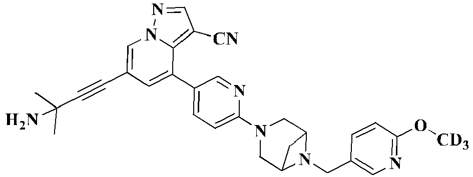

### Step 1: preparation of 6-bromo-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

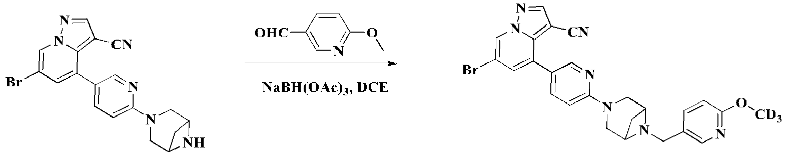

6-Bromo-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile (yellow solid) was obtained by using 4-(6-(3,6-diazabicyclo[3.1.1]heptan-3-yl)pyridin-3-yl)-6-bromopyrazolo[1,5-*a*]pyridine-3-carbonitrile as raw material with reference to step 6 of embodiment **1.** MS m/z (ESI): 519.1 [M+H]⁺.

### Step 2: preparation of 6-(3-amino-3-methylbut-1-yn-1-yl)-4-(6-(6-((6-(methoxy-d₃)pyridin-3-yl)methyl)-3,6-diazabicyclo[3.1.1]heptan-3-yl)pyridin-3-yl)pyrazolo[1,5- a]pyridine-3-carbonitrile

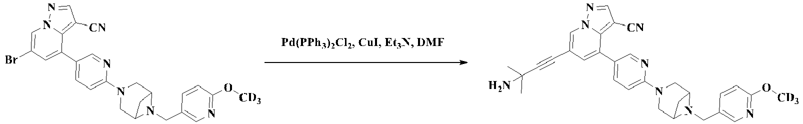

6-(3-Amino-3-methylbut-1-yn-1-yl)-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 2 of embodiment **20.**

MS m/z (ESI): 522.2 [M+H]⁺.

### Embodiment 24

### 6-(3-Amino-3-methylbut-1-yn-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

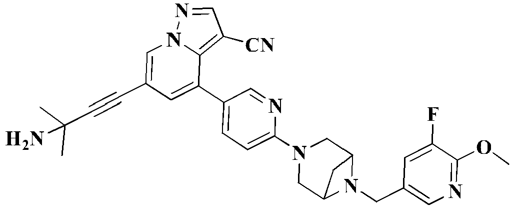

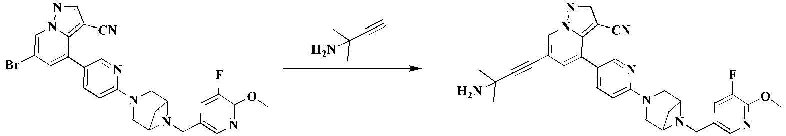

6-(3-Amino-3-methylbut-1-yn-1-yl)-4-(6-(6-((5-fluoro-6-methoxypyridin-3 - yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 2-methylbut-3-yn-2-amine as raw materials with reference to step 2 of embodiment 20.

MS m/z (ESI): 537.2 [M+H]⁺.

### Embodiment 25

### 6-(3-Hydroxy-3-methylbut-1-yn-1-yl)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

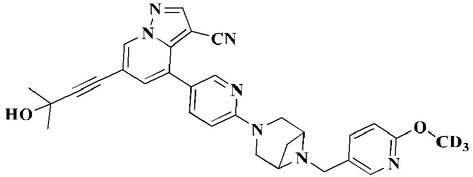

6-(3-Hydroxy-3-methylbut-1-yn-1-yl)-4-(6-(6-((6-(methoxy-*d₃*)pyridin-3 - yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 2 of embodiment **20.**

MS m/z (ESI): 523.3 [M+H]⁺.

### Embodiment 26

### 4-(6-(6-((6-Cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

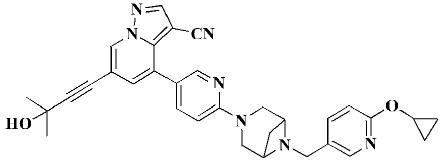

### Step 1: 5-(chloromethyl)-2-cyclopropoxypyridine

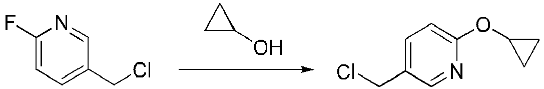

5-(Chloromethyl)-2-fluoropyridine (900 mg, 6.2 mmol) was dissolved in 30 mL of THF, and cyclopropanol (539 mg, 9.3 mmol) was added thereto. *tert*-Butyl potassium (1.04 g, 9.3 mmol) was slowly added to the reaction mixture in batches. The reaction was stirred at room temperature for 4 hours. Water was added to the reaction mixture to quench the reaction, and then the mixture was extracted with ethyl acetate. The organic phase was dried and then evaporated to dryness by rotary evaporation. The crude product was purified by column chromatography to obtain 5-(chloromethyl)-2-cyclopropoxypyridine (0.6 g, yield: 52%).

MS m/z (ESI): 184.2 [M+H]⁺.

### Step 2: 6-bromo-4-(6-(6-((6-cyclopropoxypyridin-3-yl)methyl)-3,6-diazabicyclo[3.1.1]heptan-3-yl)pyridin-3-yl)pyrazolo[1,5-a]pyridine-3-carbonitrile

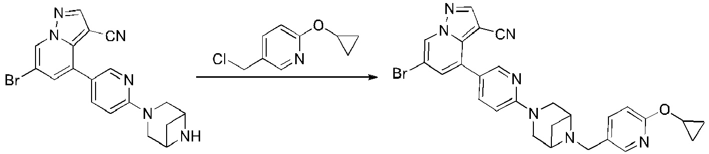

4-(6-(3,6-Diazabicyclo[3.1.1]heptan-3-yl)pyridin-3-yl)-6-bromopyrazolo[1,5-*a*]pyridine-3-carbonitrile (500 mg, 1.26 mmol) was dissolved in DMAc (15 mL), then 5-(chloromethyl)-2-cyclopropoxypyridine (279 mg, 1.52 mmol) and potassium *tert*-butoxide (284 mg, 2.53 mmol) were added thereto. The reaction mixture was stirred at 90°C for 4 hours. Water was added thereto to quench the reaction, and then the mixture was extracted with ethyl acetate. The organic phase was dried and then evaporated to dryness by rotary evaporation. The crude product was purified by column chromatography to obtain 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile (300 mg, yield: 44%).

MS m/z (ESI): 542.2 [M+H]⁺.

### Step 3: 4-(6-(6-((6-cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

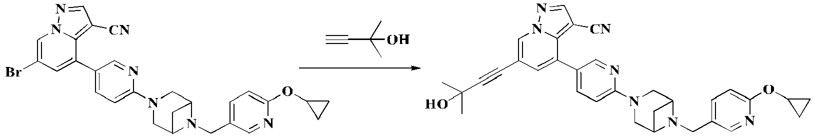

4-(6-(6-((6-Cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-*a*]pyridine-3-carbonitrile was obtained by using 2-methylbut-3-yn-2-ol and 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to embodiment 8.

MS m/z (ESI): 546.2 [M+H]⁺.

### Embodiment 27

### 6-((1-Cyanocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

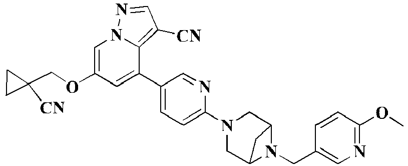

### Step 1: 6-hydroxy-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

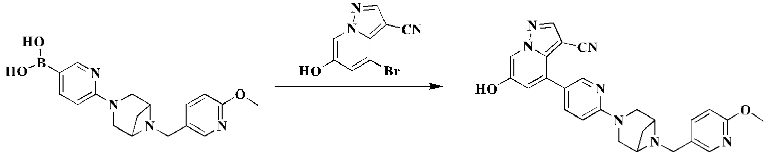

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (500mg, white solid, yield: 80%) was obtained by using (6-(6-((6-methoxypyridin-3-yl)methyl)-3,6-diazabicyclo[3.1.1]heptan-3-yl)pyridin-3-yl)boronic acid and 4-bromo-6-hydroxypyrazolo[1,5-*a*]pyridine-3-carbonitrile as raw materials with reference to step 8 of embodiment 1.

MS m/z (ESI): 454.1 [M+H]⁺.

### Step 2: 6-((1-cyanocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

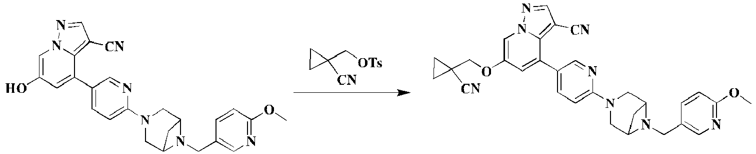

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.11 mmol) was dissolved in 2 mL of DMF; (1-cyanocyclopropyl)methyl 4-methylbenzenesulfonate (28 mg, 0.11 mmol) and potassium carbonate (42 mg, 0.3 mmol) were added thereto, and the mixture was stirred at 90°C overnight. 10 mL of water was added thereto, and the mixture was extracted with ethyl acetate (2 mL*3). The organic phase was washed with water and saturated brine, dried over anhydrous sodium sulfate. The residue was filtered, evaporated to dryness by rotary evaporation, and the crude product was separated by prep-HPLC to obtain 6-((1-cyanocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (23 mg, white solid, yield: 39%).

MS m/z (ESI): 533.2 [M+H]⁺.

¹H NMR (400 MHz, Methanol-d₄) δ 8.47 (d, J = 2.1 Hz, 1H), 8.39 - 8.33 (m, 2H), 8.09 (s, 1H), 7.86 (dd, J = 8.8, 2.5 Hz, 1H), 7.72 (dd, J = 8.5, 2.5 Hz, 1H), 7.35 (d, J = 2.1 Hz, 1H), 6.88 (d, J = 8.9 Hz, 1H), 6.78 (d, J = 8.5 Hz, 1H), 4.15 (s, 2H), 3.93 - 3.87 (m, 5H), 3.82 - 3.77 (m, 2H), 3.69 - 3.60 (m, 4H), 2.74 - 2.65 (m, 1H), 1.75 - 1.66 (m, 1H), 1.47 - 1.40 (m, 2H), 1.28 - 1.24 (m, 2H).

### Embodiment 28

### 6-((1-Cyanocyclopropyl)methoxy)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

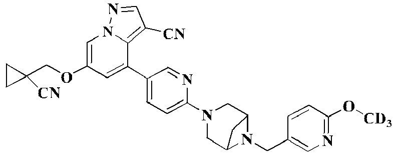

### Step 1: 3-(5-bromopyridin-2-yl)-6-((6-(methoxy-d₃)pyridin-3-yl)methyl)-3,6-diazabicyclo[3.1.1]heptane

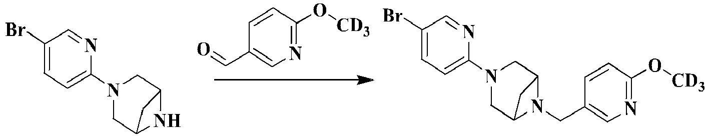

3-(5-Bromopyridin-2-yl)-6-((6-(methoxy-*d₃*)pyridin-3-yl)methyl)-3,6-diazabicyclo[3.1.1]heptane was obtained by using 3-(5-bromopyridin-2-yl)-3,6-diazabicyclo[3.1.1]heptane and 6-(methoxy-*d₃*)nicotinaldehyde as raw materials with reference to step 6 of embodiment 1.

MS m/z (ESI): 378.0 [M+H]⁺.

### Step 2: 6-((6-(methoxy-d₃)pyridin-3-yl)methyl)-3-(5-(4,4,5,5-tetramethyl-1,3,2-dioxaborolan-2-yl)pyridin-2-yl)-3,6-diazabicyclo[3.1.1]heptane

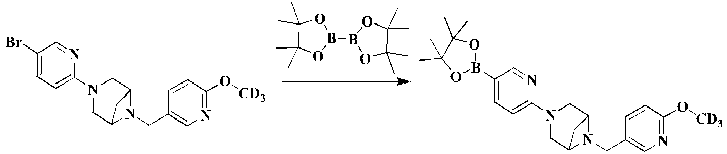

6-((6-(Methoxy-*d₃*)pyridin-3-yl)methyl)-3-(5-(4,4,5,5-tetramethyl-1,3,2-dioxaborolan-2-yl)pyridin-2-yl)-3,6-diazabicyclo[3.1.1]heptane was obtained by using 3-(5-bromopyridin-2-yl)-6-((6-(methoxy-*d₃*)pyridin-3-yl)methyl)-3,6-diazabicyclo[3.1.1]heptane as raw material with reference to step 7 of embodiment 1.

MS m/z (ESI): 426.2 [M+H]⁺.

### Step 3: 6-hydroxy-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

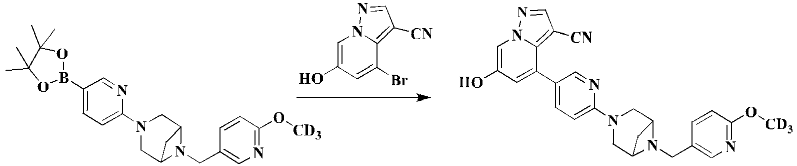

6-Hydroxy-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-((6-(methoxy-*d₃*)pyridin-3-yl)methyl)-3-(5-(4,4,5,5-tetramethyl-1,3,2-dioxaborolan-2-yl)pyridin-2-yl)-3,6-diazabicyclo[3.1.1]heptane and 4-bromo-6-hydroxypyrazolo[1,5-*a*]pyridine-3-carbonitrile as raw materials with reference to step 8 of embodiment 1.

MS m/z (ESI): 457.2 [M+H]⁺.

### Step 4: 6-((1-cyanocyclopropyl)methoxy)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

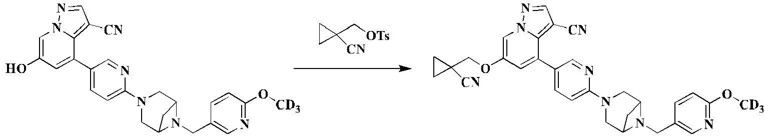

6-((1-Cyanocyclopropyl)methoxy)-4-(6-(6-((6-(methoxy-*d3*)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopropyl)methyl 4-methylbenzenesulfonate as raw materials with reference to step 2 of embodiment 27.

MS m/z (ESI): 536.2 [M+H]⁺.

¹H NMR (400 MHz, Chloroform-d) δ 8.42 (d, J = 2.5 Hz, 1H), 8.23 (s, 1H), 8.13 (d, J = 2.2 Hz, 1H), 8.11 (d, J = 2.1 Hz, 1H), 7.91 - 7.74 (m, 2H), 7.19 (d, J = 2.1 Hz, 1H), 6.76 (d, J = 8.6 Hz, 1H), 6.71 (d, J = 8.8 Hz, 1H), 4.09 - 3.99 (m, 4H), 3.98 - 3.89 (m, 2H), 3.84 - 3.64 (m, 4H), 2.05 - 1.97 (m, 1H), 1.79 - 1.71 (m, 1H), 1.53 - 1.48 (m, 2H), 1.21 - 1.15 (m, 2H).

### Embodiment 29

### 6-((1-Hydroxycyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

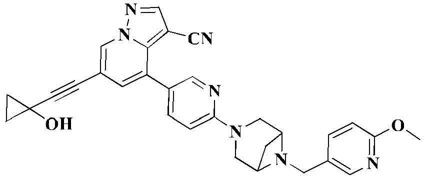

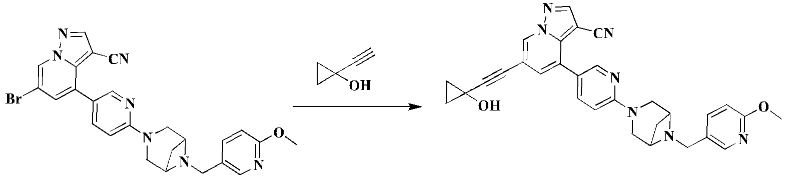

6-((1-Hydroxycyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-ethynylcyclopropan-1-ol as raw materials with reference to step 2 of embodiment 20.

MS m/z (ESI): 518.2 [M+H]⁺.

### Embodiment 30

### 6-(4-Fluoro-3-(fluoromethyl)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

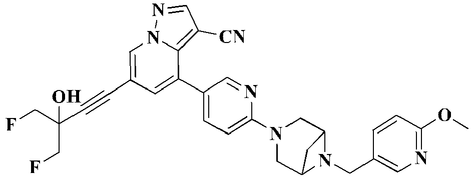

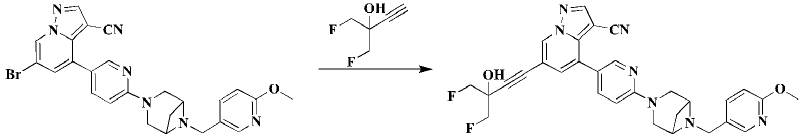

6-(4-Fluoro-3-(fluoromethyl)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-fluoro-2-(fluoromethyl)but-3-yn-2-ol as raw materials with reference to step 2 of embodiment 20.

MS m/z (ESI): 556.2 [M+H]⁺.

### Embodiment 31

### 4-(6-(6-((5-Fluoro-6-methoxypyridin-3-yl)methyl)-3,6-diazabicyclo[3.1.1] heptan-3-yl)pyridin-3-yl)-6-(3-(2-hydroxypropan-2-yl)azetidin-1-yl)pyrazolo[1,5-a]pyridine-3-carbonitrile

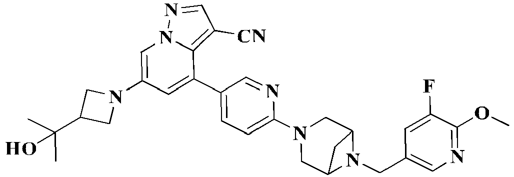

### Step 1: 4-(6-(6-((5-fluoro-6-methoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-a]pyridine-3-carbonitrile

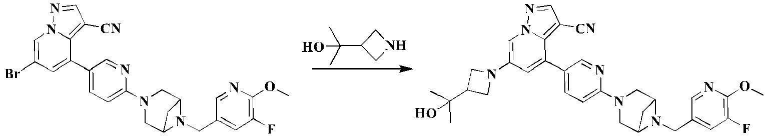

Tris(dibenzylideneacetone)dipalladium (5 mg, 0.005 mmol) and 4,5-bis(diphenylphosphino)-9,9-dimethylxanthene (3 mg, 0.005 mmol) were added to a mixture solution of 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.094 mmol), 2-(azetidin-3-yl)propan-2-ol (16 mg, 0.14 mmol ), cesium carbonate (122 mg, 0.376 mmol) and toluene (3 mL), and the mixture was replaced with nitrogen and stirred at 130°C for 2 hours under microwave irradiation. After the reaction was completed, the reaction mixture was cooled and filtered; then the filtrate was concentrated under reduced pressure to dryness and separated by preparative chromatography to obtain a white solid of 4-(6-(6-((5-fluoro-6-methoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-*a*]pyridine-3-carbonitrile (15 mg, yield: 28%).

MS m/z (ESI):569.3 [M+H]⁺.

¹H NMR (400 MHz, CDC1₃) δ 8.39 (d, *J=* 2.1 Hz, 1H), 8.14 (s, 1H), 7.88 (s, 1H), 7.79 (dd, *J=* 8.8, 2.3 Hz, 1H), 7.72 (d, *J=* 1.6 Hz, 1H), 7.57 (s, 1H), 6.75 (d, *J=* 1.7 Hz, 1H), 6.69 (d, *J=* 8.7 Hz, 1H), 4.01 (s, 3H), 3.99 - 3.93 (m, 2H), 3.91 - 3.83 (m, 4H), 3.66 (s, 4H), 2.91 - 2.79 (m, 2H), 1.25 (s, 6H).

### Embodiment 32

### 6-(6-Hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

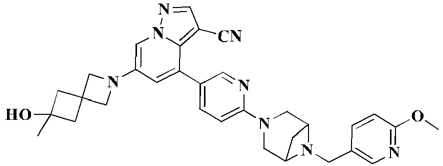

### Step 1: 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

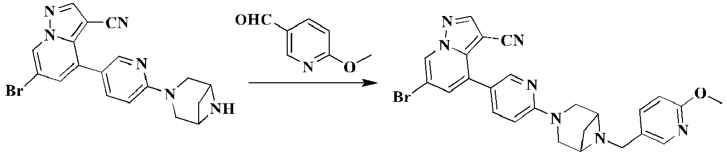

6-Bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(3,6-diazabicyclo[3.1.1]heptan-3-yl)pyridin-3-yl)-6-bromopyrazolo[1,5-*a*]pyridine-3-carbonitrile and 6-methoxynicotinaldehyde as raw materials with reference to step 9 of embodiment 11.

MS m/z (ESI):516.1 [M+H]⁺.

### Step 2: 6-(6-hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

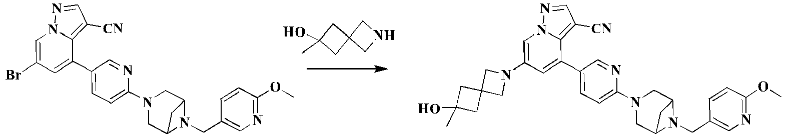

6-(6-Hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-methyl-2-azaspiro[3.3]heptan-6-ol as raw material with reference to step 1 of embodiment 31.

MS m/z (ESI):563.3 [M+H]⁺.

¹H NMR (400 MHz, CDC1₃) δ 8.38 (d, *J* = 2.1 Hz, 1H), 8.14 (d, *J* = 6.8 Hz, 2H), 7.89 (s, 1H), 7.80 (dd, *J=* 8.7, 2.3 Hz, 1H), 7.68 (d, *J=* 1.6 Hz, 1H), 6.76 (d, *J* = 8.5 Hz, 1H), 6.71 (t, *J=* 5.9 Hz, 2H),4.01- 3.93 (m, 10H), 3.74 (s, 4H), 2.96 (s, 1H), 2.39 (s, 4H), 1.40 (s, 3H).

### Embodiment 33

### 6-(3-Hydroxy-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

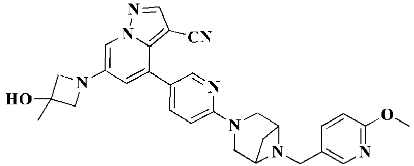

6-(3-Hydroxy-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 3-methylazetidin-3-ol as raw material with reference to step 1 of embodiment 31.

MS m/z (ESI):523.3 [M+H]⁺.

¹H NMR (400 MHz, CDC1₃) δ 8.40 (s, 1H), 8.15 (s, 3H), 7.82 (d, *J* = 7.4 Hz, 1H), 7.74 (s, 1H), 6.80 (d, *J=* 8.4 Hz, 1H), 6.75 (d, *J=* 1.7 Hz, 1H), 6.72 (d, *J* = 8.8 Hz, 1H), 4.21 (s, 2H), 4.01 (s, 2H), 3.93-3.92 (m, 7H), 3.84 (d, *J=* 7.3 Hz, 4H), 1.68 (s, 3H).

### Embodiment 34

### 6-(3-Hydroxy-3-methylpyrrolidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

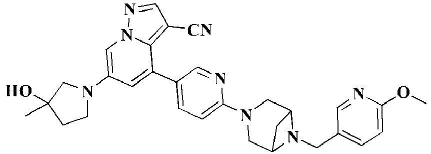

6-(3 -Hydroxy-3 -methyl pyrrolidin-1-yl)-4-(6-(6-((6-methoxypyridin-3 -yl)methyl)-3,6-diazabicyclo[3.1.1]heptan-3-yl)pyridin-3-yl)pyrazolo[1,5-*a*]pyridine-3-carbonitrile was obtained by using 3-methylpyrrolidin-3-ol as raw material with reference to step 1 of embodiment 31.

MS m/z (ESI):537.3 [M+H]⁺.

¹H NMR (400 MHz, CDCl₃) δ 8.44 (s, 2H), 8.16 (m, 2H), 7.88 (d, *J* = 6.7 Hz, 1H), 7.80 (s, 1H), 6.93 (s, 1H), 6.86 (d, *J* = 8.8 Hz, 1H), 6.76 (d, *J* = 8.6 Hz, 1H), 4.18-4.13 (m, 2H), 4.07-4.03(m, 2H), 3.93 (s, 3H), 3.65-3.61 (m, 2H), 3.46-3.41 (m, 2H), 2.24 - 2.12 (m, 3H), 2.0-1.96 (m, 3H), 1.31 (s, 3H).

### Embodiment 35

### 6-(4-Hydroxy-4-methylpiperidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

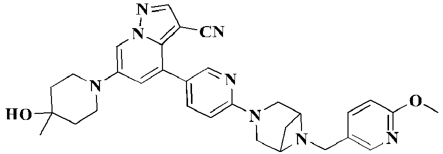

6-(4-Hydroxy-4-methylpiperidin-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-methylpiperidin-4-ol as raw material with reference to step 1 of embodiment 31.

MS m/z (ESI): 551.3 [M+H]⁺.

¹H NMR (400 MHz, CDC1₃) δ 8.41 (s, 1H), 8.18 (s, 1H), 8.14 (s, 1H), 8.07 (s, 2H), 7.82 (d, *J* = 6.7 Hz, 1H), 7.22 (d, *J* = 1.6 Hz, 1H), 6.79 (d, *J* = 8.6 Hz, 1H), 6.71 (d, *J* = 8.7 Hz, 1H), 3.99 (s, 2H), 3.93 (s, 3H), 3.85 (s, 4H), 3.36-3.32 (m, 2H), 3.23 - 3.15 (m, 2H), 1.85 - 1.74 (m, 6H), 1.35 (s, 3H).

### Embodiment 36

### 6-((1-Cyanocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

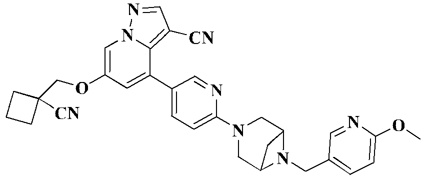

6-((1-Cyanocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained with reference to embodiment 27.

MS m/z (ESI): 547.2 [M+H]⁺.

### Embodiment 37

### 6-((3-Cyanooxetan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

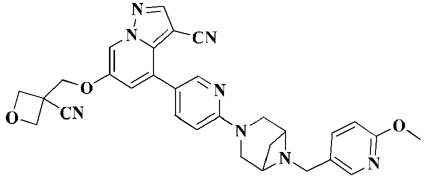

6-((3-Cyanooxetan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (12 mg, white solid, 29%) was obtained with reference to embodiment 27.

MS m/z (ESI): 549.2 [M+H]⁺.

H NMR (400 MHz, DMSO) δ 8.83 (d, *J=* 2.0 Hz, 1H), 8.64 (s, 1H), 8.42 (d, *J=* 2.1 Hz, 1H), 8.06 (s, 1H), 7.86 (d, *J=* 8.8 Hz, 1H), 7.68 (d, *J=* 6.9 Hz, 1H), 7.40 (s, 1H), 6.81 - 6.76 (m, 2H), 4.93 (d, *J=* 6.5 Hz, 2H), 4.70 (s, 2H), 4.68 (d, *J=* 6.5 Hz, 2H), 3.82 (s, 3H), 3.67 (d, *J=* 5.3 Hz, 2H), 3.53-3.47 (m, 6H), 2.01 - 1.99 (m, 2H).

### Embodiment 38

### 6-(((1-Hydroxycyclopropyl)methyl)(methyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

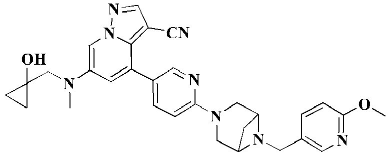

6-(((1-Hydroxycyclopropyl)methyl)(methyl)amino)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-((methylamino)methyl)cyclopropan-1-ol as raw material with reference to step 1 of embodiment 31.

MS m/z (ESI):537.3 [M+H]⁺.

### Embodiment 39

### 6-(((1-Cyanocyclopropyl)methyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

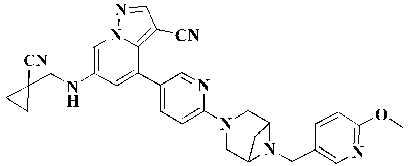

6-(((1-Cyanocyclopropyl)methyl)amino)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-(aminomethyl)cyclopropane-1-carbonitrile as raw material with reference to step 1 of embodiment 31.

MS m/z (ESI):532.3 [M+H]⁺.

### Embodiment 40

### 4-(6-(6-((6-Cyclopropoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-a]pyridine-3-carbonitrile

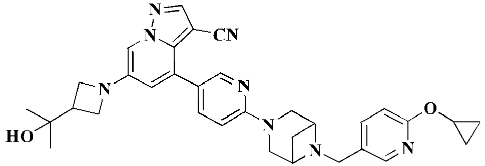

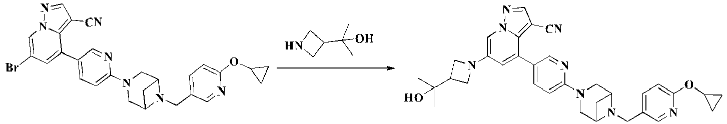

4-(6-(6-((6-Cyclopropoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-*a*]pyridine-3-carbonitrile was obtained by using 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile as raw material with reference to step 2 of embodiment 31.

MS m/z (ESI): 577.2[M+H]⁺.

### Embodiment 41

### 6-(3-(2-Hydroxypropan-2-yl)azetidin-1-yl)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

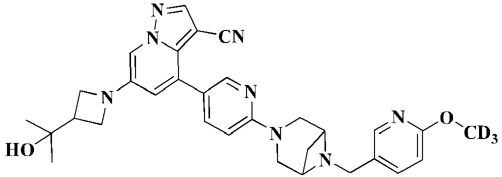

6-(3-(2-Hydroxypropan-2-yl)azetidin-1-yl)-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile (20 mg, yellow solid, 30%) was obtained by using 6-bromo-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 1 of embodiment **31.**

MS m/z (ESI): 554.3 [M+H]⁺.

¹H NMR (400 MHz, MeOD) δ 8.32 (d, J = 2.1 Hz, 1H), 8.24 (s, 1H), 8.08 (d, J = 2.0 Hz, 1H), 7.84 (d, J = 1.6 Hz, 1H), 7.82 (dd, J = 8.8, 2.4 Hz, 1H), 7.71 (dd, J = 8.6, 2.3 Hz, 1H), 6.92 (d, J = 1.7 Hz, 1H), 6.87 (d, J = 8.8 Hz, 1H), 6.78 (d, J = 8.6 Hz, 1H), 3.97 (t, J = 7.8 Hz, 2H), 3.91 - 3.85 (m, 4H), 3.79 (d, J = 5.7 Hz, 2H), 3.65 (s, 1H), 3.62 (s, 3H), 2.92 - 2.84 (m, 1H), 2.71 (s, 1H), 1.70 (d, J = 8.9 Hz, 1H), 1.21 (s, 6H).

### Embodiment 42

### N-(3-Cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)-2-hydroxy-2-methylpropanamide

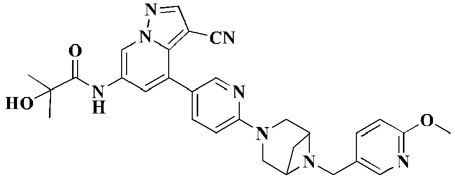

### Step 1: tert-butyl (3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)carbamate

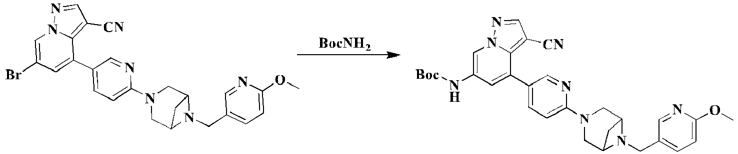

*tert*-Butyl (3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)carbamate was obtained by using *tert*-butyl carbamate as raw material with reference to step 1 of embodiment 31.

MS m/z (ESI):553.3 [M+H]⁺.

### Step 2: 6-amino-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

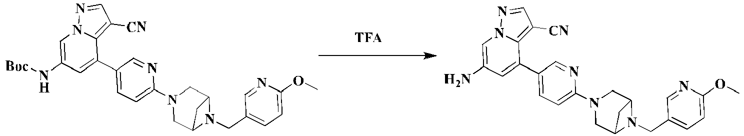

6-Amino-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using *tert*-butyl (3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)carbamate as raw material with reference to step 1 of embodiment 31.

MS m/z (ESI):453.3 [M+H]⁺.

### Step 3: N-(3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)-2-hydroxy-2-methylpropanamide

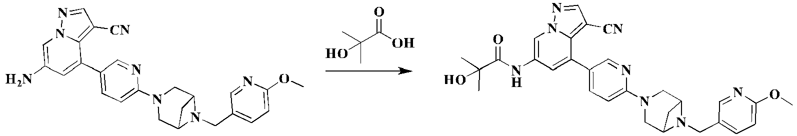

A mixture of 6-amino-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile, 2-hydroxy-2-methylpropionic acid, 2-(7-azabenzotriazol-1-yl)-*N,N,N,'N'*-tetramethyluronium hexafluorophosphate, triethylamine, *N,N-*dimethylamine hydrochloride and dichloromethane was stirred at room temperature for 16 hours. After the reaction was completed, the reaction mixture was quenched with water, extracted with ethyl acetate, and the organic phase was washed with saturated brine, dried over anhydrous sodium sulfate, filtered, and the filtrate was concentrated under reduced pressure to dryness and separated by column chromatography to obtain *N-*(3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)-2-hydroxy-2-methylpropanamide.

MS m/z (ESI): 539.2 [M+H]⁺.

### Embodiment 43

### 6-((1-Aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

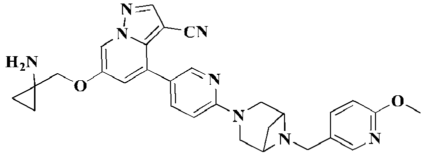

### Step 1: preparation of tert-butyl (1-(((3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxy)methyl)cyclopropyl)carbamate

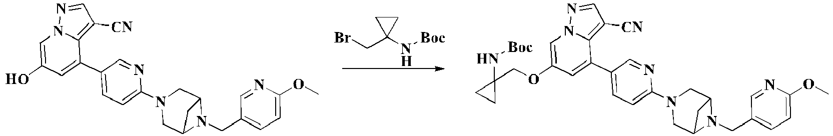

*tert*-Butyl (1-(bromomethyl)cyclopropyl)carbamate and 6-hydroxy-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile were dissolved in DMAc, and *tert*-butyl (1-(bromomethyl)cyclopropyl)carbamate and cesium carbonate were added thereto. The reaction mixture was stirred at 100°C overnight. Water was added to the reaction mixture, and then ethyl acetate was added thereto for extraction. The organic phase was dried and evaporated to dryness by rotary evaporation. The crude product was purified by prep-HPLC to obtain *tert*-butyl (1-(((3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)cyclopropyl)carbamate.

MS m/z (ESI): 623.3 [M+H]⁺.

### Step 2: preparation of 6-((1-aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

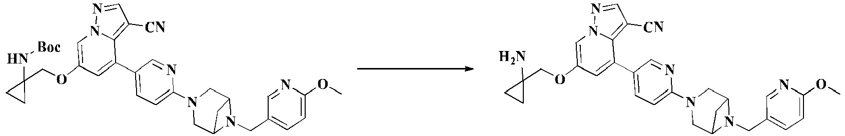

*tert*-Butyl (1-(((3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxy)methyl)cyclopropyl)carbamate was dissolved in 25% trifluoroacetic acid/dichloromethane solution. The reaction mixture was stirred at room temperature for 2 hours. The reaction mixture was evaporated to dryness by rotary evaporation and then dichloromethane was added thereto. NaHCO₃ aqueous solution was added slowly to adjust the pH value to 7-8. The organic phase was dried and then evaporated to dryness by rotary evaporation to obtain 6-((1-aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile.

MS m/z (ESI): 523.3 [M+H]⁺.

### Embodiment 44

### 6-((Cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

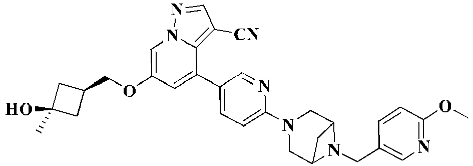

### Step 1: cis-3-(hydroxymethyl)-1-methylcyclobutan-1-ol

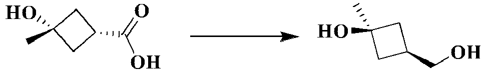

Borane-tetrahydrofuran solution (7.5 mL, 15 mmol, 2 M) was added dropwise to a solution of cis-3-hydroxy-3-methylcyclobutane-1-carboxylic acid (1 g, 7.68 mmol) in tetrahydrofuran (10 mL) at 0°C, then the mixture was stirred at room temperature for 2 hours; and after the reaction was completed, the reaction mixture was quenched with methanol, then extracted with ethyl acetate; the organic phase was combined, washed with saturated brine, dried over anhydrous sodium sulfate, filtered and concentrated under reduced pressure to dryness to obtain a colorless oil of cis-3-(hydroxymethyl)-1-methylcyclobutan-1-ol (600 mg, crude product).

### Step 2: (cis-3-hydroxy-3-methylcyclobutyl)methyl 4-methylbenzenesulfonate

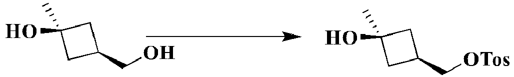

P-toluenesulfonyl chloride (987 mg ,5.17 mmol) was added to a mixed solution of cis-3-(hydroxymethyl)-1-methylcyclobutan-1-ol (600 mg, 5.17 mmol), triethylamine (1.04 g ,10.34 mmol) and dichloromethane (10 mL), and then the mixture was stirred at room temperature for 16 hours; after the reaction was completed, the reaction mixture was quenched with water, and then extracted with ethyl acetate; the organic phase was combined, washed with saturated brine, dried over anhydrous sodium sulfate, filtered, concentrated under reduced pressure to dryness, and then separated by column chromatography to obtain a colorless oil of (cis-3-hydroxy-3-methylcyclobutyl)methyl 4-methylbenzenesulfonate (800 mg, yield: 57%).

Step 3: 6-((cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile

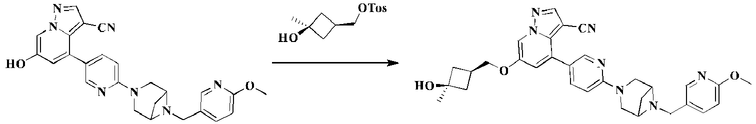

6-((Cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (cis-3-hydroxy-3-methylcyclobutyl)methyl 4-methylbenzenesulfonate as raw materials with reference to step 2 of embodiment 27.

MS m/z (ESI):552.3[M+H]⁺.

¹H NMR (400 MHz, CDC1₃) δ 8.41 (d, J = 1.9 Hz, 1H), 8.21 (s, 1H), 8.13 (s, 2H), 7.87 -7.73 (m, 2H), 7.13 (d, J = 1.6 Hz, 1H), 6.75 (d, J = 8.5 Hz, 1H), 6.70 (d, J = 8.8 Hz, 1H), 4.03 (d, J = 5.8 Hz, 2H), 3.92 (s, 7H), 3.70 (s, 4H), 2.89 (s, 1H), 2.41-2.36(m, 1H), 2.32-2.27(m, 2H), 2.05-2.00 (m, 3H), 1.45 (s, 3H).

### Embodiment 45

### 6-((3-(Hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

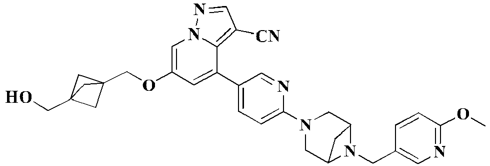

### Step 1: bicyclo[1.1.1]pentane-1,3-diyldimethanol

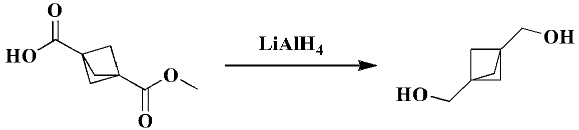

Lithium aluminum hydride (670 mg,17.63 mmol) was added to a solution of 3-(carbomethoxy<methoxycarbonyl>)bicyclo[1.1.1]pentane-1-carboxylic acid (1 g, 5.88 mmol) in tetrahydrofuran (10 mL) at 0°C, then the mixture was warmed to room temperature and stirred for 16 hours; after the reaction was completed, the reaction mixture was quenched with sodium sulfate decahydrate, filtered, and the filtrate was concentrated under reduced pressure to dryness to obtain a colorless oil of bicyclo[1.1.1]pentane-1,3-diyldimethanol (640 mg, 85%).

¹H NMR (400 MHz, DMSO) δ 4.39 (t, *J* = 5.5 Hz, 2H), 3.35 (d, *J* = 5.5 Hz, 4H), 1.45 (s, 6H).

### Step 2: (3-(hydroxymethyl)bicyclo[1.1.1]pentan-1-yl)methyl 4-methylbenzenesulfonate

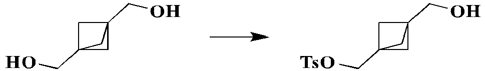

(3-(Hydroxymethyl)bicyclo[1.1.1]pentan-1-yl)methyl 4-methylbenzenesulfonate was obtained by using bicyclo[1.1.1]pentane-1,3-diyldimethanol as raw material with reference to step 2 of embodiment 44.

### Step 3: 6-((3-(hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

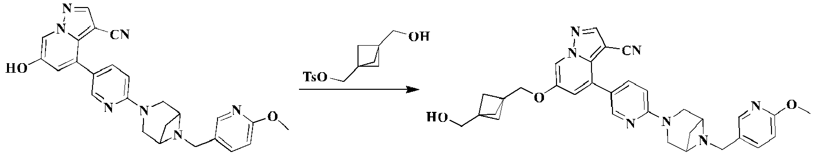

6-((3-(Hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (3-(hydroxymethyl)bicyclo[1.1.1]pentan-1-yl)methyl 4-methylbenzenesulfonate as raw materials with reference to step 2 of embodiment 27.

MS m/z (ESI):564.3[M+H]⁺.

### Embodiment 46

### (E)-6-(3-Hydroxy-3-methylbut-1-en-1-yl)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

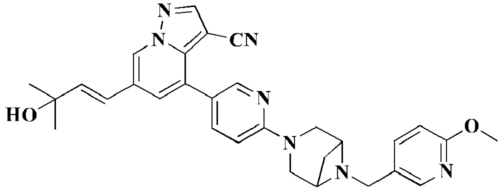

(*E*)-6-(3-Hydroxy-3-methylbut-1-en-1-yl)-4-(6-(6-((6-methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile (10 mg, white solid, 30%) was obtained by using 6-bromo-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile and (*E*)-2-methyl-4-(4,4,5,5-tetramethyl-1,3,2-dioxaborolan-2-yl)but-3-en-2-ol as raw materials with reference to step 8 of embodiment 1.

MS m/z (ESI): 522.3 [M+H]⁺.

¹H NMR (400 MHz, MeOD) δ 8.71 (s, 1H), 8.40 (s, 1H), 8.36 (d, J = 2.1 Hz, 1H), 8.09 (s, 1H), 7.86 (dd, J = 8.8, 2.3 Hz, 1H), 7.72 (dd, J = 8.5, 2.3 Hz, 1H), 7.64 (s, 1H), 6.89 (d, J = 8.9 Hz, 1H), 6.78 (d, J = 8.6 Hz, 1H), 6.65 (q, J = 16.1 Hz, 2H), 3.91 (s, 1H), 3.88 (s, 4H), 3.79 (d, J = 5.5 Hz, 2H), 3.67 - 3.62 (m, 4H), 2.71 (s, 1H), 1.71 (d, J = 9.0 Hz, 1H), 1.40 (s, 6H).

### Embodiment 47

### 6-((3-Hydroxybicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

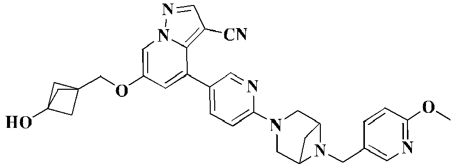

### Step 1: 6-((3-hydroxybicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

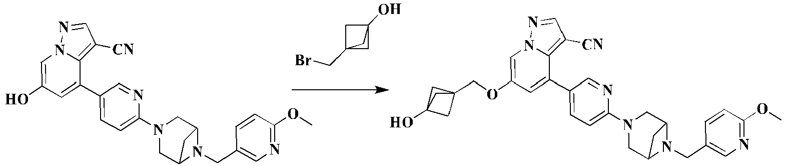

A mixture of 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.11 mmol), 3-(bromomethyl)bicyclo[1.1.1]pentan-1-ol (29 mg, 0.17 mmol), potassium carbonate (46 mg , 0.33 mmol) and acetonitrile (5 mL) was stirred at 70°C for 2 hours, then the mixture was cooled to room temperature and filtered, and the filtrate was concentrated under reduced pressure to dryness and separated by preparative chromatography to obtain 6-((3-hydroxybicyclo[1. 1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (10 mg, yield: 17%).

MS m/z (ESI): 550.2 [M+H]⁺.

¹H NMR (400 MHz, CDC1₃) δ 8.45 (s, 1H), 8.25 - 8.15 (m, 3H), 7.85 (d, *J* = 4.0 Hz, 1H), 7.17 (s, 1H), 6.84 (d, *J=* 9.2 Hz, 1H), 6.74 (d, *J=* 7.8 Hz, 2H), 4.09 (s, 2H), 4.02 (s, 2H), 3.94 (s, 3H), 3.24-3.19 (m, 2H), 2.93-2.88(m, 2H), 2.24 - 2.20 (m, 2H) , 1.58 (s, 6H).

### Embodiment 48

### 6-(4-Hydroxy-4-methylpent-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

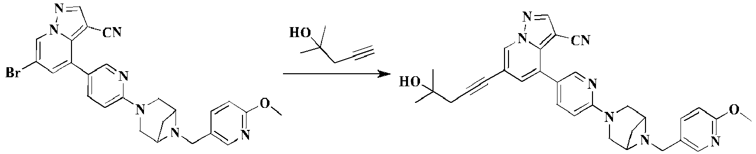

6-(4-Hydroxy-4-methylpent-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 2-methylpent-4-yn-2-ol as raw material with reference to step 2 of embodiment 8.

MS m/z (ESI):534.3 [M+H]⁺.

¹H NMR (400 MHz, CDC1₃) δ 8.62 (s, 1H), 8.41 (d, *J* = 2.1 Hz, 1H), 8.30 (s, 1H), 8.13 (s, 1H), 7.78 (dd, *J* = 8.8, 2.4 Hz, 2H), 7.31 (s, 1H), 6.75 (d, *J* = 8.6 Hz, 1H), 6.70 (d, *J* = 8.8 Hz, 1H), 3.98-3.93 (m, 7H), 3.72 (s, 4H), 2.66 (s, 2H), 1.41 (s, 6H).

### Embodiment 49

### 6-((1-Aminocyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

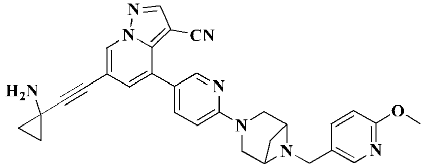

6-((1-Aminocyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-ethynylcyclopropane-1-amine as raw material with reference to step 2 of embodiment 8.

MS m/z (ESI): 517.2 [M+H]⁺.

### Embodiment 50

### 4-(6-(6-((6-Cyclopropoxypyridin-3-yl)methyl)-3,6-diazabicyclo[3.1.1]heptan-3-yl)pyridin-3-yl)-6-(2-hydroxy-2-methylpropoxy)pyrazolo[1,5-a]pyridine-3-carbonitrile

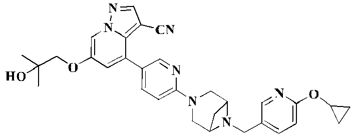

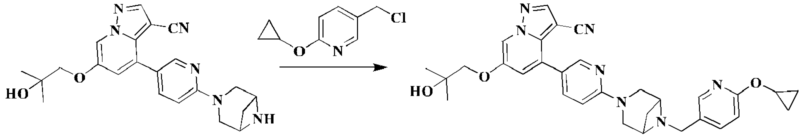

5-(Chloromethyl)-2-cyclopropoxypyridine (91 mg, 0.495 mmol) and 4-(6-(3,6-diazabicyclo[3.1.1]heptan-3-yl)pyridin-3-yl)-6-(2-hydroxy-2-methylpropoxy)pyrazolo[1,5-*a*]pyridine-3-carbonitrile (200 mg, 0.495 mmol) were dissolved in DMSO (15 mL), and cesium carbonate (322 mg, 0.989 mmol) was added thereto. The reaction was stirred at 90°C for 2 hours. Water was added to the reaction mixture, and then the reaction mixture was extracted with ethyl acetate. The organic phase was dried and then evaporated to dryness by rotary evaporation. The crude product was purified by prep-HPLC to obtain 4-(6-(6-((6-cyclopropoxypyridin-3-yl)methyl)-3,6-diazabicyclo[3.1.1]heptan-3-yl)pyridin-3-yl)-6-(2-hydroxy-2-methylpropoxy)pyrazolo[1,5-*a*]pyridine-3-carbonitrile (33.6 mg, yield: 12%).

MS m/z (ESI): 552.2 [M+H]⁺.

¹H NMR (400 MHz, DMSO-d6) δ 8.68 (d, J = 2.0 Hz, 1H), 8.59 (s, 1H), 8.41 (d, J = 2.5 Hz, 1H), 8.10 (d, J = 2.4 Hz, 1H), 7.85 (dd, J = 8.8, 2.6 Hz, 1H), 7.70 (dd, J = 8.5, 2.4 Hz, 1H), 7.31 (d, J = 2.1 Hz, 1H), 6.80 (dd, J = 8.7, 3.9 Hz, 2H), 4.72 (s, 1H), 4.21 - 4.11 (m, 1H), 3.88 (s, 2H), 3.80 - 3.64 (m, 4H), 3.62 - 3.47 (m, 4H), 2.55 - 2.53 (m, 1H), 1.59 (d, J = 8.4 Hz, 1H), 1.23 (s, 6H), 0.79 - 0.71 (m, 2H), 0.68 - 0.59 (m, 2H).

### Embodiment 51

### 6-((3-Hydroxybicyclo[1.1.1]pentan-1-yl)methoxy)-4-(6-(6-((6-(methoxy-d₃)pyridin-3-yl)methyl)-3,6-diazabicyclo[3.1.1]heptan-3-yl)pyridin-3-yl)pyrazolo[1,5-a]pyridine-3-carbonitrile

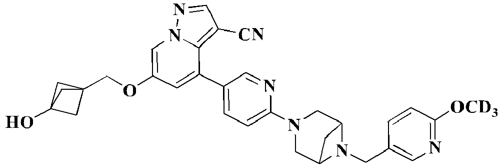

6-((3-Hydroxybicyclo[1.1.1]pentan-1-yl)methoxy)-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-(methoxy-*d₃*)pyridin-3-yl)methyl)-3,6-diazabicyclo[3.1.1]heptan-3-yl)pyridin-3-yl)pyrazolo[1,5-*a*]pyridine-3-carbonitrile and 3-(bromomethyl)bicyclo[1.1.1]pentan-1-ol as raw materials with reference to step 1 of embodiment 37.

MS m/z (ESI):553.3 [M+H]⁺.

### Embodiment 52

### 6-((3-Cyano-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

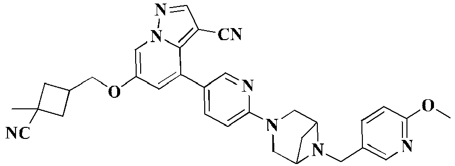

6-((3-Cyano-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (3-cyano-3-methylcyclobutyl)methyl 4-methylbenzenesulfonate as raw materials with reference to step 2 of embodiment 27.

MS m/z (ESI): 561.2 [M+H]⁺.

### Embodiment 53

### 6-((3-Cyanobicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

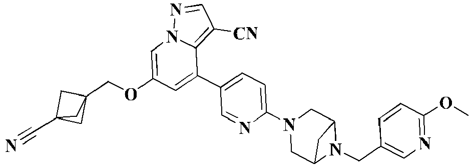

6-((3-Cyanobicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyanobicyclo[1.1.1]pentan-1-yl)methyl 4-methylbenzenesulfonate as raw material with reference to step 2 of embodiment 27.

MS m/z (ESI): 559.3.[M+H]⁺.

### Embodiment 54

### 6-((1-Imino-1-hydroxyhexahydro-1l6-thiopyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

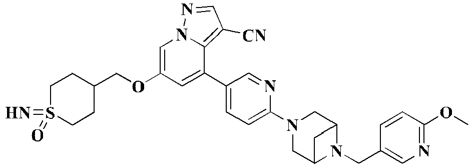

6-((1-Imino-1-hydroxyhexahydro-1l6-thiopyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (tetrahydro-2*H*-thiopyran-4-yl)methanol as raw material with reference to embodiment 16.

MS m/z (ESI): 599.2 [M+H]⁺.

¹H NMR (400 MHz, DMSO-d6) δ 8.70 (d, J = 2.1 Hz, 1H), 8.60 (s, 1H), 8.41 (d, J = 2.5 Hz, 1H), 8.07 (d, J = 2.4 Hz, 1H), 7.84 (dd, J = 8.8, 2.5 Hz, 1H), 7.68 (dd, J = 8.5, 2.4 Hz, 1H), 7.32 (d, J = 2.1 Hz, 1H), 6.78 (t, J = 8.5 Hz, 2H), 4.16 (t, J = 6.5 Hz, 1H), 4.09 - 3.98 (m, 1H), 3.89 (d, J = 17.8 Hz, 1H), 3.82 (s, 3H), 3.77 - 3.64 (m, 4H), 3.60 - 3.45 (m, 4H), 3.32 - 3.26 (m, 1H), 3.20 - 3.09 (m, 1H), 3.08 - 2.97 (m, 1H), 2.79 (q, J = 11.3 Hz, 1H), 2.65 - 2.53 (m, 1H), 2.38 - 2.25 (m, 1H), 2.19 - 2.05 (m, 1H), 2.02 - 1.93 (m, 1H), 1.92 - 1.73 (m, 1H), 1.58 (d, J = 8.4 Hz, 1H).

### Embodiment 55

### 6-((1-Cyanocyclopropyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

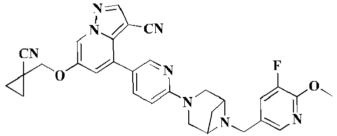

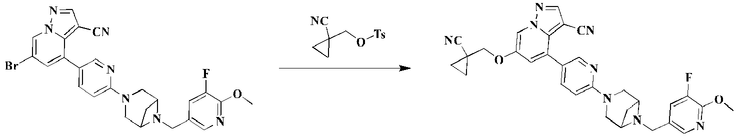

6-((1-Cyanocyclopropyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopropyl)methyl 4-methylbenzenesulfonate as raw materials with reference to step 2 of embodiment 27.

MS m/z (ESI): 551.2 [M+H]⁺.

¹H NMR (400 MHz, DMSO-d₆) δ 8.67 (d, J = 2.1 Hz, 1H), 8.62 (s, 1H), 8.42 (d, J = 2.5 Hz, 1H), 7.92 (d, J = 1.8 Hz, 1H), 7.86 (dd, J = 8.8, 2.6 Hz, 1H), 7.64 (dd, J = 11.6, 1.9 Hz, 1H), 7.39 (d, J = 2.1 Hz, 1H), 6.79 (d, J = 8.8 Hz, 1H), 4.21 (s, 2H), 3.92 (s, 3H), 3.84 - 3.65 (m, 4H), 3.63 - 3.48 (m, 4H), 2.61 - 2.53 (m, 1H), 1.59 (d, J = 8.4 Hz, 1H), 1.51 - 1.33 (m, 2H), 1.31-1.16 (m, 2H).

### Embodiment 56

### 4-(6-(6-((5-Fluoro-6-methoxypyridin-3-yl)methyl)-3,6-diazabicyclo[3.1.1]heptan-3-yl)pyridin-3-yl)-6-((3-hydroxybicyclo[1.1.1]pentan-1-yl)methoxy)pyrazolo[1,5-a]pyridine-3-carbonitrile

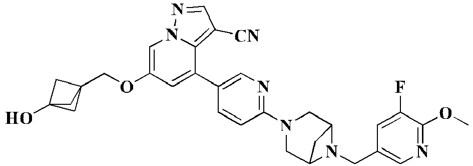

4-(6-(6-((5-Fluoro-6-methoxypyridin-3-yl)methyl)-3,6-diazabicyclo[3.1.1]heptan-3-yl)pyridin-3-yl)-6-((3-hydroxybicyclo[1.1.1]pentan-1-yl)methoxy)pyrazolo[1,5-*a*]pyridine-3-carbonitrile was obtained by using 3-(bromomethyl)bicyclo[1.1.1]pentan-1-ol and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 1 of embodiment 47.

MS m/z (ESI): 568.2 [M+H]⁺.

### Embodiment 57

### 6-((1-Cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

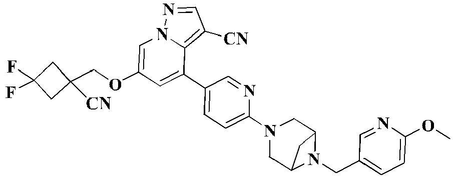

### Step 1: 1-(chloromethyl)-3,3-difluorocyclobutane-1-carbonitrile

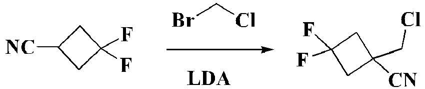

3,3-Difluorocyclobutane-1-carbonitrile (200 mg, 1.7 mmol) was dissolved in 10 mL of THF, LDA (1 mL, 2 M) was added thereto at -78°C, and the mixture was stirred at -78°C for 1 hour. Bromochloromethane (441 mg, 3.4 mmol) was added thereto at -78°C, and the mixture was stirred at -78°C to room temperature for 3 hours. Water (20 mL) was added to the reaction mixture, and then the reaction mixture was extracted with ethyl acetate (20 mL*3). The organic phase was dried and then evaporated to dryness by rotary evaporation to obtain a crude product of 1-(chloromethyl)-3,3-difluorocyclobutane-1-carbonitrile (280 mg, yield: 99%).

### Step 2: 6-((1-Cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

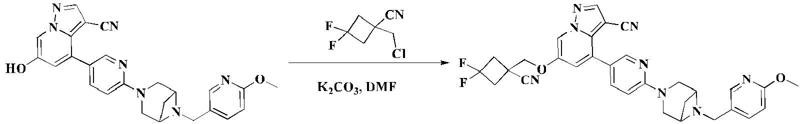

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.13 mmol) was dissolved in 5 mL of DMF; then 1-(chloromethyl)-3,3-difluorocyclobutane-1-carbonitrile (280 mg) and potassium carbonate (55 mg, 0.4 mmol) were added thereto, and the mixture was stirred at 80°C for 5 hours. The residue was evaporated to dryness by rotary evaporation, and the crude product was purified by prep-HPLC to obtain a product og 6-((1-cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (3.4 mg, yield: 4.5%).

MS m/z (ESI): 583.2[M+H]⁺.

¹H NMR (400 MHz, CDC1₃) δ 8.45 (d, *J*= 2.4 Hz, 1H), 8.26 (s, 1H), 8.23 (d, *J* = 1.3 Hz, 1H), 8.18 (s, 1H), 8.01 (s, 1H), 7.86 (d, *J=* 4.5 Hz, 1H), 7.22 - 7.19 (m, 1H), 6.90 - 6.83 (m, 1H), 6.77 (s, 1H), 4.28 (s, 2H), 3.94 (s, 3H), 3.34 - 3.22 (m, 3H), 3.07 - 2.97 (m, 3H), 2.96 - 2.88 (m, 2H), 2.25 - 2.19 (m, 2H), 2.06 - 1.93 (m, 4H).

### Embodiment 58

### 6-((3-Cyanooxetan-3-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

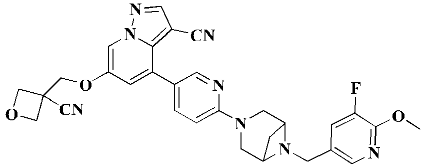

6-((3-Cyanooxetan-3-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw material with reference to embodiment 31.

MS m/z (ESI): 567.2 [M+H]⁺.

### Embodiment 59

### 6-((4-Cyanotetrahydro-2H-pyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

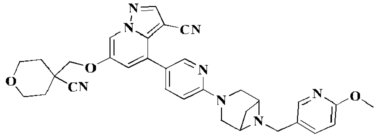

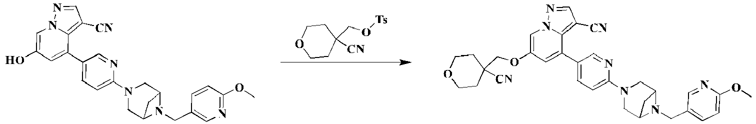

6-((4-Cyanotetrahydro-2*H*-pyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (4-cyanotetrahydro-2*H*-pyran-4-yl)methyl 4-methylbenzenesulfonate as raw materials with reference to step 2 of embodiment 31.

MS m/z (ESI): 577.2 [M+H]⁺.

¹H NMR (400 MHz, Chloroform-d) δ 8.42 (d, J = 2.5 Hz, 1H), 8.24 (s, 1H), 8.17 (d, J = 2.1 Hz, 1H), 8.13 (d, J = 2.4 Hz, 1H), 7.90-7.78 (m, 2H), 7.18 (d, J = 2.1 Hz, 1H), 6.76 (d, J = 8.5 Hz, 1H), 6.71 (d, J = 8.8 Hz, 1H), 4.14 - 4.02 (m, 4H), 3.99-3.88 (m, 6H), 3.86 - 3.61 (m, 6H), 2.12-2.05 (m, 2H), 1.90 - 1.78 (m, 3H), 1.78 - 1.64 (m, 2H).

### Embodiment 60

### 6-((4-Cyanotetrahydro-2H-pyran-4-yl)methoxy)-4-(6-(6-((5-fluoro-6-methoxypyridin-3-yl)methyl)-3,6-diazabicyclo [3.1.1] heptan-3-yl)pyridin-3-yl)pyrazolo[1,5-a]pyridine-3-carbonitrile

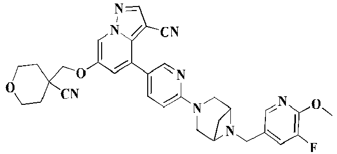

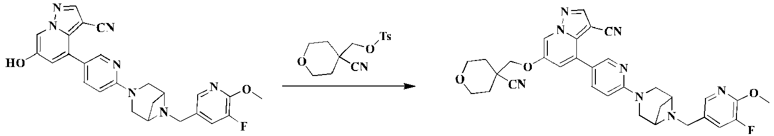

6-((4-Cyanotetrahydro-2*H*-pyran-4-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile and (4-cyanotetrahydro-2*H*-pyran-4-yl)methyl 4-methylbenzenesulfonate as raw materials with reference to step 2 of embodiment 31.

MS m/z (ESI): 595.2 [M+H]⁺.

¹H NMR (400 MHz, Chloroform-d) δ 8.42 (d, J = 2.5 Hz, 1H), 8.24 (s, 1H), 8.17 (d, J = 2.1 Hz, 1H), 7.88 (s, 1H), 7.79 (dd, J = 8.8, 2.6 Hz, 1H), 7.55 (d, J = 19.9 Hz, 1H), 7.17 (d, J = 2.1 Hz, 1H), 6.70 (d, J = 8.8 Hz, 1H), 4.09 (d, J = 4.0 Hz, 1H), 4.06 (s, 3H), 4.01 (s, 3H), 3.91 - 3.78 (m, 6H), 3.70 - 3.62 (m, 3H), 2.90 - 2.73 (m, 1H), 2.11 - 2.05 (m, 2H), 1.89 - 1.77 (m, 3H), 1.73 - 1.69 (m, 1H).

### Embodiment 61

### 4-(6-(6-((5-Fluoro-6-methoxypyridin-3-yl)methyl)-3,6-diazabicyclo[3.1.1]heptan-3-yl)pyridin-3-yl)-6-(3-hydroxy-4-methoxy-3-methylbut-1-yn-1-yl)pyrazolo[1,5-a]pyridine-3-carbonitrile

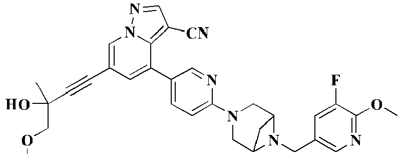

4-(6-(6-((5-Fluoro-6-methoxypyridin-3-yl)methyl)-3,6-diazabicyclo[3.1.1]heptan-3-yl)pyridin-3-yl)-6-(3-hydroxy-4-methoxy-3-methylbut-1-yn-1-yl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridine-3-yl)pyrazolo[1,5-*a*]pyridine-3-carbonitrile and 1-methoxy-2-methylbut-3-yn-2-ol as raw materials with reference to step 2 of embodiment 31.

MS m/z (ESI):568.2 [M+H]⁺.

¹H NMR (400 MHz, CDC1₃) δ 8.64 (s, 1H), 8.40 (d, *J=* 2.1 Hz, 1H), 8.31 (s, 1H), 7.90 (s, 1H), 7.78 (dd, *J* = 8.8, 2.4 Hz, 1H), 7.67 (dd, *J* = 11.8, 7.1 Hz, 1H), 7.32 (s, 1H), 6.70 (d, *J* = 8.7 Hz, 1H), 4.02 (s, 3H), 3.89 (s, 3H), 3.72 (s, 5H), 3.60 (d, *J* = 9.1 Hz, 1H), 3.52 (s, 3H), 3.45 (d, *J* = 9.1 Hz, 1H), 2.97 (m, 2H), 1.58 (s, 3H).

### Embodiment 62

### 6-((2-Hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

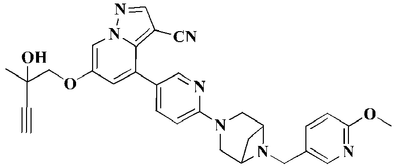

### Step 1: 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

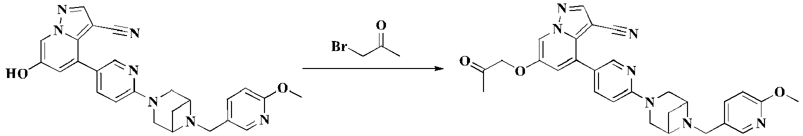

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (200 mg, 0.441 mmol) was dissolved in DMF (20 mL), then bromoacetone (121 mg, 0.882 mmol), cesium carbonate (431 mg, 1.32 mmol) and sodium iodide (66 mg, 0.441 mmol) were respectively added thereto. The reaction was stirred at room temperature overnight. Water was added to the reaction mixture, and then the reaction mixture was extracted with ethyl acetate. The organic phase was dried and then evaporated to dryness by rotary evaporation. The crude product was purified by column chromatography to obtain 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, yield: 71%).

MS m/z (ESI): 510.1 [M+H]⁺.

### Step 2: 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

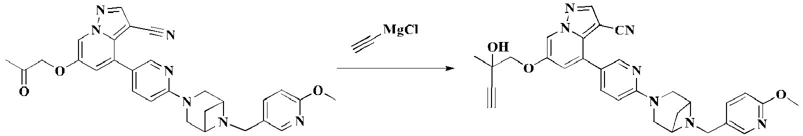

4-(6-(6-((6-Methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, 0.314 mmol) was dissolved in anhydrous tetrahydrofuran (15 mL), and then ethynylmagnesium chloride ( 6.28 mL, 3.14 mmol, 0.5 M) was slowly added thereto. After the addition was completed, the reaction mixture was stirred for 1 hour. Ammonium chloride aqueous solution was added to quench the reaction, and then the reaction mixture was extracted with ethyl acetate. The organic phase was dried and evaporated to dryness by rotary evaporation. The crude product was purified by column chromatography to obtain 6-((2-hydroxy-2-methylbut-3-yn-1-yl)oxy)-4-(6-(6-((6-methoxypyri din-3-yl)methyl)-3,6-diazabicyclo[3.1.1]heptan-3-yl)pyri din-3-yl)pyrazolo[1,5-*a*]pyridine-3-carbonitrile(48 mg, yield: 28%).

MS m/z (ESI): 536.1 [M+H]⁺.

¹H NMR (400 MHz, DMSO-d6) δ 8.74 (d, J = 2.1 Hz, 1H), 8.60 (s, 1H), 8.42 (d, J = 2.5 Hz, 1H), 8.07 (d, J = 2.4 Hz, 1H), 7.85 (dd, J = 8.8, 2.6 Hz, 1H), 7.68 (dd, J = 8.5, 2.4 Hz, 1H), 7.33 (d, J = 2.1 Hz, 1H), 6.78 (dd, J = 10.8, 8.6 Hz, 2H), 5.84 (s, 1H), 4.08 (s, 2H), 3.82 (s, 3H), 3.78 - 3.70 (m, 2H), 3.70 - 3.65 (m, 2H), 3.61 - 3.52 (m, 2H), 3.50 (s, 2H), 3.40 (s, 1H), 2.57 - 2.53 (m, 1H), 1.59 (d, J = 8.5 Hz, 1H), 1.49 (s, 3H).

### Embodiment 63

### 6-(((R)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

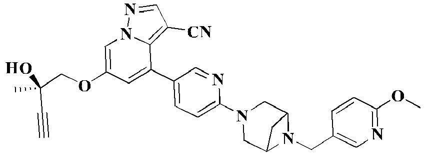

### Step 1: 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

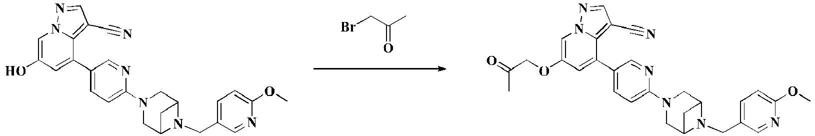

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (200 mg, 0.441 mmol) was dissolved in DMF (20 mL), then bromoacetone (121 mg, 0.882 mmol), cesium carbonate (431 mg, 1.32 mmol) and sodium iodide (66 mg, 0.441 mmol) were respectively added thereto. The reaction was stirred at room temperature overnight. Water was added to the reaction mixture, and then the mixture was extracted with ethyl acetate. The organic phase was dried and then evaporated to dryness by rotary evaporation. The crude product was purified by column chromatography to obtain 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, yield: 71%).

MS m/z (ESI): 510.1 [M+H]⁺.

### Step 2: 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

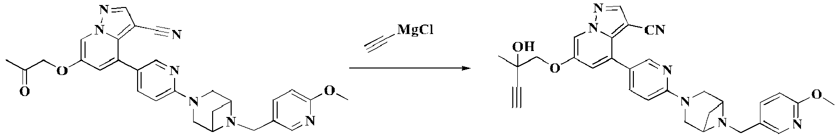

4-(6-(6-((6-Methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, 0.314 mmol) was dissolved in anhydrous tetrahydrofuran (15 mL), and then ethynylmagnesium chloride (6.28 mL, 3.14 mmol, 0.5 M) was slowly added thereto. After the addition was completed, the reaction mixture was stirred for 1 hour. Ammonium chloride aqueous solution was added thereto to quench the reaction, and then the reaction mixture was extracted with ethyl acetate. The organic phase was dried and then evaporated to dryness by rotary evaporation. The crude product was purified by column chromatography to obtain 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (48 mg, yield: 28%).

MS m/z (ESI): 536.1 [M+H]⁺.

### Step 3: 6-(((R)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

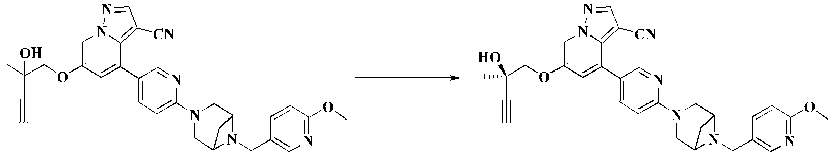

6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (16 mg) was obtained by chiral resolution from 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (48.2 mg, 0.09 mmol).

Chiral resolution conditions:

**Table 6**

| | |
|---|---|
| Instrument | CHIRALPAK IBN |
| Column type | 5.0 cm I.D. × 25 cm L, 10 µm |
| Mobile phase | Hexane/EtOH/DCM=60/30/10(V/V/V) |
| Flow rate | 60 mL/min |
| Detection wavelength | UV 254 nm |
| Column temperature | 35°C |

| | |
|---|---|
| t*_{R}*=9.002 min | |

MS m/z (ESI): 536.1 [M+H]⁺.

¹H NMR (400 MHz, DMSO-d₆) δ 8.74 (d, *J* = 2.1 Hz, 1H), 8.60 (s, 1H), 8.42 (d, *J* = 2.5 Hz, 1H), 8.07 (d, *J* = 2.4 Hz, 1H), 7.85 (dd, *J* = 8.8, 2.6 Hz, 1H), 7.68 (dd, *J* = 8.5, 2.4 Hz, 1H), 7.33 (d, *J=* 2.1 Hz, 1H), 6.78 (m, 2H), 5.84 (s, 1H), 4.08 (s, 2H), 3.82 (s, 3H), 3.78 - 3.63 (m, 4H), 3.61 - 3.46 (m, 4H), 3.40 (s, 1H), 2.57 - 2.53 (m, 1H), 1.59 (d, *J=* 8.5 Hz, 1H), 1.49 (s, 3H).

### Embodiment 64

### 6-(((S)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

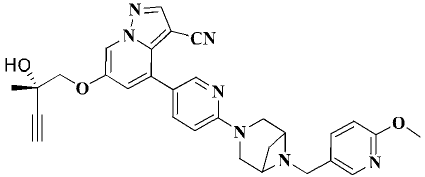

### Step 1: 6-(((S)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

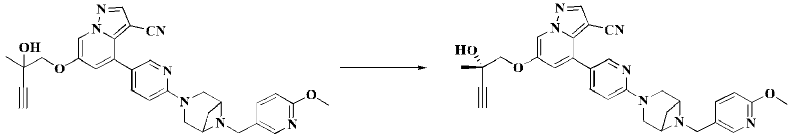

6-(((*S*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (18 mg) was obtained by chiral resolution from 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (48.2 mg, 0.09 mmol).

### Chiral resolution conditions:

**Table 7**

| | |
|---|---|
| Instrument | CHIRALPAK IBN |
| Column type | 5.0 cm I.D. x 25 cm L, 10 µm |
| Mobile phase | Hexane/EtOH/DCM=60/30/10(V/V/V) |
| Flow rate | 60 mL/min |
| Detection wavelength | UV 254 nm |
| Column temperature | 35°C |

| | |
|---|---|
| t*_{R}*=7.431 min | |

MS m/z (ESI): 536.1 [M+H]⁺.

¹H NMR (400 MHz, DMSO-d₆) δ 8.74 (d, *J* = 2.1 Hz, 1H), 8.60 (s, 1H), 8.42 (d, *J* = 2.5 Hz, 1H), 8.07 (d, *J* = 2.4 Hz, 1H), 7.85 (dd, *J* = 8.8, 2.6 Hz, 1H), 7.68 (dd, *J* = 8.5, 2.4 Hz, 1H), 7.33 (d, *J=* 2.1 Hz, 1H), 6.78 (m, 2H), 5.84 (s, 1H), 4.08 (s, 2H), 3.82 (s, 3H), 3.78 - 3.63 (m, 4H), 3.61 - 3.46 (m, 4H), 3.40 (s, 1H), 2.57 - 2.53 (m, 1H), 1.59 (d, *J=* 8.5 Hz, 1H), 1.49 (s, 3H).

### [Embodiment 65

### 4-(6-(6-((5-Fluoro-6-methoxypyridin-3-yl)methyl)-3,6-diazabicyclo[3.1.1]heptan-3-yl)pyridin-3-yl)-6-(((S)-2-hydroxy-2-methylbut-3-yn-1-yl)oxy)pyrazolo[1,5-a]pyridine-3-carbonitrile

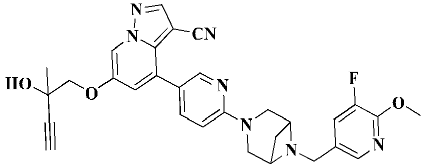

### Step 1: 4-(6-(6-((5-fluoro-6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

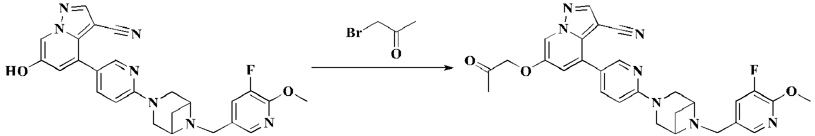

4-(6-(6-((5-Fluoro-6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (190 mg, yield: 84%) was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw material with reference to step 1 of embodiment **63.**

MS m/z (ESI): 528.1 [M+H]⁺.

### Step 2: 4-(6-(6-((5-fluoro-6-methoxypyridin-3-yl)methyl)-3,6-diazabicyclo[3.1.1]heptan-3-yl)pyridin-3-yl)-6-(((S)-2-hydroxy-2-methylbut-3-yn-1-yl)oxy)pyrazolo[1,5-a]pyridine-3-carbonitrile

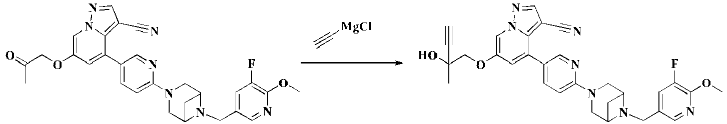

4-(6-(6-((5-Fluoro-6-methoxypyridin-3-yl)methyl)-3,6-diazabicyclo[3.1.1]heptan-3-yl)pyridin-3-yl)-6-(((*S*)-2-hydroxy-2-methylbut-3-yn-1-yl)oxy)pyrazolo[1,5-*a*]pyridine-3-carbonitrile (40 mg, yield: 22%) was obtained by using 4-(6-(6-((5-fluoro-6-methoxypyridin-3-yl)methyl)-3,6-diazabicyclo[3.1. 1]heptan-3-yl)pyridin-3-yl)-6-(2-oxopropoxy)pyrazolo[1,5-*a*]pyridine-3-carbonitrile as raw material with reference to step 2 of embodiment **63.**

MS m/z (ESI): 554.3 [M+H]⁺.

¹H NMR (400 MHz, DMSO-d₆) δ 8.74 (d, *J* = 2.1 Hz, 1H), 8.60 (s, 1H), 8.42 (d, *J* = 2.5 Hz, 1H), 7.92 (d, *J* = 1.9 Hz, 1H), 7.85 (dd, *J=* 8.8, 2.6 Hz, 1H), 7.64 (dd, *J* = 11.5, 1.9 Hz, 1H), 7.33 (d, *J* = 2.1 Hz, 1H), 6.79 (d, *J* = 8.8 Hz, 1H), 5.84 (s, 1H), 4.08 (s, 2H), 3.92 (s, 3H), 3.81 - 3.65 (m, 4H), 3.64 - 3.48 (m, 4H), 3.40 (s, 1H), 2.59 - 2.53 (m, 1H), 1.59 (d, *J=* 8.4 Hz, 1H), 1.49 (s, 3H).

### Embodiment 66

### 3-(((3-Cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxy)methyl)bicyclo[1.1.1]pentane-1-carboxamide

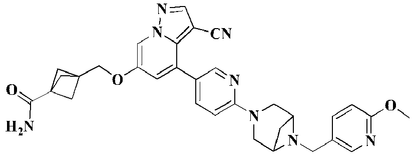

3-(((3-Cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)bicyclo[1.1.1]pentane-1-carboxamide was obtained by using (3-carbamoylbicyclo[1.1.1]pentan-1-yl)methyl 4-methylbenzenesulfonate and 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 27.

MS m/z (ESI):577.3 [M+H]⁺.

¹H NMR (400 MHz, CDCl₃) δ 9.70 - 9.62 (m, 1H), 8.41 (d, *J* = 2.1 Hz, 1H), 8.21 (s, 1H), 8.16 - 8.08 (m, 2H), 7.93 - 7.77 (m, 2H), 7.13 (d, *J* = 1.9 Hz, 1H), 6.73 (dd, *J* = 22.6, 8.7 Hz, 2H), 5.52 (s, 1H), 5.39 (s, 1H), 4.07 (s, 2H), 3.93 (s, 5H), 3.72 (s, 4H), 2.96 (s, 1H), 2.88 (s, 1H), 2.15 (s, 6H).

### Embodiment 67

### 3-(((3-Cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxy)methyl)-N-methylbicyclo[1.1.1]pentane-1-carboxamide

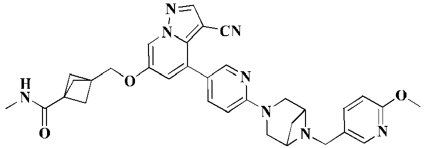

3-(((3-Cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)-*N-*methylbicyclo[1.1.1]pentane-1-carboxamide was obtained by using (3-(methylcarbamoyl)bicyclo[1.1.1]pentan-1-yl)methyl 4-methylbenzenesulfonate and 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 27.

MS m/z (ESI):591.3 [M+H]⁺.

¹H NMR (400 MHz, CDC1₃) δ 8.40 (d, *J* = 2.2 Hz, 1H), 8.21 (s, 1H), 8.14 - 8.05 (m, 2H), 7.78 (dd, *J* = 8.8, 2.4 Hz, 1H), 7.67 (d, *J* = 7.5 Hz, 1H), 7.12 (d, *J* = 1.9 Hz, 1H), 6.70 (dd, *J* = 15.4, 8.7 Hz, 2H), 5.58 (d, *J* = 4.5 Hz, 1H), 4.05 (s, 2H), 3.92 (s, 3H), 3.83 (dd, *J* = 17.6, 8.8 Hz, 4H), 3.60 (s, 4H), 2.83 (d, *J* = 4.9 Hz, 3H), 2.72 (d, *J* = 5.8 Hz, 1H), 2.11 (s, 6H), 1.67 (d, *J* = 8.7 Hz, 1H).

### Embodiment 68

### 4-(6-(6-((5-Fluoro-6-methoxypyridin-3-yl)methyl)-3,6-diazabicyclo[3.1.1] heptan-3-yl)pyridin-3-yl)-6-((R)-3-hydroxybut-1-yn-1-yl)pyrazolo[1,5-a]pyridine-3-carbonitrile

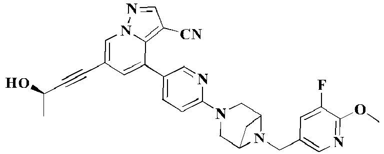

4-(6-(6-((5-Fluoro-6-methoxypyridin-3-yl)methyl)-3,6-diazabicyclo[3.1.1]heptan-3-yl)pyridin-3-yl)-6-((*R*)-3-hydroxybut-1-yn-1-yl)pyrazolo[1,5-*a*]pyridine-3-carbonitrile was obtained by using (*R*)-but-3-yn-2-ol and 6-bromo-4-(6-(6-((5-fluoro-6-methoxypyridin-3-yl)methyl)-3,6-diazabicyclo[3.1.1]heptan-3-yl)pyrazolo[1,5-*a*]pyridine-3-carbonitrile as raw materials with reference to step 2 of embodiment 8.

MS m/z (ESI):524.2 [M+H]⁺.

¹H NMR (400 MHz, CDC1₃) δ 8.62 (d, *J* = 1.0 Hz, 1H), 8.40 (d, *J* = 2.2 Hz, 1H), 8.31 (s, 1H), 7.89 (s, 1H), 7.78 (dd, *J* = 8.8, 2.4 Hz, 1H), 7.62 (s, 1H), 7.30 (d, *J* = 1.2 Hz, 1H), 6.70 (d, *J* = 8.8 Hz, 1H), 4.80 (q, *J* = 6.6 Hz, 1H), 4.01 (s, 3H), 3.97 - 3.82 (m, 3H), 3.71 (s, 4H), 2.87 (s, 2H), 1.74 (d, *J* = 8.2 Hz, 1H), 1.59 (d, *J* = 6.6 Hz, 3H).

### Embodiment 69

### 6-((3-Cyanobicyclo[1.1.1]pentan-1-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

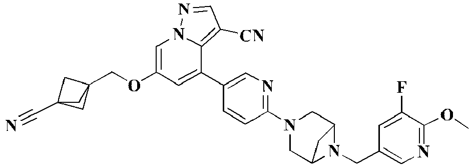

6-((3-Cyanobicyclo[1.1.1]pentan-1-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyanobicyclo[1.1.1]pentan-1-yl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 31.

MS m/z (ESI):577.2 [M+H]⁺.

¹H NMR (400 MHz, CDC1₃) δ 8.40 (d, *J* = 1.9 Hz, 1H), 8.22 (s, 1H), 8.07 (s, 1H), 7.88 (s, 1H), 7.79 (dd, *J* = 8.7, 2.0 Hz, 1H), 7.60 (s, 1H), 7.11 (s, 1H), 6.70 (d, *J* = 8.8 Hz, 1H), 4.01 (s, 5H), 3.88 (m, 4H), 3.68 (s, 4H), 2.85 (s, 1H), 2.37 (s, 6H), 1.76 - 1.69 (m, 1H).

### Embodiment 70

### 6-((3-Cyano-3-methylcyclobutyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

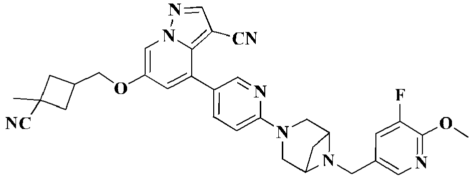

6-((3-Cyano-3-methylcyclobutyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyano-3-methylcyclobutyl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 31.

MS m/z (ESI): 579.3 [M+H]⁺.

### Embodiment 71

### 4-(6-(6-((5-Fluoro-6-methoxypyridin-3-yl)methyl)-3,6-diazabicyclo[3.1.1]heptan-3-yl)pyridin-3-yl)-6-((3-(hydroxymethyl)bicyclo[1.1.1]pentan-1-yl)methoxy)pyrazolo[1,5-a]pyridine-3-carbonitrile

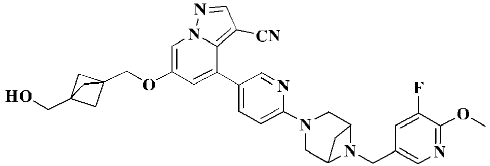

4-(6-(6-((5-Fluoro-6-methoxypyridin-3-yl)methyl)-3,6-diazabicyclo[3.1.1]heptan-3-yl)pyridin-3-yl)-6-((3-(hydroxymethyl)bicyclo[1.1.1]pentan-1-yl)methoxy)pyrazolo[1,5-*a*]pyridine-3-carbonitrile was obtained by using (3-(hydroxymethyl)bicyclo[1.1.1]pentan-1-yl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 27.

MS m/z (ESI): 582.3 [M+H]⁺.

¹H NMR (400 MHz, CDC1₃) δ 8.41 (d, *J* = 2.1 Hz, 1H), 8.21 (s, 1H), 8.11 (d, *J* = 1.9 Hz, 1H), 7.88 (s, 1H), 7.79 (dd, *J* = 8.8, 2.4 Hz, 1H), 7.54 (d, *J* = 10.8 Hz, 1H), 7.14 (d, *J=* 1.9 Hz, 1H), 6.69 (d, *J* = 8.8 Hz, 1H), 4.05 (s, 2H), 4.01 (s, 3H), 3.85 (d, *J* = 11.3 Hz, 4H), 3.66 (s, 6H), 2.79 (s, 1H), 1.82 (s, 6H), 1.70 (d, *J=* 8.8 Hz, 1H).

### Embodiment 72

### 4-(6-(6-((5-Fluoro-6-methoxypyridin-3-yl)methyl)-3,6-diazabicyclo [3.1.1] heptan-3-yl)pyridin-3-yl)-6-((cis-3-hydroxy-3-methylcyclobutyl)methoxy)pyrazolo[1,5-a]pyridine-3-carbonitrile

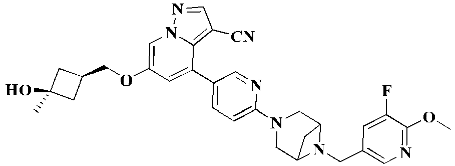

4-(6-(6-((5-Fluoro-6-methoxypyridin-3-yl)methyl)-3,6-diazabicyclo[3.1.1]heptan-3-yl)pyridin-3-yl)-6-((cis-3-hydroxy-3-methylcyclobutyl)methoxy)pyrazolo[1,5-*a*]pyridine-3-carbonitrile was obtained by using (cis-3-hydroxy-3-methylcyclobutyl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 27.

MS m/z (ESI):570.3 [M+H]⁺.

¹H NMR (400 MHz, CDCl₃) δ 8.42 (d, *J* = 2.1 Hz, 1H), 8.21 (s, 1H), 8.13 (d, *J=* 1.9 Hz, 1H), 7.92 (s, 1H), 7.81 (dd, *J* = 8.7, 2.2 Hz, 1H), 7.75 (s, 1H), 7.14 (d, *J* = 1.9 Hz, 1H), 6.71 (d, *J* = 8.8 Hz, 1H), 4.14 - 3.99 (m, 7H), 3.93 (s, 2H), 3.77 (s, 4H), 3.00 (s, 1H), 2.38 (dd, *J* = 15.0, 7.6 Hz, 1H), 2.29 (dd, *J* = 12.5, 7.4 Hz, 2H), 2.02 (t, *J* = 10.2 Hz, 2H), 1.78 (s, 2H), 1.45 (s, 3H).

### Embodiment 73

### 4-(6-(6-((5-Fluoro-6-methoxypyridin-3-yl)methyl)-3,6-diazabicyclo[3.1.1] heptan-3-yl)pyridin-3-yl)-6-(3-hydroxy-3-methylazetidin-1-yl)pyrazolo[1,5-a]pyridine-3-carbonitrile

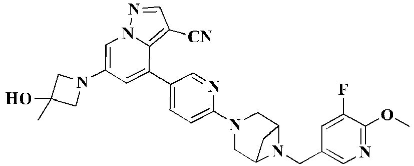

4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylazetidin-1-yl)pyrazolo[1,5-*a*]pyridine-3-carbonitrile was obtained by using 3-methylazetidin-3-ol and 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 1 of embodiment 31.

MS m/z (ESI): 541.2 [M+H]⁺.

¹H NMR (400 MHz, CDC1₃) δ 8.40 (s, 1H), 8.15 (s, 1H), 7.95 (s, 2H), 7.82 (d, *J* = 8.2 Hz, 1H), 7.74 (d, *J* = 1.6 Hz, 1H), 6.74 (dd, *J* = 11.8, 5.2 Hz, 2H), 4.25 (s, 2H), 4.02 (s, 3H), 3.88 (dd, *J* = 36.0, 7.4 Hz, 7H), 3.22 (s, 1H), 2.25 - 2.18 (m, 1H), 2.01 (s, 1H), 1.85 (s, 2H), 1.68 (s, 3H).

### Embodiment 74

### 1-Cyano-N-(3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)cyclopropane-1-carboxamide

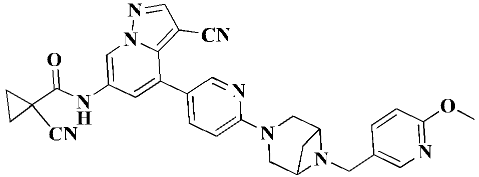

### Step 1: 1-cyano-N-(3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)cyclopropane-1-carboxamide

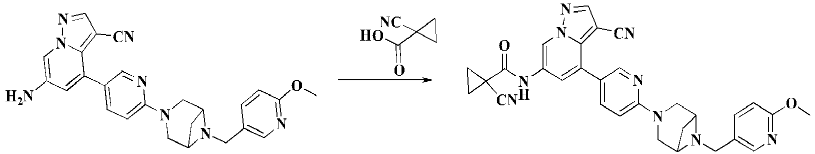

6- Amino-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was dissolved in DMF, and then 1-cyanocyclopropane-1-carboxylic acid, HATU and DIEA were added thereto. The reaction mixture was stirred at room temperature overnight. Water was added thereto, and then the reaction mixture was extracted with ethyl acetate. The organic phase was washed with saturated sodium chloride and dried, then evaporated to dryness by rotary evaporation. The crude product was purified by column chromatography to obtain 1-cyano-*N*-(3-cyano-4-(6-(6-((6-methoxypyridin-3-yl)methyl)-3,6-diazabicyclo[3 .1.1]heptan-3-yl)pyridin-3-yl)pyrazolo[1,5-*a*]pyridin-6-yl)cyclopropane-1-carboxamide (off-white solid).

MS m/z (ESI): 546.1 [M+H]⁺.

¹H NMR (400 MHz, DMSO-*d*₆) δ 10.43 (br., s, 1H), 9.26 (d, *J* = 1.7 Hz, 1H), 8.65 (s, 1H), 8.40 (d, *J* = 2.6 Hz, 1H), 8.07 (d, *J* = 2.3 Hz, 1H), 7.83 (dd, *J* = 8.8, 2.6 Hz, 1H), 7.74 - 7.64 (m, 2H), 6.82 (d, *J* = 8.8 Hz, 1H), 6.77 (d, *J* = 8.5 Hz, 1H), 3.82 (s, 3H), 3.80 - 3.65 (m, 4H), 3.61 - 3.49 (m, 4H), 1.79 - 1.70 (m, 3H), 1.59 (d, *J* = 8.4 Hz, 1H), 1.26 - 1.13 (m, 2H).

### Embodiment 75

### 6-(3-Hydroxy-4-methoxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

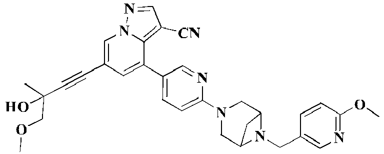

6-(3-Hydroxy-4-methoxy-3-methylbut-1 -yn-1 -yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (off-white solid) was obtained from 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-methoxy-2-methylbut-3-yn-2-ol.

MS m/z (ESI):550.3 [M+H]⁺.

¹H NMR (400 MHz, DMSO) δ 9.09 (s, 1H), 8.76 (s, 1H), 8.42 (s, 1H), 8.09 (s, 1H), 7.86 (s, 1H), 7.70 (s, 1H), 7.40 (s, 1H), 6.80 (s, 1H), 5.72 (s, 1H), 3.79 (m, 14H), 3.15 - 2.98 (m, 4H), 1.47 (s, 3H).

### Embodiment 76

### 6-(3-Cyano-3-methylazetidin-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

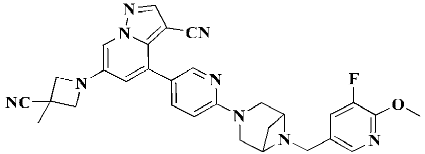

6-(3-Cyano-3-methylazetidin-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-(5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 3-methylazetidin-3-carbonitrile as raw materials with reference to step 1 of embodiment 31.

MS m/z (ESI):550.2 [M+H]⁺.

¹H NMR (400 MHz, CDC1₃) δ 8.38 (d, *J* = 2.2 Hz, 1H), 8.18 (s, 1H), 7.87 (s, 1H), 7.79 (dd, *J* = 8.8, 2.4 Hz, 1H), 7.73 (d, *J* = 1.7 Hz, 1H), 7.54 (d, *J* = 10.5 Hz, 1H), 6.70 (dd, *J* = 5.2, 3.4 Hz, 2H), 4.29 (d, *J* = 7.0 Hz, 2H), 4.01 (s, 3H), 3.91-3.85 (m, 6H), 3.67-3.64 (m, 4H), 2.80 (s, 1H), 1.83 (s, 3H), 1.70 (d, *J* = 8.7 Hz, 1H).

### Embodiment 77

### 6-(3-Cyano-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

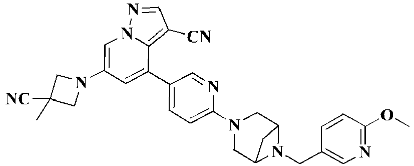

### Step 1: preparation of 6-(3-cyano-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

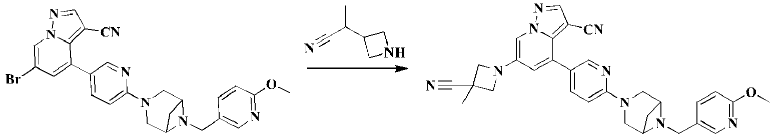

A mixture of 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (100 mg, 0.19 mmol), tris(dibenzylideneacetone)dipalladium (9 mg, 0.0095 mmol), 2-dicyclohexylphospho-2',4',6'-triisopropylbiphenyl (5 mg, 0.011 mmol), cesium carbonate (123 mg, 0.38 mmol), 3-methylazetidin-3-carbonitrile (28 mg, 0.29 mmol) and toluene (5 mL) was replaced with nitrogen, then stirred at 130°C under microwave irradiation for 2 hours. After the reaction was completed, the reaction mixture was cooled to room temperature, concentrated, dissolved in ethyl acetate and washed with saturated brine, the organic phase was dried over anhydrous sodium sulfate, filtered, evaporated to dryness by rotary evaporation, and then purified by preparative chromatography to obtain 6-(3-cyano-3-methylazetidin-1-yl)-4-(6-(6-((6-methoxypyridin-3-yl)methyl)-3,6-diazabicyclo[3 .1.1]heptan-3-yl)pyridin-3-yl)pyrazolo[1,5-*a*]pyridine-3-carbonitrile (45 mg, white solid, yield: 45%).

MS m/z (ESI):532.3 [M+H]⁺.

¹H NMR (400 MHz, DMSO) δ 8.53 (s, 1H), 8.40 (d, *J* = 2.1 Hz, 1H), 8.21 (d, *J=* 1.5 Hz, 1H), 8.07 (s, 1H), 7.83 (d, *J* = 8.7 Hz, 1H), 7.68 (d, *J* = 8.5 Hz, 1H), 7.03 (d, *J=* 1.7 Hz, 1H), 6.78 (t, *J* = 9.3 Hz, 2H), 4.27 (d, *J* = 7.7 Hz, 2H), 3.91 (d, *J* = 7.7 Hz, 2H), 3.82 (s, 3H), 3.76-3.67 (m, 4H), 3.56-3.50 (m, 4H), 2.04 - 1.93 (m, 1H), 1.67 (s, 3H), 1.59 (d, *J* = 7.9 Hz, 1H).

### Embodiment 78

### 6-((4-Hydroxytetrahydro-2H-pyran-4-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

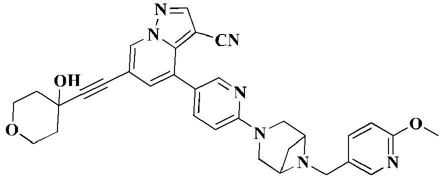

6-((4-Hydroxytetrahydro-2*H*-pyran-4-yl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 4-ethynyltetrahydro-2*H*-pyran-4-ol as raw materials with reference to step 2 of embodiment 8.

MS m/z (ESI):562.3 [M+H]⁺.

¹H NMR (400 MHz, CDC1₃) δ 8.66 (s, 1H), 8.42 (d, *J* = 2.0 Hz, 1H), 8.32 (s, 1H), 8.14 (s, 1H), 8.02 (s, 1H), 7.80 (d, *J* = 8.6 Hz, 1H), 7.31 (s, 1H), 6.79 (d, *J* = 8.6 Hz, 1H), 6.72 (d, *J* = 8.9 Hz, 1H), 4.12 (d, *J* = 7.1 Hz, 2H), 4.04 - 3.90 (m, 7H), 3.88 - 3.68 (m, 6H), 2.32 (s, 1H), 2.13 - 2.03 (m, 3H), 1.96-1.90 (m, 2H).

### Embodiment 79

### 4-(6-(6-((5-Fluoro-6-methoxypyridin-3-yl)methyl)-3,6-diazabicyclo[3.1.1]heptan-3-yl)pyridin-3-yl)-6-((4-hydroxytetrahydro-2H-pyran-4-yl)ethynyl)pyrazolo[1,5-a]pyridine-3-carbonitrile

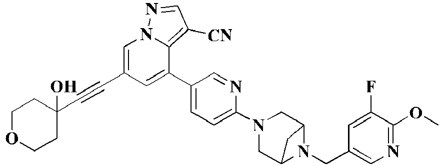

4-(6-(6-((5-Fluoro-6-methoxypyridin-3-yl)methyl)-3,6-diazabicyclo[3.1.1]heptan-3-yl)pyridin-3-yl)-6-((4-hydroxytetrahydro-2*H*-pyran-4-yl)ethynyl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 4-ethynyltetrahydro-2*H*-pyran-4-ol as raw materials with reference to step 2 of embodiment 8.

MS m/z (ESI):580.2 [M+H]⁺.

¹H NMR (400 MHz, CDC1₃) δ 8.66 (s, 1H), 8.42 (s, 1H), 8.32 (s, 1H), 7.97 -7.75 (m, 3H), 7.31 (s, 1H), 6.72 (d, *J* = 8.6 Hz, 1H), 4.20 - 4.08 (m, 2H), 4.05 - 3.93 (m, 7H), 3.84-3.70 (m, 6H), 2.13 - 2.03 (m, 3H), 1.97 - 1.88 (m, 3H).

### Embodiment 80

### 6-((3-Hydroxyoxetan-3-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

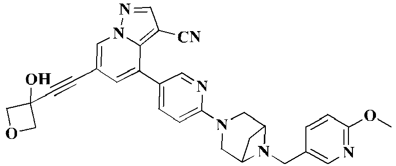

6-((3-Hydroxyoxetan-3-yl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained with reference to step 2 of embodiment **8.**

MS m/z (ESI): 534.2 [M+H]⁺.

¹H NMR (400 MHz, CDC1₃) δ 8.66 (s, 1H), 8.40 (s, 1H), 8.33 (s, 1H), 8.12 (s, 1H), 7.77 (d, *J* = 8.7 Hz, 2H), 7.29 (d, *J* = 10.6 Hz, 1H), 6.72 (dd, *J* = 16.3, 8.5 Hz, 2H), 4.95 (d, *J* = 6.7 Hz, 2H), 4.83 (d, *J* = 6.7 Hz, 2H), 3.92 (s, 3H), 3.89 (s, 4H), 3.67 (s, 4H), 2.82 (s, 1H), 1.72 (d, *J* = 8.2 Hz, 2H).

### Embodiment 81

### 4-(6-(6-((5-Fluoro-6-methoxypyridin-3-yl)methyl)-3,6-diazabicyclo[3.1.1]heptan-3-yl)pyridin-3-yl)-6-((3-hydroxyoxetan-3-yl)ethynyl)pyrazolo[1,5-a]pyridine-3-carbonitrile

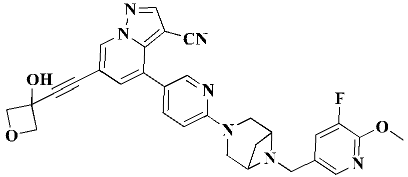

4-(6-(6-((5-Fluoro-6-methoxypyridin-3-yl)methyl)-3,6-diazabicyclo[3.1.1]heptan-3-yl)pyridin-3-yl)-6-((3-hydroxyoxetan-3-yl)ethynyl)pyrazolo[1,5-*a*]pyridine-3-carbonitrile (white solid) was obtained with reference to step 2 of embodiment **8.**

MS m/z (ESI): 552.2 [M+H]⁺.

¹H NMR (400 MHz, CDC1₃) δ 8.66 (s, 1H), 8.41 (s, 1H), 8.33 (s, 1H), 7.89 (s, 1H), 7.77 (d, *J* = 8.7 Hz, 1H), 7.54 (s, 1H), 7.30 (s, 1H), 6.70 (d, *J* = 8.7 Hz, 1H), 4.95 (d, *J* = 6.7 Hz, 2H), 4.83 (d, *J* = 6.8 Hz, 2H), 4.01 (s, 3H), 3.88 (s, 4H), 3.66 (s, 4H), 2.81 (s, 1H), 1.71 (d, *J* = 6.8 Hz, 2H).

### Embodiment 82

### 6-((1-Hydroxycyclobutyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

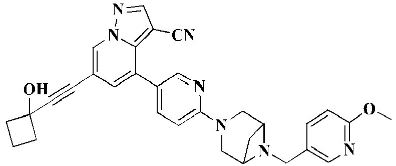

6-((1-Hydroxycyclobutyl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-ethynylcyclobutan-1-ol as raw materials with reference to step 2 of embodiment 8.

MS m/z (ESI): 532.2 [M+H]⁺.

¹H NMR (400 MHz, DMSO-d₆) δ 9.14 (d, J = 1.3 Hz, 1H), 8.75 (s, 1H), 8.42 (d, J = 2.5 Hz, 1H), 8.07 (d, J = 2.3 Hz, 1H), 7.86 (dd, J = 8.8, 2.5 Hz, 1H), 7.68 (dd, J = 8.5, 2.4 Hz, 1H), 7.46 (d, J = 1.4 Hz, 1H), 6.83 - 6.73 (m, 2H), 6.00 (s, 1H), 3.82 (s, 3H), 3.79 - 3.67 (m, 4H), 3.60 - 3.50 (m, 4H), 2.58 - 2.56 (m, 1H), 2.47 - 2.39 (m, 2H), 2.29 - 2.19 (m, 2H), 1.85 - 1.78 (m, 2H), 1.62 - 1.57 (m, 1H).

### Embodiment 83

### 4-(6-(6-((5-Fluoro-6-methoxypyridin-3-yl)methyl)-3,6-diazabicyclo[3.1.1]heptan-3-yl)pyridin-3-yl)-6-((1-hydroxycyclobutyl)ethynyl)pyrazolo[1,5-a]pyridine-3-carbonitrile

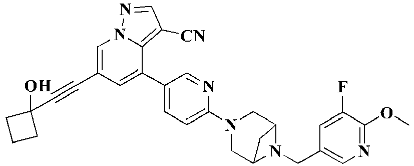

4-(6-(6-((5-Fluoro-6-methoxypyridin-3-yl)methyl)-3,6-diazabicyclo[3.1.1]heptan-3-yl)pyridin-3-yl)-6-((1-hydroxycyclobutyl)ethynyl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 1-ethynylcyclobutan-1-ol as raw materials with reference to step 2 of embodiment 8.

MS m/z (ESI): 550.2 [M+H]⁺.

¹H NMR (400 MHz, DMSO-d₆) δ 9.14 (s, 1H), 8.75 (s, 1H), 8.42 (s, 1H), 7.91 (s, 1H), 7.86 (dd, J = 8.7, 2.6 Hz, 1H), 7.64 (d, J = 11.4 Hz, 1H), 7.45 (d, J = 1.3 Hz, 1H), 6.80 (d, J = 8.8 Hz, 1H), 5.99 (s, 1H), 3.92 (s, 3H), 3.76 - 3.68 (m, 4H), 3.60 - 3.51 (m, 4H), 2.58 - 2.56 (m, 1H), 2.45 - 2.35 (m, 2H), 2.27 - 2.23 (m, 2H), 1.83 - 1.78 (m, 2H), 1.59 (d, J = 8.2 Hz, 1H).

### Embodiment 84

### N (1-(5-(3-Cyano-6-(3-hydroxy-3-methylbut-1-yn-1-yl)pyrazolo[1,5-a]pyridin-4-yl)pyridin-2-yl)-4-methylpiperidin-4-yl)-5-fluoro-2-methylbenzamide

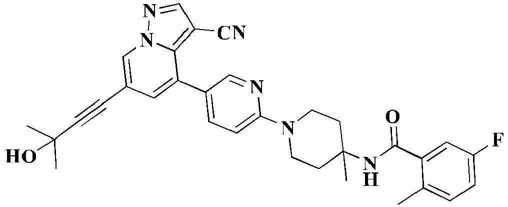

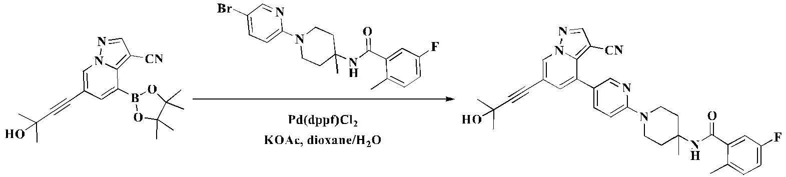

*N*-(1-(5-(3-Cyano-6-(3-hydroxy-3-methylbut-1-yn-1-yl)pyrazolo[1,5-*a*]pyridin-4-yl)pyridin-2-yl)-4-methylpiperidin-4-yl)-5-fluoro-2-methylbenzamide (30 mg, white solid, yield: 45%) was obtained by using 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile and *N*-(1-(5-bromopyridin-2-yl)-4-methylpiperidin-4-yl)-5-fluoro-2-methylbenzamide as raw materials with reference to step 8 of embodiment **1.**

MS m/z (ESI): 551.2 [M+H]⁺.

¹H NMR (400 MHz, Methanol-d₄) δ 8.82 (d, J = 1.3 Hz, 1H), 8.46 (s, 1H), 8.29 (d, J = 2.5 Hz, 1H), 7.76 (dd, J = 9.0, 2.5 Hz, 1H), 7.41 - 7.36 (m, 1H), 7.28 - 7.21 (m, 1H), 7.09 - 7.02 (m, 2H), 6.98 (d, J = 8.9 Hz, 1H), 4.10 - 4.00 (m, 2H), 3.44 - 3.39 (m, 2H), 2.44 - 2.38 (m, 2H), 2.38 (s, 3H), 1.77 - 1.69 (m, 2H), 1.58 (s, 6H), 1.54 (s, 3H).

### Embodiment 85

### 3-Chloro-N (1-(5-(3-cyano-6-(3-hydroxy-3-methylbut-1-yn-1-yl)pyrazolo[1,5-a]pyridin-4-yl)pyridin-2-yl)-4-methylpiperidin-4-yl)picolinamide

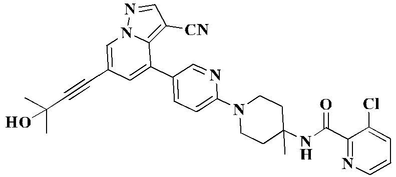

### Step 1: tert-butyl (1-(5-(6-bromo-3-cyanopyrazolo[1,5-a]pyridin-4-yl)pyridin-2-yl)-4-methylpiperidin-4-yl)carbamate

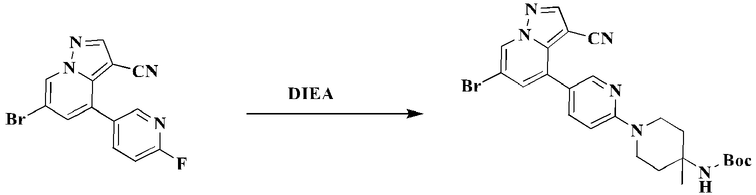

*tert*-Butyl (1-(5-(6-bromo-3-cyanopyrazolo[1,5-*a*]pyridin-4-yl)pyridin-2-yl)-4-methylpiperidin-4-yl)carbamate (white solid) was obtained by using 6-bromo-4-(6-fluoropyridin-3-yl)pyrazolo[1,5-*a*]pyridine-3-carbonitrile as raw material with reference to step 4 of embodiment 1.

MS m/z (ESI): 511.1 [M+H]⁺.

### Step 2: 4-(6-(4-amino-4-methylpiperidin-1-yl)pyridin-3-yl)-6-bromopyrazolo[1,5-a]pyridine-3-carbonitrile

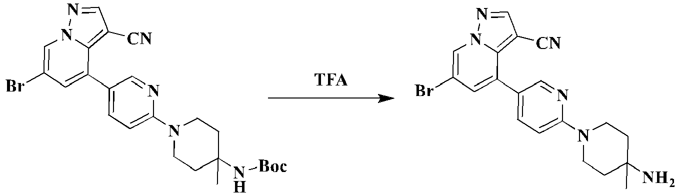

4-(6-(4-Amino-4-methylpiperidin-1-yl)pyridin-3-yl)-6-bromopyrazolo[1,5-*a*]pyridine-3-carbonitrile (white solid) was obtained by using *tert*-butyl (1-(5-(6-bromo-3-cyanopyrazolo[1,5-*a*]pyridin-4-yl)pyridin-2-yl)-4-methylpiperidin-4-yl)carbamate as raw material with reference to step 5 of embodiment 1.

MS m/z (ESI): 411.1 [M+H]⁺.

### Step 3: N-(1-(5-(6-bromo-3-cyanopyrazolo[1,5-a]pyridin-4-yl)pyridin-2-yl)-4-methylpiperidin-4-yl)-3-chloropicolinamide

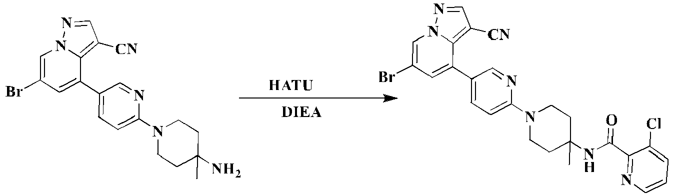

*N*-(1-(5-(6-Bromo-3-cyanopyrazolo[1,5-*a*]pyridin-4-yl)pyridin-2-yl)-4-methylpiperidin-4-yl)-3-chloropicolinamide (white solid) was obtained by using 4-(6-(4-amino-4-methylpiperidin-1-yl)pyridin-3-yl)-6-bromopyrazolo[1,5-*a*]pyridine-3-carbonitrile as raw material with reference to step 1 of embodiment 20.

MS m/z (ESI): 550.0 [M+H]⁺.

### Step 4: 3-chloro-N-(1-(5-(3-cyano-6-(3-hydroxy-3-methylbut-1-yn-1-yl)pyrazolo[1,5-a]pyridin-4-yl)pyridin-2-yl)-4-methylpipeiidin-4-yl)picolinamide

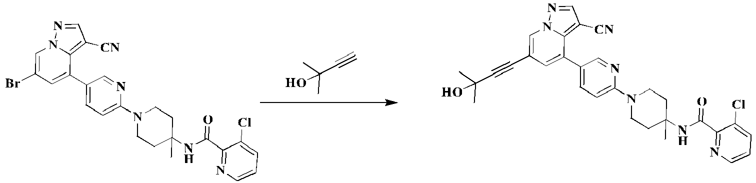

3-Chloro-*N*-(1-(5-(3-cyano-6-(3-hydroxy-3-methylbut-1-yn-1-yl)pyrazolo[1,5-a]pyridin-4-yl)pyridin-2-yl)-4-methylpiperidin-4-yl)picolinamide (white solid) was obtained by using *N*-(1-(5-(6-bromo-3-cyanopyrazolo[1,5-*a*]pyridin-4-yl)pyridin-2-yl)-4-methylpiperidin-4-yl)-3-chloropicolinamide as raw material with reference to step 2 of embodiment 20.

MS m/z (ESI): 554.2 [M+H]⁺.

¹H NMR (400 MHz, Methanol-d₄) δ 8.81 (d, J = 1.3 Hz, 1H), 8.50 (dd, J = 4.7, 1.3 Hz, 1H), 8.45 (s, 1H), 8.28 (d, J = 2.6 Hz, 1H), 7.96 (dd, J = 8.2, 1.4 Hz, 1H), 7.75 (dd, J = 8.8, 2.6 Hz, 1H), 7.48 (dd, J = 8.2, 4.7 Hz, 1H), 7.38 (d, J = 1.3 Hz, 1H), 6.97 (d, J = 8.9 Hz, 1H), 4.18 - 4.03 (m, 2H), 3.48 - 3.37 (m, 2H), 2.54 - 2.34 (m, 2H), 1.80 - 1.68 (m, 2H), 1.58 (s, 6H), 1.56 (s, 3H).

### Embodiment 86

### N-(1-(5-(6-(3-Amino-3-methylbut-1-yn-1-yl)-3-cyanopyrazolo[1,5-a]pyridin-4-yl)pyridin-2-yl)-4-methylpiperidin-4-yl)-3-chloropicolinamide

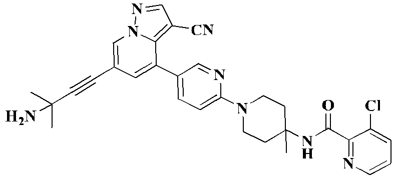

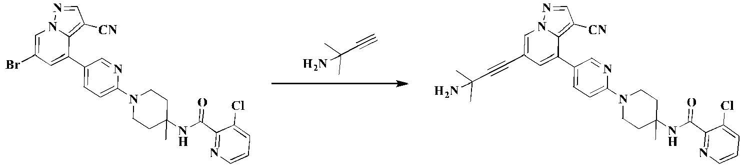

*N*-(1-(5-(6-(3-Amino-3-methylbut-1-yn-1-yl)-3-cyanopyrazolo[1,5-*a*]pyridin-4-yl)pyridin-2-yl)-4-methylpiperidin-4-yl)-3-chloropicolinamide (white solid) was obtained by using *N*-(1-(5-(6-bromo-3-cyanopyrazolo[1,5-*a*]pyridin-4-yl)pyridin-2-yl)-4-methylpiperidin-4-yl)-3-chloropicolinamide and 2-methylbut-3-yn-2-amine as raw materials with reference to step 2 of embodiment 20.

MS m/z (ESI): 553.2 [M+H]⁺.

¹H NMR (400 MHz, Methanol-d₄) δ 8.80 (d, J = 1.4 Hz, 1H), 8.50 (d, J = 4.8 Hz, 1H), 8.45 (s, 1H), 8.28 (d, J = 2.5 Hz, 1H), 7.99 - 7.94 (m, 1H), 7.78 - 7.73 (m, 1H), 7.51 - 7.46 (m, 1H), 7.38 (s, 1H), 6.98 (d, J = 8.8 Hz, 1H), 4.15 - 4.05 (m, 2H), 3.50 - 3.45 (m, 2H), 2.44 - 2.38 (m, 2H), 1.78 - 1.72 (m, 2H), 1.56 (s, 3H), 1.52 (s, 6H).

### Embodiment 87

### N-(1-(5-(6-(3-amino-3-methylbut-1-yn-1-yl)-3-cyanopyrazolo[1,5-a]pyridin-4-yl)pyridin-2-yl)-4-methylpiperidin-4-yl)-5-fluoro-2-methylbenzamide

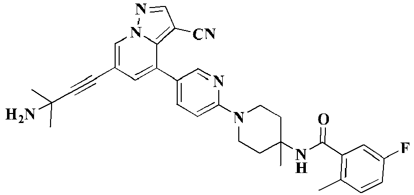

### Step 1: N-(1-(5-(6-bromo-3-cyanopyrazolo[1,5-a]pyridin-4-yl)pyridin-2-yl)-4-methylpiperidin-4-yl)-5-fluoro-2-methylbenzamide

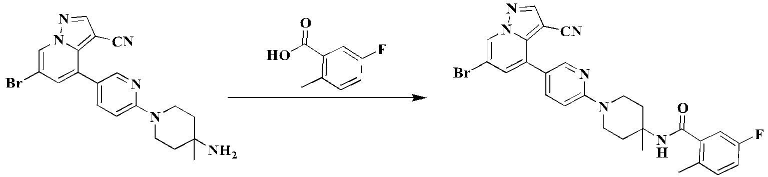

*N*-(1-(5-(6-Bromo-3-cyanopyrazolo[1,5-*a*]pyridin-4-yl)pyridin-2-yl)-4-methylpiperidin-4-yl)-5-fluoro-2-methylbenzamide (white solid) was obtained by using 4-(6-(4-amino-4-methylpiperidin-1-yl)pyridin-3-yl)-6-bromopyrazolo[1,5-*a*]pyridine-3-carbonitrile and 5-fluoro-2-methylbenzoic acid as raw materials with reference to step 2 of embodiment 20.

MS m/z (ESI): 547.1 [M+H]⁺.

### Step 2: N-(1-(5-(6-(3-amino-3-methylbut-1-yn-1-yl)-3-cyanopyrazolo[1,5-a]pyridin-4-yl)pyridin-2-yl)-4-methylpiperidin-4-yl)-5-fluoro-2-methylbenzamide

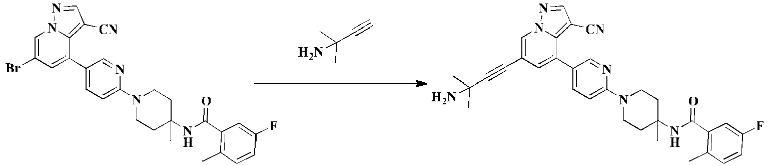

*N*-(1-(5-(6-(3-Amino-3-methylbut-1-yn-1-yl)-3-cyanopyrazolo[1,5-*a*]pyridin-4-yl)pyridin-2-yl)-4-methylpiperidin-4-yl)-5-fluoro-2-methylbenzamide (white solid) was obtained by using *N*-(1-(5-(6-bromo-3-cyanopyrazolo[1,5-*a*]pyridin-4-yl)pyridin-2-yl)-4-methylpiperidin-4-yl)-5-fluoro-2-methylbenzamide and 2-methylbut-3-yn-2-amine as raw materials with reference to step 2 of embodiment 20.

MS m/z (ESI): 550.2 [M+H]⁺.

¹H NMR (400 MHz, Methanol-d₄) δ 8.80 (d, J = 1.3 Hz, 1H), 8.45 (s, 1H), 8.28 (d, J = 2.6 Hz, 1H), 7.76 (dd, J = 8.9, 2.6 Hz, 1H), 7.38 (d, J = 1.2 Hz, 1H), 7.28 - 7.21 (m, 1H), 7.11 - 7.02 (m, 2H), 6.98 (d, J = 8.9 Hz, 1H), 4.08 - 4.00 (m, 2H), 3.43 - 3.38 (m, 2H), 2.46 - 2.39 (m, 2H), 2.38 (s, 3H), 1.75 - 1.69 (m, 2H), 1.54 (s, 3H), 1.52 (s, 6H).

### Embodiment 88

### 3-Chloro-N (1-(5-(3-cyano-6-(3-(2-hydroxypropan-2-yl)azetidin-1-yl)pyrazolo[1,5-a]pyridin-4-yl)pyridin-2-yl)-4-methylpiperidin-4-yl)picolinamide

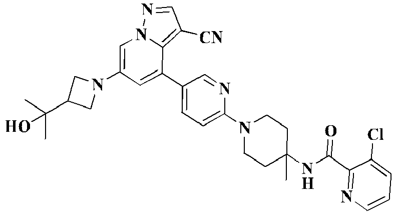

3-Chloro-*N*-(1-(5-(3-cyano-6-(3-(2-hydroxypropan-2-yl)azetidin-1-yl)pyrazolo[1,5-*a*]pyridin-4-yl)pyridin-2-yl)-4-methylpiperidin-4-yl)picolinamide was obtained by using 2-(azetidin-3-yl)propan-2-ol and *N*-(1-(5-(6-bromo-3-cyanopyrazolo[1,5-*a*]pyridin-4-yl)pyridin-2-yl)-4-methylpiperidin-4-yl)-3-chloropicolinamide as raw materials with reference to step 1 of embodiment 31.

MS m/z (ESI):585.2 [M+H]⁺.

¹H NMR (400 MHz, CDCl₃) δ 8.46 (d, *J* = 3.9 Hz, 1H), 8.34 (s, 1H), 8.13 (s, 1H), 7.94 (s, 1H), 7.83 (d, *J* = 8.1 Hz, 2H), 7.71 (s, 1H), 7.38 (dd, *J* = 8.1, 4.5 Hz, 1H), 6.77 (s, 1H), 4.14 (s, 2H), 3.96 (t, *J* = 7.6 Hz, 2H), 3.85 (t, *J* = 6.6 Hz, 2H), 3.48 (s, 2H), 2.48 (s, 2H), 1.89 - 1.80 (m, 2H), 1.25 (s, 3H), 1.24 (s, 6H).

### Embodiment 89

### 3-Chloro-N(1-(5-(3-cyano-6-(6-hydroxy-6-methyl-2-azaspiro [3.3] heptan-2-yl)pyrazolo[1,5-a]pyridin-4-yl)pyridin-2-yl)-4-methylpiperidin-4-yl)picolinamide

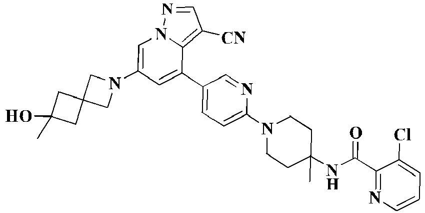

3-Chloro-*N-*(1-(5-(3-cyano-6-(6-hydroxy-6-methyl-2-azaspiro[3.3]heptan-2-yl)pyrazolo[1,5-*a*]pyridin-4-yl)pyridin-2-yl)-4-methylpiperidin-4-yl)picolinamide was obtained by using 6-methyl-2-azaspiro[3.3]heptan-6-ol as raw material with reference to step 1 of embodiment 31.

MS m/z (ESI): 597.2 [M+H]⁺.

¹H NMR (400 MHz, CDCl₃) δ 8.47 (s, 1H), 8.32 (s, 1H), 8.13 (s, 1H), 7.94 (s, 1H), 7.83 (d, J = 8.6 Hz, 1H), 7.67 (s, 1H), 7.39 (d, J = 4.6 Hz, 1H), 6.89 (s, 1H), 6.71 (s, 1H), 4.15 (s, 2H), 3.94 (d, J = 5.8 Hz, 4H), 3.47 (s, 2H), 2.48 (s, 2H), 2.38 (s, 4H), 1.84 (s, 2H), 1.39 (s, 3H), 1.25 (s, 3H).

### Embodiment 90

### N-(1-(5-(6-((1-Aminocyclopropyl)ethynyl)-3-cyanopyrazolo[1,5-a]pyridin-4-yl)pyridin-2-yl)-4-methylpiperidin-4-yl)-3-chloropicolinamide

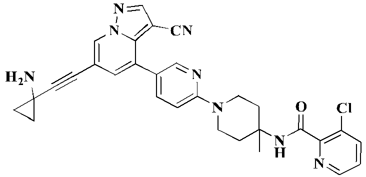

*N*-(1-(5-(6-((1-Aminocyclopropyl)ethynyl)-3-cyanopyrazolo[1,5-*a*]pyridin-4-yl)pyridin-2-yl)-4-methylpiperidin-4-yl)-3-chloropicolinamide was obtained by using 1-ethynylcyclopropane-1-amine as raw material with reference to step 2 of embodiment 31.

MS m/z (ESI): 551.2[M+H]⁺.

### Embodiment 91

### N-(1-(5-(6-((1-aminocyclopropyl)methoxy)-3-cyanopyrazolo[1,5-a]pyridin-4-yl)pyridin-2-yl)-4-methylpiperidin-4-yl)-3-chloropicolinamide

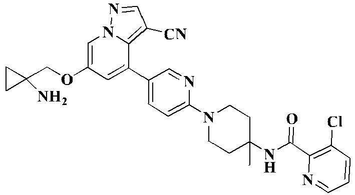

### Step 1: tert-butyl (1-(((4-(6-(4-(3-chloropicolinamido)-4-methylpiperidin-1-yl)pyridin-3-yl)-3-cyanopyrazolo[1,5-a]pyridin-6-yl)oxy)methyl)cyclopropyl)carbamate

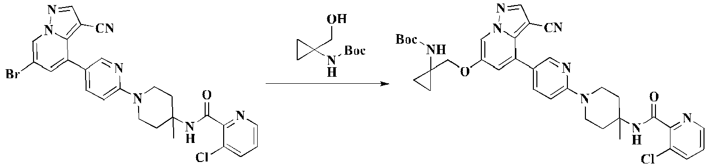

*N-*(1-(5-(6-Bromo-3-cyanopyrazolo[1,5-*a*]pyridin-4-yl)pyridin-2-yl)-4-methylpiperidin-4-yl)-3-chloropicolinamide (100 mg, 0.182mmol) was dissolved in NMP (2 mL), then *tert*-butyl (1-(hydroxy)cyclopropyl)carbamate (51 mg, 0.272 mmol) and cesium carbonate (177 mg, 0.545 mmol) were added thereto. The reaction was directly prepared by stirring at 160°C for 1 hour under microwave irradiation to obtain *tert*-butyl (1-(((4-(6-(4-(3-chloropicolinamido)-4-methylpiperidin-1-yl)pyridin-3-yl)-3-cyanopyrazolo[1,5-*a*]pyridin-6-yl)oxy)methyl)cyclopropyl)carbamate.

MS m/z (ESI): 657.2 [M+H]⁺.

### Step 2: N-(1-(5-(6-((1-aminocyclopropyl)methoxy)-3-cyanopyrazolo[1,5-a]pyridin-4-yl)pyridin-2-yl)-4-methylpiperidin-4-yl)-3-chloropicolinamide

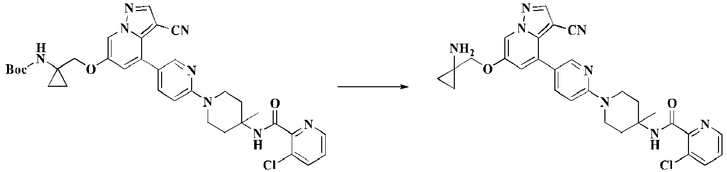

*N*-(1-(5-(6-((1-Aminocyclopropyl)methoxy)-3-cyanopyrazolo[1,5-*a*]pyridin-4-yl)pyridin-2-yl)-4-methylpiperidin-4-yl)-3-chloropicolinamide was obtained by using *tert-*butyl (1-(((4-(6-(4-(3-chloropicolinamido)-4-methylpiperidin-1-yl)pyridin-3-yl)-3-cyanopyrazolo[1,5-*a*]pyridin-6-yl)oxy)methyl)cyclopropyl)carbamate as raw material with reference to step 5 of embodiment **1.**

MS m/z (ESI): 557.2 [M+H]⁺.

### Embodiment 92

### 3-Chloro-N-(1-(5-(3-cyano-6-((1-cyanocyclopropyl)methoxy)pyrazolo[1,5-a]pyridin-4-yl)pyridin-2-yl)-4-methylpiperidin-4-yl)picolinamide

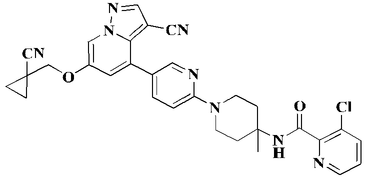

### Step 1: 3-chloro-N-(1-(5-(3-cyano-6-hydroxypyrazolo[1,5-a]pyridin-4-yl)pyridin-2-yl)-4-methylpiperidin-4-yl)picolinamide

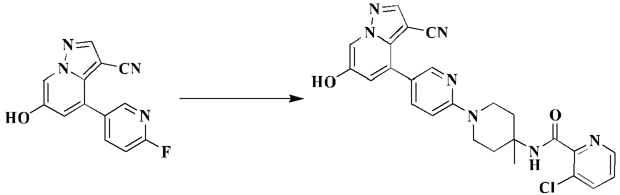

3-Chloro-*N*-(1-(5-(3-cyano-6-hydroxypyrazolo[1,5-*a*]pyridin-4-yl)pyridin-2-yl)-4-methylpiperidin-4-yl)picolinamide was obtained by using 4-(6-fluoropyridin-3-yl)-6-hydroxypyrazolo[1,5-*a*]pyridine-3-carbonitrile as raw material with reference to steps 1-3 of embodiment 85.

MS m/z (ESI):488.1 [M+H]⁺.

### Step 2: 3-chloro-N-(1-(5-(3-cyano-6-((1-cyanocyclopropyl)methoxy)pyrazolo[1,5-a]pyridin-4-yl)pyridin-2-yl)-4-methylpiperidin-4-yl)picolinamide

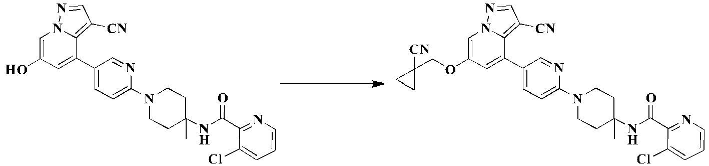

3-Chloro-*N-*(1-(5-(3-cyano-6-((1-cyanocyclopropyl)methoxy)pyrazolo[1,5-***a*]pyridin-4-yl)pyridin-2-yl)-4-methylpiperidin-4-yl)picolinamide** was obtained by using 3-chloro-*N*-(1-(5-(3-cyano-6-hydroxypyrazolo[1,5-*a*]pyridin-4-yl)pyridin-2-yl)-4-methylpiperidin-4-yl)picolinamide and (1-cyanocyclopropyl)methyl 4-methylbenzenesulfonate as raw materials with reference to step 2 of embodiment 27.

MS m/z (ESI): 567.1 [M+H]⁺.

¹H NMR (400 MHz, DMSO-d₆) δ 8.66 (d, J = 2.3 Hz, 1H), 8.60 (s, 1H), 8.53 (d, J = 4.7 Hz, 1H), 8.34 (d, J = 2.5 Hz, 1H), 8.28 (s, 1H), 8.03 - 7.98 (m, 1H), 7.82 - 7.74 (m, 1H), 7.52 - 7.48 (m, 1H), 7.39 (d, J = 1.9 Hz, 1H), 6.99 (d, J = 8.7 Hz, 1H), 4.20 (s, 2H), 4.10 - 4.01 (m, 2H), 3.40 - 3.34 (m, 2H), 2.34 - 2.30 (m, 2H), 1.63 - 1.54 (m, 2H), 1.45 (s, 3H), 1.44 - 1.39 (m, 2H), 1.22 -1.16 (m, 2H).

### Embodiment 93

### N-(1-(5-(3-Cyano-6-((1-cyanocyclopropyl)methoxy)pyrazolo[1,5-a]pyridin-4-yl)pyridin-2-yl)-4-methylpiperidin-4-yl)-3-fluoropicolinamide

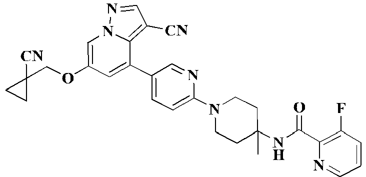

### Step 1: N-(1-(5-(3-cyano-6-hydroxypyrazolo[1,5-a]pyridin-4-yl)pyridin-2-yl)-4-methylpiperidin-4-yl)-3-fluoropicolinamide

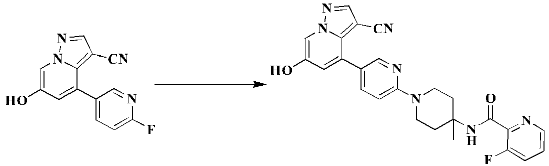

*N*-(1-(5-(3-cyano-6-hydroxypyrazolo[1,5-*a*]pyridin-4-yl)pyridin-2-yl)-4-methylpiperidin-4-yl)-3-fluoropicolinamide was obtained by using 4-(6-fluoropyridin-3-yl)-6-hydroxypyrazolo[1,5-*a*]pyridine-3-carbonitrile as raw material with reference to steps 1-3 of embodiment 85.

MS m/z (ESI):472.1 [M+H]⁺.

### Step 2: N-(1-(5-(3-cyano-6-((1-cyanocyclopropyl)methoxy)pyrazolo[1,5-a]pyridin-4-yl)pyridin-2-yl)-4-methylpiperidin-4-yl)-3-fluoropicolinamide

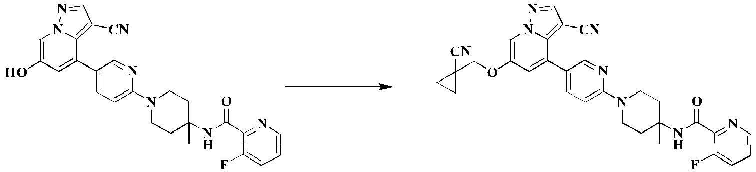

*N*-(1-(5-(3-Cyano-6-((1-cyanocyclopropyl)methoxy)pyrazolo[1,5-*a*]pyridin-4-yl)pyridin-2-yl)-4-methylpiperidin-4-yl)-3-fluoropicolinamide was obtained by using *N*-(1-(5-(3-cyano-6-hydroxypyrazolo[1,5-*a*]pyridin-4-yl)pyridin-2-yl)-4-methylpiperidin-4-yl)-3-fluoropicolinamide and (1-cyanocyclopropyl)methyl 4-methylbenzenesulfonate as raw materials with reference to step 2 of embodiment 31.

MS m/z (ESI): 551.2 [M+H]⁺.

¹H NMR (400 MHz, DMSO-d₆) δ 8.66 (d, J = 2.1 Hz, 1H), 8.60 (s, 1H), 8.51 - 8.43 (m, 1H), 8.35 (d, J = 2.5 Hz, 1H), 8.21 (s, 1H), 7.89 - 7.81 (m, 1H), 7.78 (dd, J = 8.8, 2.6 Hz, 1H), 7.68 - 7.58 (m, 1H), 7.39 (d, J = 2.1 Hz, 1H), 6.99 (d, J = 8.9 Hz, 1H), 4.20 (s, 2H), 4.10 - 3.98 (m, 2H), 3.31 - 3.24 (m, 2H), 2.40 - 2.27 (m, 2H), 1.69 - 1.56 (m, 2H), 1.46 (s, 3H), 1.45 - 1.40 (m, 2H), 1.24 - 1.16 (m, 2H).

### Embodiment 94

### 3-Chloro-N-(1-(5-(3-cyano-6-((3-cyanooxetan-3-yl)methoxy)pyrazolo[1,5-a]pyridin-4-yl)pyridin-2-yl)-4-methylpiperidin-4-yl)picolinamide

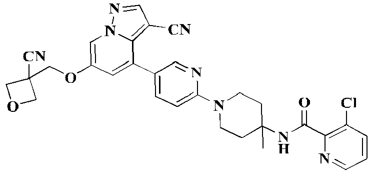

6-((3-Cyanooxetan-3-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 3-chloro-*N-*(1-(5-(3-cyano-6-hydroxypyrazolo[1,5-*a*]pyridin-4-yl)pyridin-2-yl)-4-methylpiperidin-4-yl)picolinamide as raw material with reference to embodiment 31.

MS m/z (ESI): 583.2 [M+H]⁺.

### Embodiment 95

### 3-Chloro-N-(1-(5-(3-cyano-6-(3-hydroxy-3-methylazetidin-1-yl)pyrazolo[1,5-a]pyridin-4-yl)pyridin-2-yl)-4-methylpiperidin-4-yl)picolinamide

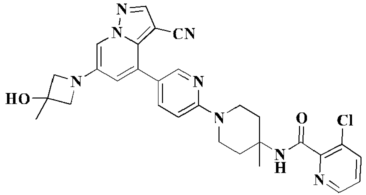

3-Chloro-*N*-(1-(5-(3-cyano-6-(3-hydroxy-3-methylazetidin-1-yl)pyrazolo[1,5-*a*]pyridin-4-yl)pyridin-2-yl)-4-methylpiperidin-4-yl)picolinamide was obtained by using 3-methylazetidin-3-ol and *N*-(1-(5-(6-bromo-3-cyanopyrazolo[1,5-*a*]pyridin-4-yl)pyridin-2-yl)-4-methylpiperidin-4-yl)-3-chloropicolinamide as raw materials with reference to step 1 of embodiment 31.

MS m/z (ESI):557.2 [M+H]⁺.

¹H NMR (400 MHz, CDCl₃) δ 8.46 (d, *J* = 3.7 Hz, 1H), 8.33 (s, 1H), 8.14 (s, 1H), 7.94 (s, 1H), 7.83 (d, *J* = 8.6 Hz, 1H), 7.72 (s, 1H), 7.38 (dd, *J* = 8.1, 4.5 Hz, 1H), 6.92 (d, *J* = 9.6 Hz, 1H), 6.75 (s, 1H), 4.15 (s, 2H), 3.91 (d, *J=* 7.3 Hz, 2H), 3.82 (d, *J=* 7.2 Hz, 2H), 3.49 (s, 2H), 2.49 (s, 2H), 2.24 - 2.18 (m, 1H), 1.88 - 1.80 (m, 2H), 1.68 - 1.65 (m, 3H), 1.25 (s, 3H).

### Embodiment 96

### 3-Chloro-N-(1-(5-(3-cyano-6-(3-cyano-3-methylazetidin-1-yl)pyrazolo[1,5-a]pyridin-4-yl)pyridin-2-yl)-4-methylpiperidin-4-yl)picolinamide

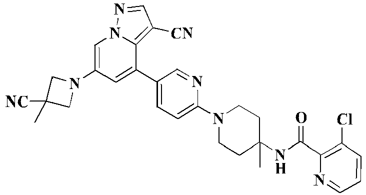

3-Chloro-*N*-(1-(5-(3-cyano-6-(3-cyano-3-methylazetidin-1-yl)pyrazolo[1,5-*a*]pyridin-4-yl)pyridin-2-yl)-4-methylpiperidin-4-yl)picolinamide was obtained by using 3-methylazetidin-3-carbonitrile and *N*-(1-(5-(6-bromo-3-cyanopyrazolo[1,5-*a*]pyridin-4-yl)pyridin-2-yl)-4-methylpiperidin-4-yl)-3-chloropicolinamide as raw materials with reference to step 1 of embodiment 31.

MS m/z (ESI):566.2 [M+H]⁺.

¹H NMR (400 MHz, CDCl₃) δ 8.46 (dd, *J* = 4.5, 1.3 Hz, 1H), 8.31 (d, *J* = 2.3 Hz, 1H), 8.16 (s, 1H), 7.91 (s, 1H), 7.83 (dd, *J* = 8.1, 1.3 Hz, 1H), 7.71 (d, *J* = 1.9 Hz, 1H), 7.37 (dd, *J* = 8.1, 4.5 Hz, 1H), 6.82 (d, *J=* 8.9 Hz, 1H), 6.67 (d, *J=* 1.9 Hz, 1H), 4.28 (d, *J=* 7.0 Hz, 2H), 4.09 (d, *J* = 13.6 Hz, 2H), 3.88 (d, *J* = 7.0 Hz, 2H), 3.39 (t, *J* = 11.1 Hz, 2H), 2.43 (d, *J* = 13.8 Hz, 2H), 1.84 (d, *J=* 4.3 Hz, 1H), 1.82 (s, 3H), 1.78 (d, *J=* 4.1 Hz, 1H), 1.60 (s, 3H).

### Embodiment 97

### 3-Chloro-N-(1-(5-(3-cyano-6-((3-hydroxyoxetan-3-yl)ethynyl)pyrazolo[1,5-a]pyridin-4-yl)pyridin-2-yl)-4-methylpiperidin-4-yl)picolinamide

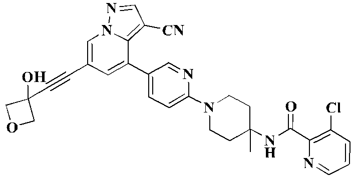

3-Chloro-*N*-(1-(5-(3-cyano-6-((3-hydroxyoxetan-3-yl)ethynyl)pyrazolo[1,5-*a*]pyridin-4-yl)pyridin-2-yl)-4-methylpiperidin-4-yl)picolinamide was obtained by using 3-ethynyloxetan-3-ol and *N*-(1-(5-(6-bromo-3-cyanopyrazolo[1,5-*a*]pyridin-4-yl)pyridin-2-yl)-4-methylpiperidin-4-yl)-3-chloropicolinamide as raw materials with reference to step 2 of embodiment 8.

MS m/z (ESI):568.2 [M+H]⁺.

¹H NMR (400 MHz, CDCl₃) δ 8.57 (s, 1H), 8.39 (d, *J* = 3.9 Hz, 1H), 8.24 (s, 2H), 7.84 (s, 1H), 7.76 (d, *J* = 8.1 Hz, 1H), 7.67 - 7.60 (m, 1H), 7.31 (dd, *J* = 7.8, 4.6 Hz, 1H), 7.23 (s, 1H), 6.77 (d, *J* = 9.0 Hz, 1H), 4.87 (d, *J* = 6.6 Hz, 2H), 4.64 (d, *J* = 6.7 Hz, 2H), 3.99 (d, *J* = 13.6 Hz, 2H), 3.34 (t, *J* = 11.0 Hz, 2H), 2.36 (d, *J* = 14.0 Hz, 2H), 1.73 (d, *J* = 3.8 Hz, 2H), 1.53 (s, 3H).

### 2. Biological tests and evaluation of compounds

The present disclosure is further described below in conjunction with test embodiments to explain the disclosure, but these embodiments are not meant to limit the scope of the disclosure.

### I. Testing enzymology experiment

### Test embodiment 1. Determination of the inhibitory effect of compounds of the present disclosure on the activity of RET wild-type and mutant kinases

### 1. Experimental purpose:

The purpose of this test embodiment is to measure the inhibitory ability of the compounds on the activity of RET wild-type and mutant kinases.

### 2.1 Experimental instruments:

Centrifuge (Eppendorf 5810R);
Microplate reader (BioTek Synergy H1);
Pipette (Eppendorf & Rainin).

### 2.2 Experimental reagents:

RET enzyme was purchased from Carna Company, and the article number was 08-159;
RET M918T enzyme was purchased from Carna Company, and the article number was 08-508;
KIF5B-RET was purchased from SignalChem Company, the article number was R02-19FG-05;
CCDC6-RET was purchased from SignalChem Company, the article number was R02-19BG-05;
RET V804M enzyme was purchased from Thermofisher Company, and the article number was PV6223;
RET V804L enzyme was purchased from Thermofisher Company, and the article number was PV4397;
HTRF KinEASE-TK kit was purchased from Cisbio Company, and the article number was 62TK0PEC;
ATP was purchased from Thermofisher Company, and the article number was PV3227;
384-well plate was purchased from PerkinElmer Company, and the article number was 6007290.

### 2.3 Test compounds:

The compounds of the embodiments of the present disclosure were self-made.

### 3. Experimental methods:

In this experiment, a homogeneous time-resolved fluorescence (HTRF) method was used for the detection of compounds on RET kinase activity. The experiment was carried out in 384-well plate, different concentrations of compound solutions were prepared using experimental buffers (25 mM HEPES, 10 mM MgCl₂, 0.01% TritonX-100) and were added to 384-well plate, then diluted RET, RET M918T, CCDC6-RET, KIF5B-RET, RET V804M or RET V804L kinase solutions (0.01-2 nM) and substrate TK-substrate biotin (500 nM-1 µM) and Km (0.19-200 µM) concentrations of ATP solution were added thereto respectively, the total reaction system was 10 µL, the mixture was mixed well by centrifugation at 1000 rpm for 1 minute; and the reaction was carried out at room temperature for 45 minutes, 10 µL of a mixed solution of Sa-XL665 and TK-ab-Cryptate prepared with assay solution was added, then the mixture was mixed well by centrifugation at 1000 rpm for 1 minute, and readings at 665 nm and 620 nm were recorded using a BioTek Synergy H1 instrument after reaction was carried out for 1 hour at room temperature.

### 4. Experimental data processing methods:

The IC₅₀ values were obtained by taking readings with a BioTek Synergy H1 instrument, the readings at 665 nm and 620 nm were recorded, and the ratio (665 nm/620 nm) was calculated, and then the inhibition rate was calculated, and the concentrations and the inhibition rates were fitted to a nonlinear regression curve using Graphpad Prism software.

### 5. Experimental results:

According to the above test method, the test data obtained of the compounds of specific embodiments against multiple mutant kinases of RET were shown in Table 8.

**Table 8: The IC₅₀ values of the compounds for inhibiting the activity of multiple mutant kinases of RET**

| Embodiment number | RET M918T IC₅₀ (nM) | CCDC6-RET IC₅₀ (nM) | KIF5B-RET IC₅₀ (nM) | RET V804M IC₅₀ (nM) | RET V804L IC₅₀ (nM) |
|---|---|---|---|---|---|
| 7 | 1.06 | NA | NA | NA | NA |
| 8 | 0.67 | NA | NA | NA | NA |
| 17 | 0.38 | NA | NA | NA | NA |
| 18 | 0.66 | NA | 0.97 | 1.8 | 0.74 |
| 31 | 0.47 | NA | NA | NA | NA |
| 63 | 0.21 | 0.85 | 0.53 | 0.6 | 0.24 |
| 64 | 0.17 | 1.74 | 0.36 | 3.0 | 0.23 |
| 77 | 0.41 | 0.98 | 0.42 | 0.6 | 0.49 |
| 85 | 0.79 | NA | NA | NA | NA |
| 97 | 0.37 | 1.72 | 0.50 | 1.1 | 0.28 |

| | | | | | |
|---|---|---|---|---|---|
| Note: "NA" in the table means no test was performed. | | | | | |

### 6. Experimental conclusion:

The compounds of embodiments of the present disclosure show good inhibitory activity against multiple mutant kinases of RET and significant activity in the drug-resistant mutations RET M918T, KIF5B-RET and RET V804L.

### Test embodiment 2. Determination of the inhibitory effect of the compounds of the present disclosure on the activity of KDR kinase

### 1. Experimental purpose:

The purpose of this test embodiment was to measure the inhibitory ability of compounds on the activity of KDR kinases.

### 2.1 Experimental instruments:

Centrifuge (Eppendorf 5810R);
Microplate reader (BioTek Synergy H1);
Pipette (Eppendorf & Rainin).

### 2.2 Experimental reagents:

KDR kinase was purchased from Carna Company, and the article number was 08-191;
HTRF KinEASE-TK kit was purchased from Cisbio Company, and the article number was 62TK0PEC;
ATP was purchased from Thermofisher Company, and the article number was PV3227;
384-well plate was purchased from PerkinElmer Company, and the article number was 6007290.

### 2.3 Test compounds:

The compounds of the embodiments of the present disclosure were self-made.

### 3. Experimental methods:

In this experiment, the homogeneous time-resolved fluorescence (HTRF) method was used for the detection of compounds on KDR kinase activity. The experiment was carried out in 384-well plate, different concentrations of compound solutions were prepared using experimental buffers (25 mM HEPES, 10 mM MgCl₂, 0.01% TritonX-100) and was added to 384-well plate, then diluted KDR kinase solutions (0.05 nM) and substrate TK-substrate biotin (500 nM-1 µM) and Km (0.19-200 µM) concentrations of ATP solution were added, the total reaction system was 10 µL, the mixture was mixed well by centrifugation at 1000 rpm for 1 min; and after the reaction was carried out at room temperature for 45 minutes, 10 µL of a mixed solution of Sa-XL665 and TK-ab-Cryptate prepared with the assay solution was added, then the mixture was mixed well by centrifugation at 1000 rpm for 1 min, and readings at 665 nm and 620 nm were recorded using a BioTek Synergy H1 instrument after reaction was carried out for 1 hour at room temperature.

### 4. Experimental data processing methods:

The IC₅₀ values were obtained by taking readings with a BioTek Synergy H1 instrument, the readings at 665 nm and 620 nm were recorded, and the ratio (665 nm/ 620 nm) was calculated, and then the inhibition rate was calculated, and the concentrations and the inhibition rates were fitted to a nonlinear regression curve using Graphpad Prism software.

### 5. Experimental results:

The test data of specific embodiments obtained through the above test methods were shown in Table 9:

**Table 9: Relative IC₅₀ values of compounds inhibiting the activity of RET kinases and KDR kinases**

| Embodiment number | RET IC₅₀ (nM) | KDR IC₅₀ (nM) | Ratio(KDR/RET) |
|---|---|---|---|
| 1 | 0.47 | 28 | 59 |
| 6 | 0.79 | 27.7 | 35 |
| 7 | 0.69 | 11 | 16 |
| 8 | 0.48 | 16 | 34 |
| 9 | 0.76 | 70.0 | 92 |
| 12 | 1.3 | 69 | 55 |
| 17 | 0.39 | 8.2 | 21 |
| 18 | 0.31 | 23 | 73 |
| 21 | 0.41 | 9.4 | 23 |
| 22 | 0.32 | 24 | 75 |
| 27 | 0.32 | 88 | 280 |
| 31 | 0.75 | 23 | 30 |
| 32 | 0.49 | 1.8 | 4 |
| 33 | 0.4 | 5.6 | 14 |
| 34 | 0.7 | 4.7 | 7 |
| 35 | 0.49 | 6.2 | 13 |
| 36 | 0.83 | 213.3 | 257 |
| 37 | 0.71 | 60.4 | 85 |
| 41 | 0.4 | 11 | 27 |
| 42 | 1.56 | 104.8 | 67 |
| 46 | 0.26 | 6.5 | 25 |
| 47 | 0.40 | 176 | 438 |
| 48 | 0.42 | 13 | 30 |
| 53 | 0.8 | 21.6 | 27 |
| 54 | 0.34 | 9 | 26 |
| 55 | 0.31 | 37.5 | 121 |
| 57 | 1.1 | 209.3 | 187 |
| 59 | 0.58 | 183.9 | 317 |
| 60 | 0.58 | 257.1 | 445 |
| 61 | 0.35 | 11.2 | 32 |
| 62 | 0.48 | 35.24 | 73 |
| 63 | 0.34 | 21.7 | 63 |
| 64 | 0.38 | 44.34 | 118 |
| 65 | 0.33 | 14.5 | 44 |
| 66 | 0.25 | 3.0 | 12 |
| 67 | 0.25 | 4.0 | 16 |
| 68 | 0.44 | 18.9 | 43 |
| 72 | 0.37 | 7.4 | 20 |
| 74 | 1.8 | 246.6 | 137 |
| 77 | 0.37 | 6.90 | 19 |
| 85 | 0.38 | 46 | 122 |
| 88 | 1.2 | 61 | 49 |
| 89 | 1.1 | 8.9 | 8 |
| 91 | 1.54 | 204.4 | 133 |
| 92 | 0.85 | 185.3 | 218 |
| 93 | 1.1 | 150.0 | 136 |
| 95 | 1.0 | 24 | 24 |
| 96 | 0.72 | 15.8 | 22 |

### 6. Experimental conclusion:

The above data show that the compounds of the embodiments shown in the present disclosure have strong inhibitory effect on RET kinase activity and poor inhibitory effect on KDR kinase activity. Comparing the two groups of data, it can be seen that the series of compounds of the present disclosure have high selectivity in inhibiting KDR/RET kinase activity.

### II. Testing cytological experiments

### Test embodiment 1. Determination of the inhibitory effect of compounds of the present disclosure on the proliferation activity of TT cells

### 1. Experimental purpose:

The purpose of this test embodiment was to measure the inhibitory effect of the compounds on the proliferative activity of TT cells.

### 2.1 Experimental instruments:

Microplate reader (BioTek Synergy H1);
Pipette (Eppendorf & Rainin).

### 2.2 Experimental reagents:

TT cells were purchased from the cell bank of the Chinese Academy of Sciences.
Cell Titer-Glo cells were purchased from Promega Company, and the article number was G7573.

### 2.3 Test compounds:

The compounds of the embodiments of the present disclosure were self-made.

### 3. Experimental methods:

When TT cells were cultured to the appropriate fusion level, TT cells were collected, and the cells were adjusted to the appropriate cell concentration using complete medium, and the cell suspension was spread in a 96-well plate, 90 µL per well, and placed to the wall of a 37°C, 5% CO₂ incubator overnight; and compound solutions of different concentrations were prepared using DMSO and culture medium; and a solvent control was set, the compound solution was added to a 96-well plate, 10 µL per well, and placed to the wall of a 37°C, 5% CO₂ incubator for 72 hours; CellTiter- Glo solution was added thereto and the mixture was mixed well by shaking, incubated for 10 minutes in the dark, and read by BioTek Synergy H1 microplate reader.

### 4. Experimental data processing methods:

The IC₅₀ values were obtained by using luminescence signal values to calculate the inhibition rate, the concentration and the inhibition rate were fitted to a nonlinear regression curve using Graphpad Prism software.

### 5. Experimental results:

**Table 10**

| Embodiment number | TT (MTC, RET C634W) IC₅₀ (nM) |
|---|---|
| 1 | 11.53 |
| 7 | 11.00 |
| 8 | 17.00 |
| 9 | 29.30 |
| 17 | 13.13 |
| 18 | 18.99 |
| 21 | 18.11 |
| 22 | 20.71 |
| 25 | 14.97 |
| 26 | 18.97 |
| 31 | 6.59 |
| 32 | 2.07 |
| 41 | 9.45 |
| 44 | 12.22 |
| 45 | 9.17 |
| 46 | 5.87 |
| 47 | 13.30 |
| 48 | 19.60 |
| 53 | 24.67 |
| 54 | 9.00 |
| 55 | 28.53 |
| 60 | 28.40 |
| 63 | 14.11 |
| 64 | 27.78 |
| 65 | 12.65 |
| 66 | 5.18 |
| 67 | 6.84 |
| 71 | 8.65 |
| 72 | 13.86 |
| 73 | 5.95 |
| 75 | 24.39 |
| 76 | 3.48 |
| 77 | 4.10 |
| 78 | 13.92 |
| 79 | 10.51 |
| 80 | 17.30 |
| 81 | 13.77 |
| 82 | 17.77 |
| 85 | 9.44 |
| 86 | 14.62 |
| 87 | 23.63 |
| 91 | 29.61 |
| 92 | 27.45 |
| 95 | 15.01 |

### 6. Experimental conclusion:

The above data show that the compounds of embodiments of the present disclosure have good inhibitory effect on the proliferation of TT cells.

### Test embodiment 2. Determination of the inhibitory effect of compounds of the present disclosure on the proliferation activity of Ba/F3 HIFSB-RET cells

### 1. Experimental purpose:

To measure the inhibitory effect of the compounds on the proliferative activity of Ba/F3 KIF5B-RET cells.

### 2. Instruments and reagents:

### 2.1 Experimental instruments:

Microplate reader (BioTek Synergy H1);
Pipette (Eppendorf & Rainin).

### 2.2 Experimental reagents:

Ba/F3 KIF5B-RET was provided by Kyinno biotechnology Co., Ltd, and the cell number was CVCL_UE86, which can be found on the cell information website https://web.expasy.org/cellosaurus/, the stably transformed cell line does not need to rely on IL-3 for growth;
Cell Titer-Glo cells were purchased from Promega Company, and the article number was G7573.

### 2.3 Test compounds:

The compounds of the embodiments of the present disclosure were self-made.

### 3. Experimental methods:

When Ba/F3 KIF5B-RET cells were cultured to the appropriate cell density, cells were collected, and the cells were adjusted to the appropriate cell concentration using complete medium, and the cell suspension was spread in a 96-well plate, 90 µL per well, and placed in the wall of a 37°C, 5% CO₂ incubator overnight; compound solutions of different concentrations were prepared using DMSO and culture medium; a solvent control was set, and the compound solution was added to a 96-well plate with 10 µL per well at 37°C in a 5% CO₂ incubator for 72 to 144 hours, CellTiter- Glo solution was added thereto and mixed well by shaking, incubated for 10 minutes in the dark, and read by BioTek Synergy H1 microplate reader.

### 4. Experimental data processing methods:

The IC₅₀ values were obtained by using the luminescence signal values to calculate the inhibition rate, the concentrations and the inhibition rates were fitted to a nonlinear regression curve using Graphpad Prism software.

### 5. Experimental results:

**Table 11**

| Embodiment number | Ba/F3 KIF5B-RET IC₅₀ (nM) |
|---|---|
| 1 | 24.62 |
| 7 | 13.00 |
| 8 | 12.00 |
| 9 | 28.88 |
| 18 | 22.62 |
| 19 | 27.83 |
| 22 | 16.94 |
| 25 | 13.54 |
| 27 | 15.90 |
| 44 | 15.30 |
| 45 | 7.06 |
| 53 | 30.64 |
| 55 | 19.61 |
| 59 | 33.97 |
| 60 | 17.96 |
| 61 | 16.77 |
| 63 | 11.05 |
| 64 | 22.56 |
| 65 | 7.03 |
| 66 | 14.84 |
| 67 | 5.33 |
| 68 | 26.30 |
| 71 | 5.59 |
| 72 | 8.59 |
| 73 | 12.04 |
| 75 | 34.73 |
| 76 | 6.04 |
| 77 | 4.79 |
| 78 | 15.62 |
| 79 | 13.66 |
| 80 | 23.93 |
| 81 | 16.79 |
| 82 | 21.93 |
| 86 | 15.42 |
| 87 | 18.55 |
| 92 | 32.10 |
| 95 | 22.46 |

### 6. Experimental conclusion:

The above data show that the compounds of embodiments of the present disclosure have good inhibitory effect on the proliferation of Ba/F3 KIF5B-RET cells.

### Test embodiment 3. The inhibitory effect of the compounds of the present disclosure on the phosphorylation of ERK, a downstream signal factor of TT cells

### 1. Experimental purpose:

To detect the inhibitory effect of the compounds on the phosphorylation level of ERK, a downstream signal factor of TT cells.

### 2. Experimental instruments and reagents:

### 2.1 Experimental instruments:

Imager (Biorad ChemiDoc^{™}MP);
Pipette (Eppendorf & Rainin).

### 2.2 Experimental reagents:

pERK antibody was purchased from Cell Signaling Technology Company, the article number was 4370S;
Total ERK antibody was purchased from Cell Signaling Technology Company, the article number was 4696S;
The internal reference GAPDH was purchased from Cell Signaling Technology Company, the article number was 5174S;
The fluorescent secondary antibodies were purchased from LI-COR Company, the article numbers were PIN 925-68071 and PIN 926-32210.

### 2.3 Test compounds:

The compounds of the embodiments of the present disclosure were self-made.

### 3. Experimental methods:

In this experiment, the inhibitory effect of compounds on the phosphorylation level of ERK, a downstream signaling factor in TT cells was measured by Western Blot method. When TT cells were cultured to the appropriate fusion level, the cells were collected and adjusted to the appropriate cell concentration using complete medium, the cell suspension was spread in a 24-well plate, 1 mL per well, and placed in ther wall of a 37°C, 5% CO₂ incubator overnight, and compound dilutions of different concentrations (3.7 nM, 11.1 nM, 33.3 nM, 100 nM, 300 nM) were added at 37°C and acted for 2 hours; the cell supernatant was aspirated, washed one time with PBS, and the proteins were collected with lysate; after protein denaturation, Western blot experiments were performed: performing protein electrophoresis at 120V for approximately 75 minutes, followed by 45 minutes of transfer to PVDF membranes at 10V with a semi-dry transfer instrument, enclosing at 5% BSA for 1 hour at room temperature, and then PVDF membranes were cut into strips of appropriate size and incubated with prepared antibody dilutions overnight at 4°C and washed 6 times with TBST, followed by incubating with goat anti-mouse secondary antibody and goat anti-rabbit secondary antibody for 1 hour at room temperature, and the membrane was washed 6 times with TBST for imaging in the Biorad ChemiDoc^{™} MP Imaging System.

### 4. Experimental data processing methods:

The inhibitory effect of compounds on ERK phosphorylation levels in TT cells at different concentrations was determined by detecting protein bands.

### 5. Experimental results:

Embodiment 63 and embodiment 64 can significantly inhibit the phosphorylation level of ERK in TT cells with a dose-dependent effect. After the compound was incubated with TT cells for 2 hours at 37°C, embodiment 63 almost completely inhibited ERK phosphorylation at 300 nM, 100 nM, 33.3 nM, 11.1 nM; and at 3.7 nM, it could inhibit about half of the ERK phosphorylation level, whereas embodiment 64 can completely inhibit the phosphorylation of ERK at 300 nM and 100 nM, the degree of inhibition was reduced at 33.3 nM, at 11.1 nM, it could inhibit half of the ERK phosphorylation level, and the inhibition level was weaker at 3.7 nM.

### 6. Experimental conclusion:

According to the above scheme, the compounds shown in the present disclosure show dose-dependent inhibitory effect on the phosphorylation of ERK, a downstream signaling factor of TT cells.

### III. Determination of Balb/C mouse pharmacokinetics

### 1. Research purpose:

The pharmacokinetic behavior of the following compound embodiments, administered orally at a dose of 5 mg/kg in plasma in mice, was studied using Balb/C mice as test animals.

### 2. Test scheme:

### 2.1 Test drugs:

The compounds of the embodiments of the present disclosure were self-made.

### 2.2 Test animals:

Balb/C Mouse 6/embodiment, male, Shanghai Jiesijie Laboratory Animal Co., Ltd, Animal Production License No.(SCXK (Shanghai) 2013-0006 N0.311620400001794).

### 2.3 Administration:

Balb/C mice, males; p.o. after overnight fasting, respectively, at a dose of 5 mg/kg, administered in a volume of 10 mL/kg.

### 2.4 Sample preparation:

0.5%CMC-Na (1%Tween80) was dissolved by ultrasound, and prepared into clear solution or uniform suspension.

### 2.5 Sample collection:

Before and after administration, 0.1 mL of blood was collected from mice at 0, 0.5, 1, 2, 4, 6, 8 and 24 hours by orbital collection, placed in EDTA-K₂ tubes, centrifuged at 6000 rpm at 4°C for 6 minutes to separate plasma, and stored at -80°C.

### 2.5 Sample handling:

1) 40 µL of plasma sample was precipitated by adding 160 µL of acetonitrile, mixed and centrifuged at 3500 × g for 5-20 minutes.
2) 100 µL of the supernatant solution after treatment was taken to analyze the concentration of the test compound by LC/MS/MS.

### 2.6 Liquid phase analysis

Liquid phase conditions: Shimadzu LC-20AD pump
Mass spectrometry conditions: AB Sciex API 4000 mass spectrometer
Chromatographic column: phenomenex Gemiu 5 µM C18 50 × 4.6 mm
Mobile phase: Liquid A was 0.1% formic acid aqueous solution, liquid B was acetonitrile
Flow rate: 0.8 mL/min
Elution time: 0-4.0 minutes, the eluent was as follows:

**Table 12**

| Time/minute | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main pharmacokinetic parameters were calculated by WinNonlin 6.1 and the results of the mouse pharmacokinetic experiments were shown in Table 13 below.

**Table 13 Results of pharmacogenetic experiments in mice**

| Embodiment Number | Pharmacokinetic experiment (5 mg/kg) | | | | | |
|---|---|---|---|---|---|---|
| | Peak time | Blood concentration | Curve area | Curve area | Half-life | Average residence time |
| | tₘₐₓ (ng/mL) | Cₘₐₓ (ng/mL) | AUC₀₋ₜ (ng/mL×h) | AUC_{0-∞} (ng/mL×h) | t_{1/2}(h) | MRT(h) |
| 7 | 0.5 | 3283.3 | 13821.5 | 13830.0 | 2.18 | 3.76 |
| 8 | 0.5 | 3706.7 | 21369.7 | 21511.8 | 3.37 | 4.91 |
| 9 | 0.5 | 1920.0 | 16076.7 | 16389.2 | 4.2 | 5.84 |
| 18 | 0.5 | 2786.7 | 18167.5 | 18230.7 | 2.9 | 4.61 |
| 31 | 0.5 | 3120.0 | 18865.2 | 18904.9 | 2.66 | 4.71 |
| 36 | 0.5 | 1490.0 | 6208.1 | 6220.8 | 2.19 | 3.80 |
| 44 | 0.5 | 1616.7 | 13953.1 | 14019.7 | 3.45 | 5.22 |
| 55 | 2.0 | 1943.3 | 21624.6 | 22084.0 | 4.26 | 6.51 |
| 59 | 0.5 | 1326.7 | 7343.3 | 7369.3 | 2.34 | 4.27 |
| 60 | 0.5 | 3083.0 | 27955.0 | 28150.0 | 3.26 | 5.56 |
| 62 | 0.5 | 2816.7 | 13773.0 | 13789.2 | 2.4 | 4.07 |
| 63 | 1.0 | 2936.7 | 10978.5 | 10981.2 | 1.3 | 2.90 |
| 64 | 1.0 | 3920.0 | 14458.5 | 14460.7 | 1.2 | 2.70 |
| 65 | 2.0 | 2400.0 | 20159.0 | 20251.0 | 3.05 | 5.24 |
| 68 | 0.5 | 1703.3 | 7208.0 | 7218.5 | 1.73 | 3.47 |
| 77 | 0.5 | 4680.0 | 51613.2 | 51946.2 | 3.2 | 6.0 |
| 92 | 0.5 | 9340.0 | 24470.9 | 24473.6 | 1.7 | 2.85 |

### 4. Experimental conclusion:

It can be seen from the results of mouse pharmacokinetic experiments in the table that the compounds of the embodiments of the present disclosure show good metabolic properties, and both the exposure AUC and the maximum blood drug concentration Cₘₐₓ show good performance.

### IV. Tumor suppression experiment on Ba/F3 KIF5B-RET transplanted tumor model

### 1. Experimental purpose:

To evaluate the anti-tumor activity of the test compounds against the subcutaneous transplanted tumor of Ba/F3 KIF5B-RET cells in nude mice.

### 2. Experimental instruments and reagents:

### 2.1 Instruments:

Ultra Clean Bench (BSC-1300II A2, Shanghai Boxun Industrial Co., Ltd. Medical Equipment Factory);
CO₂ incubator (311,Thermo);
Centrifuge (Centrifuge 5720R, Eppendorf);
Automatic cell counter (Countess II, Life);
Pipette (10-20 µL, Eppendorf);
Microscope (TS100, Nikon);
Vernier caliper (500-196, Sanfeng, Japan);
Cell culture flask (T25/T75/T225, Corning).

### 2.2 Reagents:

RPMI1640 (22400-089, Gibco);
Fetal bovine serum (FBS)(10099-141, Gibco);
Phosphate buffer (PBS)(10010-023, Gibco).

### 2.3 Test compounds:

The compounds of the embodiments of the present disclosure were self-made.

### 3. Experimental operation:

Ba/F3 KIF5B-RET cells were removed from the cell bank, resuscitated and added to RPMI1640 medium (RPMI1640+10% FBS+1% Glu+1% P/S), then cultured in a CO₂ incubator (incubator temperature was 37°C, CO₂ concentration was 5%), and when the cell number expanded to the required number for *in vivo* inoculation, Ba/F3 KIF5B-RET cells were collected. The cells were counted with an automatic cell counter, resuspended with PBS according to the count results, made into a cell suspension (density was 2×10⁷/mL), and placed in an ice box for use.

Female BALB/c nude mice aged 6-8 weeks were used, weighing about 18-22g. Mice were reared in SPF animal room, and were reared in a single cage with 5 mice in each cage. All cages, bedding and water were sterilized at high temperature before use, and all animals were allowed to eat and drink freely. Nude mice were labeled with disposable universal ear tags for mice and rats before the start of the experiment, and the skin of the inoculation site was disinfected with 75% medical alcohol before inoculation. 0.1 mL (containing 2^{∗}10⁶ cells) of Ba/F3 KIF5B-RET cells were inoculated subcutaneously on the right back of each mouse. Group administration was initiated when the tumor volume reached 60-200 mm³, with 5 mice in each group. Each test compound was administered orally twice a day for 14 days. The tumor volume and the weight of mice were measured twice a week, and the tumor TGI (%) was calculated, and the tumor TGI (%) was calculated.

### 4. Data processing:

The tumor volume (mm³) was calculated as V=0.5*D*d*d, where D and d were the long and short diameters of the tumor, respectively.

### Calculation of TGI (%):

When there was no tumor regression, TGI (%) = [(1-(mean tumor volume at the end of the administration in a treatment group - mean tumor volume at the start of administration in the treatment group))/(mean tumor volume at the end of treatment in the solvent control group - mean tumor volume at the start of treatment in the solvent control group)] × 100%.

When there was tumor regression, TGI (%) = [1-(mean tumor volume at the end of administration in a treatment group - mean tumor volume at the beginning of administration in the treatment group)/mean tumor volume at the beginning of administration in the treatment group] × 100%.

### 5. Experimental results:

**Table 14 Pharmacodynamic parameters**

| **Grouping** | **Tumor volume (mm³, Mean±SD)** | | **TGI (%)** |
|---|---|---|---|
| | **Day 0** | **Day 14** | **Day 14** |
| 7 | 100.80±31.28 | 232.46±133.65 | 90.82 |
| 10 mg/kg | (101.24±35.15) | (1,536.07±263.57) | |
| 18 | 132.34±19.40 | 118.64±84.34 | 110.35 |
| 30 mg/kg | (133.86±28.86) | (1,366.89±291.81) | |
| 62 | 100.24±27.85 | 247.52±85.65 | 89.74 |
| 10 mg/kg | (101.24±35.15) | (1,536.07±263.57) | |
| 63 | 126.13±17.24 | 219.86±64.31 | 94.66 |
| 10 mg/kg | (126.52±16.11) | 1,882.69±968.48) | |
| 64 | 126.93±16.98 | 330.85±147.00 | 88.39 |
| 10 mg/kg | (126.52±16.11) | (1,882.69±968.48) | |
| 65 | 100.40±29.77 | 276.69±106.21 | 87.71 |
| 10 mg/kg | (101.24±35.15) | (1,536.07±263.57) | |
| 77 | 101.59±36.86 | 40.84±53.92 | 159.80 |
| 10 mg/kg | (101.24±35.15) | (1,536.07±263.57) | |
| 92 | 133.41±18.82 | 98.26±33.71 | 126.35 |
| 30 mg/kg | (133.86±28.86) | (1,366.89±291.81) | |

| | | | |
|---|---|---|---|
| Note: The data in parentheses indicate that the tumor volume of the embodiment corresponding to the Vehicle QD × 3w group (i.e., control group) at the corresponding time | | | |

### 6. Experimental conclusion:

The above data show that after 14 days of continuous oral administration, the compounds of embodiments of the present disclosure can significantly inhibit the growth of Ba/F3 KIF5B-RET nude mouse transplanted tumors.

### V. Pharmacodynamic experiment on subcutaneous xenograft tumor model of human myeloid thyroid cancer cell TT

### 1. Experimental purpose:

BALB/c nude mice were used as the test animals, and the xenograft tumor model of human myeloid thyroid cancer cell TT was used for *in vivo* pharmacodynamic experiments to evaluate the antitumor effects of the test compounds.

### 2. Experimental instruments and reagents:

### 2.1 Instruments:

CO₂ incubator (311, Thermo);
High-speed refrigerated centrifuge (Multifuge X3R, Thermo);
Automatic cell counter (Mini-006-0484, cellometer);
Biosafety cabinet (1300 SERIES A2, Thermo);
Electronic balance (JJ300Y, Changshu Shuangjie);
Vernier caliper (0-150 mm/0.01 mm, Mitutoyo, Japan).

### 2.2 Reagents:

F-12K (21127-022, Gibco);
Fetal bovine serum (FBS)(10099-141, Gibco);
Penicillin-streptomycin double antibodies (PS) (SV30010, Hyclone).

### 3. Test animals:

BALB/c nude mice, female, 6-8 weeks old, weighing 18-22 g, provided by Shanghai Lingchang Biotechnology Co., Ltd.

### 4. Test compounds:

The compounds of the embodiments of the present disclosure were self-made.

### 5. Experimental operation:

### 5.1 Cell culture:

TT cells were cultured *in vitro* in a monolayer under the following conditions: F-12K medium with 20% heat-inactivated fetal bovine serum and 1% penicillin-streptomycin double antibodies at 37°C and 5% CO₂. Digestion with trypsin-EDTA was performed to treat the passage twice a week. When cells were in an exponential growth period, cells were collected, counted and inoculated.

### 5.2 Tumor inoculation:

TT cells were collected when they were full in a logarithmic growth period, ensuring vitality over 90%. 0.2 mL of cell suspension containing about 1 × 10⁷ of TT cells (cells suspended in basic F-12K medium and added with 50% Matrigel) was inoculated subcutaneously into the right back tumor of each mouse when the mean volume of the tumor reached about 150-200 mm³ to start group administration.

### 5.3 Experimental grouping and administration:

1. The day of grouping was day 0 (D0). The interval of BID administration was 6-8 h/18-16 h. The first administration was given in the afternoon of day D0 and the last administration was given in the morning of day D21.
2. Administration volume: 10 µL/g according to the body weight of nude mice.

Animal husbandry: animals were kept in the experimental environment for 7 days after arrival to start the experiment. Animals were reared in SPF animal rooms in IVC (Independent Ventilation System) cages (5 animals per cage).

Animal grouping: animals were weighed before administration and the tumor volume was measured. Animals were randomly grouped according to tumor volume (randomized grouping design) with 5 animals per group.

Observation: animals were monitored daily for health status and death, routine checks include observation of the effects of drug on daily behavioral performance such as behavioral activity, food and water intake, changes in body weight (weight measurement twice a week or every other day), appearance signs or other abnormalities. The number of animal deaths and side effects within the group were recorded based on the number of animals in each group.

Experimental index: the diameter of tumor was measured by vernier caliper twice a week. The tumor volume was calculated by the formula: V = a×b²/2, a and b denote the long and short diameter of the tumor, respectively. The tumor inhibition efficacy of the compounds was evaluated by TGI (%). TGI (%) =(1-(TV_{Treatment/Dx} -TV_{Treatment/D1})/(TV_{Control/Dx}-TV_{Control/D1}))×100%. The tumor inhibition efficacy TGI=(1-(TW_{control}-TWₜᵣₑₐₜₘₑₙₜ)/TW_{control}×100%. TGI ≥58% indicates that the test substance can inhibit tumor growth effectively, and TGI ≥ 90% indicates that the test substance can inhibit tumor growth great effectively.

### 5.4 Experimental results:

**Table 15 Pharmacodynamic parameters**

| Grouping | Tumor volume /(mm³, mean±SEM) | | Tumor inhibition rate /TGI |
|---|---|---|---|
| | d0 | d22 | d22 |
| Vehicle | 206.10±22.69 | 1,276.23±161.33 | - |
| 18 30 mg/kg | 199.73±24.07 | 203.56±31.74 | 99.64% |
| 77 10 mg/kg | 205.23±16.57 | 144.14±8.95 | 129.77% |

### 7. Experimental conclusion:

The above data show that after 21 days of continuous oral administration, the compounds of embodiments of the present disclosure can significantly inhibit the growth of nude mouse transplanted tumors of thyroid cancer cells TT.

### VI. Effect of compounds of the present disclosure on hERG current

### 1. Experimental purpose:

The effect of compounds on hERG current of HEK293 cells was evaluated by manual patch clamp whole-cell recording technique.

### 2. Experimental instruments and reagents:

### 2.1 Reagents:

Terfenadine (Sigma-Aldrich, MKBX6318V);
HEK293-hERG cell line (human embryonic kidney cells stably expressing hERG channels), provided by Academy of Military Medical Sciences.

### 2.2 Instruments:

Patch clamp amplifier (700B, Axon Instruments, USA);
Digital-to-analog converter (DigitData 1440A, Axon Instruments, USA);
Inverted microscope (Ti-S, Nikon, Japan);
Micromanipulator (MP-225, Sutter Instrument, USA);
Microelectrode puller (P-97, Sutter Instrument, USA);
Microelectrode (Borosilicate glass, Sutter Instrument, USA);
Peristaltic pump (BT100-2J, Longerpump, China);
Vibration isolation tableand shielding net (TMC, USA);
Data acquisition software (Clampex 10.3, Axon Instruments, USA);
Data analysis software (Clampfit 10.3, Axon Instruments, USA);
Ultra-pure water system (Synergy UV, Merck)

### 3. Experimental methods:

### Cell culture:

Human embryonic kidney cells (HEK293) stably expressing hERG channels were cultured in DMEM medium (DMEM, HyClone) in a 37°C cell incubator containing 5% CO₂. About 10% fetal bovine serum (FBS, gibco) was added to the cell culture medium.

The composition of cell culture medium was shown in the following table:

| Reagents | Manufacturer | Batch No. |
|---|---|---|
| **DMEM** (high glucose) culture solution | HyClone | AE29431648 |
| Fetal bovine serum (FBS) | gibco | 2148169CP |
| Penicillin-streptomycin mixed solution | gibco | 2145460 |

Human embryonic kidney cells stably expressing hERG channels (HEK293-hERG) were used for the experiment. HEK293 cells were clamped with the Multi Clamp 700B manual patch clamp system to form a whole-cell voltage clamp pattern, and hERG current was induced with the corresponding voltage. Cells were administered embodiment 63 with concentrations of 0.1, 1, 3, 10, and 30 µmol/L or positive control terfenadine with a concentration of 10 µmol/L via a 6-channel perfusion system, respectively. The hERG channel tail current was recorded and the peak values of tail current at each administration concentration were obtained by using Clampex 10.3 software, and the data were analyzed by using Clampfit 10.3 software. The peak values of tail current recorded under the solvent control were taken as 100%, and the data of positive control and test samples were standardized. Curve fitting and IC₅₀ calculation were done in the software GraphPad Prism 5.00 using the standard Hill equation.

### 4. Experimental results:

In this experiment, the actual concentration was lower than the theoretical concentration due to the adsorption effect of the tube wall of the drug delivery system on the test samples, so the actual concentration was used for statistics. The actual concentrations of embodiment 63 formulated at 0.1, 1, 3, 10 and 30 µmol/Lwere 0.07, 0.58, 2.17, 6.14 and 28.61 µmol/L, respectively. Under the conditions of this experiment, the mean inhibition rates of embodiment 63 on hERG current in HEK293 cells were 3.24%±5.14%, 8.39%±3.51%, 12.69%±5.67%, 35.40%±2.15% and 58.84%±3.92%, and the IC₅₀ of embodiment 63 on hERG current was 16.89 µmol/L. Under the same experimental conditions, the positive control terfenadine at 10 µmol/L inhibited hERG current similarly with a mean inhibition rate of 97.45%±1.07%.

### VII. Study on crystal form

### 1. Experimental instruments:

### 1.1 Experimental instruments-some parameters of physical chemistry testing instruments

**Table 16**

| | | |
|---|---|---|
| XRPD | Instrument model | BRUKER D8 ADVANCE |
| | Diffracted ray | CuK (40 kV, 25 mA) |
| | Scan rate | 0.02°/S (2θ value) |
| | Scan range | 4° to 40° (2θ value) |
| DSC | Instrument model | NETZSCH DSC 214 polyma |
| | Purge gas | Nitrogen |
| | Purge speed | 40 mL/min |
| | Heating rate | 10°C /min |
| | Temperature range | 25 to 300°C |
| | Plate type | Aluminium plate |
| TGA | Instrument model | NETZSCH TG 209 Tarsus |
| | Purge gas | Nitrogen |
| | Purge speed | 40 mL/min |
| | Heating rate | 10°C /min |
| | Temperature range | 35 to 350°C |
| | Plate type | Al₂O₃ |
| High performance liquid chromatography | Agilent | 1260 |
| | Pump | Agilent G 1311B |
| | Sample Injector | G1329B |
| | Column oven | G1316A |
| | Detector | G1315D |
| High performance liquid chromatography | Thermo | U3000 |
| | Pump | LPG-3400SDN |
| | Sample Injector | WPS-3000TSL |
| | Column oven | TCC-3000SD |
| | Detector | DAD-3000 |

### 2. Preparation of crystal forms

### 2.1 Preparation of crystal forms of a free base of the compound of embodiment 63

### 2.1.1 Preparation of a crystal form I of the free base

10 mg of compound (amorphous) was weighed, 10 mL of methanol was added thereto, then the mixture was heated to 50°C, dissolved clarification, filtered while hot, put into refrigerator (2 to 8°C) overnight, observed the precipitation, centrifuged rapidly. The supernatant was removed, and the solid was dried in vacuum at 40°C to obtain crystal form I, which has an XRPD pattern as shown in FIG. 1, a DSC pattern as shown in FIG. 2 and a TGA pattern as shown in FIG. 3 through detection and analysis.

### 2.1.2 Preparation of a crystal form II of the free base

15 mg of compound (crystal form I) was weighed and added into a 2 mL glass bottle, 200 µL of 2-methyltetrahydrofuran was added thereto to obtain a suspension; the suspension was placed on a magnetic stirrer to slurry at 50°C for 2 to 3 days, then centrifuged, the supernatant was removed, and the solid was further dried in a vacuum drying oven overnight (vavuum drying under reduced pressure at 50°C) to obtain the crystal form II, which has an XRPD pattern as shown in FIG. 4, a DSC pattern as shown in FIG. 5 and a TGA pattern as shown in FIG. 6 through detection and analysis.

### 2.1.3 Preparation of a crystal form III of the free base

15 mg of compound (crystal form I) was weighed and added into a 2 mL glass bottle, 200 µL of dibutanone was added thereto to obtain a suspension; the suspension was placed on a magnetic stirrer to slurry at 50°C for 2 to 3 days, then centrifuged, the supernatant was removed, and the solid was further dried in a vacuum drying oven overnight (vavuum drying under reduced pressure at 50°C) to obtain the crystal form III, which has an XRPD pattern as shown in FIG. 7, a DSC pattern as shown in FIG. 8 and a TGA pattern as shown in FIG. 9 through detection and analysis.

### 2.1.4 Preparation of a crystal form IV of the free base

20 mg of compound (crystal form I) was weighed, 3.5 mL of 88% acetone was added thereto, then the mixture was heated to 50°C, dissolved clarification, filtered while hot. The filtrate was dried in vacuum at 50°C and evaporated rapidly to obtain the crystal IV, which has an XRPD pattern as shown in FIG. 10, a DSC pattern as shown in FIG. 11 and a TGA pattern as shown in FIG. 12 through detection and analysis.

### 2.1.5 Preparation of a crystal form V of the free base

10 mg of compound (crystal form I) was weighed, 500 µL of tetrahydrofuran was added thereto, then the mixture was heated to 50°C, then dissolved. 700 µL of cyclohexane was added thereto, a solid was precipitated, and the mixture was stirred overnight, then centrifuged rapidly. The supernatant was removed, the solid was dried in vacuum at 50°C to obtain the crystal form V, which has an XRPD pattern as shown in FIG. 13, a DSC pattern as shown in FIG. 14 and a TGA pattern as shown in FIG. 15 through detection and analysis.

### 3. Experiment on solid stability of the free base of the compound

### 3.1 Stability experiment of the compound of embodiment 63

### 3.1.1 Experimental purpose:

To investigate the physical and chemical stability of the compound under light 5000 lx, high temperature 60°C, high humidity 92.5%RH, high temperature and high humidity 50°C/75%RH conditions to provide a basis for the storage of the compound.

### 3.1.2 Instrument and liquid chromatographic analysis conditions

Chromatographic conditions:

**Table 17**

| Instrument | Thermo Ultimate 3000 | |
|---|---|---|
| Mobile phase A | 25 mM Phosphate buffer (NH₄H₂PO₄, pH2.0) | |
| Mobile phase B | MeOH | |
| Flow rate | 1.0 mL/min | |
| Injection volume | 5.0 µL | |
| Chromatographic column | Waters x-bridge (150*4.6 mm, 3.5 µm) | |
| Column temperature | 35°C | |
| Detection wavelength | 235, 238, 326 nm | |

| Elution gradient (min) | A% | B% |
|---|---|---|
| 0.00 | 65 | 35 |
| 10.00 | 20 | 80 |
| 12.00 | 20 | 80 |
| 12.01 | 65 | 35 |
| 15.00 | 65 | 35 |

### 3.1.3 Experimental scheme:

1 mg of crystal form I was taken and observed for 5, 10 days under the conditions of light 5000 lx, high temperature 60°C, high humidity 92.5%RH, high temperature and high humidity 50°C/75%RH, and the content was determined by HPLC with external standard method, and the changes of the related substances were calculated by the area normalization method for chromatographic peak.

### 3.1.4 Experimental results:

The results of physical and chemical stability of the crystal form I of the free base were shown in Table 18:

**Table 18**

| Solid stability of the crystal form I of the free base (14 days) (Increase of impurities) | | |
|---|---|---|
| Conditions | Increase of total impurities% | Maximum increase of single impurity% |
| High temperature/60°C | <0.02 | 0.06 |
| High humidity/92.5%RH | <0.02 | <0.02 |
| High temperature and high humidity/50°C/75%RH | <0.02 | 0.11 |
| Light/5000 lx | <0.02 | 0.18 |

### 3.1.5 Experimental conclusion:

The above data show that the crystal form I of the free base has good stability under the conditions of high temperature, high humidity, high temperature and high humidity and light.

### 4. Hygroscopicity experiment

### 4.1 Hygroscopicity experiment of the compound of embodiment 63

### 4.1.1 Experimental purpose:

To investigate the hygroscopicity of the crystal form I of the free base of the compound under different relative humidity conditions to provide a basis for the storage of the compound.

### 4.1.2 Instrument and parameters:

| Instrument model | SMS Intrinsic |
|---|---|
| Experimental temperature | 25°C |
| Drying time | 0%RH 120 min |
| Balance dm/dt | 0.02%/min (Min 10 min, Max 180 min) |
| RH(%) step length | 10% |
| Measurement gradient | 0-95-0% |
| Cycle | 2 |

### 4.1.3 Experimental scheme:

The crystal form I of the free base of the compound was placed in the saturated water vapor at the same relative humidity to make the compound reach dynamic equilibrium with the water vapor, and the percentage of hygroscopic weight gain of the compound was calculated after equilibrium.

### 4.1.4 Experimental results:

The hygroscopic weight gain of the crystal form I of the free base was about 0.43% at RH80%, with slightly hygroscopicity; after two cycles of moisture absorption and desorption at 0-95% RH, the XRPD pattern of the crystal form I of the free base has not changed, i.e., the crystal form has not transformed, and the specific isotherms were shown in FIG. 16.

### 4.1.5 Experimental conclusion:

The crystal form I of the free base is slightly hygroscopic and stable under high humidity conditions.

### 5. Solubility experiment in different medium

### 5.1 Solubility experiment of the compound of embodiment 63

### 5.1.1 Experimental purpose:

To compare the solubility of the crystal form I of the free base in pH 1-8 USP buffer, artificial simulated gastric fluid (FaSSGF), fasting artificial simulated intestinal fluid (FaSSIF), non-fasting artificial simulated intestinal fluid (FeSSIF) and pure water and other medium to provide a basis for the assessment of druggablitity.

### 5.1.2 Experimental scheme:

### About 1 mg of the crystal form I of the free base was mixed and suspended in different mediums for 4 hours. The thermodynamic solubility of the compound at 37°C was determined by HPLC, with external standard method.

### 5.1.3 Experimental results:

The results of solubility experiment of the crystal form I of the free base were shown in Table 19:

**Table 19**

| No. | Medium | Solubility of crystal form I (mg/mL) |
|---|---|---|
| 1 | pH 1 | >2.00 |
| 2 | pH 2 | >2.00 |
| 3 | pH 3 | 0.71 |
| 4 | pH 4 | 0.37 |
| 5 | pH 5 | 0.02 |
| 6 | pH 6 | 0.001 |
| 7 | FaSSGF | >2.00 |
| 8 | FaSSIF | 0.001 |
| 9 | FeSSIF | 0.09 |

### 6. Thermodynamically stable crystal form confirmation experiment

### 6.1 Competitive slurry experiment of the compound of embodiment 63.

### 6.1.1 Experimental purpose:

Thermodynamically stable crystal form was confirmed by competitive slurry experiments.

### 6.1.2 Experimental scheme:

The crystal forms I to V were weighed respectively and mixed pairwise in equal amounts, the ethyl acetate was added thereto, the mixture was stirred at 50°C for 3 days, then centrifuged rapidly. The supernatant was removed, and the solid precipitate was dried in vacuum in an oven at 40°C for XRPD characterization.

### 6.1.3 Experimental results:

**Table 20 Results of polycrystal forms competition experiments**

| Solvent | Crystal form I+II | Crystal form I+III | Crystal form I+ IV | Crystal form I+V |
|---|---|---|---|---|
| Ethyl | I | I | I | I |

### 7. Polycrystal forms screening experiment

### 7.1 Polycrystal forms screening of embodiment 63:

### 7.1.1 Experimental purpose:

Polycrystal forms screening experiments were conducted by various methods to obtain more crystal forms.

### 7.1.2 Experimental scheme:

### 7.1.2.1 Suspension slurrying method:

15 mg of the compound was weighed and added into 2 mL glass bottles, respectively, and 200 µL of solvent was added thereto to obtain a suspension; the suspension was placed on a magnetic stirrer to slurry at 50°C for 2 to 3 days, then centrifuged, the supernatant was removed, the solid was further dried in a vacuum drying oven overnight (vavuum drying under reduced vacuum at 50°C). XRPD was detected, and if there was any change, DSC was then detected. The screening and characterization results were shown in Table 21.

**Table 21 Results of polycrystal form screening by free base suspension slurrying method**

| No. | Solvent | Free base |
|---|---|---|
| - | initial crystal form | crystal form I |
| 1 | methanol | crystal form I |
| 2 | ethanol | crystal form II |
| 3 | isopropanol | crystal form I |
| 4 | acetonitrile | crystal form I |
| 5 | acetone | crystal form III |
| 6 | 2-methyl-tetrahydrofuran | crystal form II |
| 7 | ethyl acetate | crystal form I |
| 8 | toluene | crystal form I |
| 9 | 2-butanone | crystal form III |
| 10 | isopropyl acetate | crystal form I |

### 7.1.2.2 Rapid cooling method:

10 mg of the compound was weighed, corresponding solvent was added thereto, then the mixture was heated to 50°C, if not dissolved clear, an additional solvent was added until dissolved clear, the mixture was filtered while hot, cooled to -25°C. Whether there was precipitation was observed, if there was precipitation, the filtrate was centrifuged rapidly, the supernatant was removed, the solid was dried in vacuum at 40°C overnight, then XRPD of the solid was determined and compared with initial XRPD,. The results were shown in Table 22.

**Table 22. Results of polycrystal form screening experiment by free base rapid cooling method**

| No. | Solvent | Free base |
|---|---|---|
| - | initial crystal form | crystal form I |
| 1 | dichloromethane | crystal form I |
| 2 | 88% acetone | crystal form III |
| 3 | acetone | crystal form III |
| 4 | methanol | crystal form I |

### 7.1.2.3 Rapid evaporation method

10 mg of the compound was weighed, corresponding solvent was added thereto, then the mixture was heated to 50°C, if not dissolved clear, an additional solvent was added until dissolved clear, the mixture was filtered while hot, then placed in a vacuum drying oven at 50°C overnight. After rapid evaporation, XRPD of the solid was determined and compared with initial XRPD. The results were shown in Table 23.

**Table 23 Results of polycrystal form screening experiment by free base rapid evaporation method**

| No. | Solvent | Free base |
|---|---|---|
| - | initial crystal form | crystal form I |
| 1 | dichloromethane | crystal form I |
| 2 | 88% acetone | crystal form IV |
| 3 | acetone | crystal form I |
| 4 | methanol | crystal form I |
| 5 | 2-methyl-tetrahydrofuran | crystal form I |
| 6 | chloroform | crystal form I |

### 7.1.2.4 Anti-solvent crystallization method:

An appropriate amount of compound was weighed, respectively, a certain volume of a good solvent was added thereto, dissolved at high temperature. The mixture was filtered, divided into equal parts of 1 mL each, and a poor solvent was added thereto. If there was precipitation, the mixture was centrifuged rapidly after stirred overnight, the supernatant was removed, and the solid was dried in vacuum at 50°C overnight. XRPD of the solid was determined and compared with initial XRPD. Four polycrystal forms were found, and the results were shown in Table 24.

**Table 24 Results of polycrystal form screening experiment by free base anti-solvent method**

| Solvent | | Good solvent | |
|---|---|---|---|
| | | Tetrahydrofuran | Dichloromethane |
| Anti-solvent | ethyl acetate | / | crystal form I |
| | ethanol | crystal form I | crystal form I |
| | acetonitrile | crystal form I | crystal form I |
| | heptane | crystal form II | crystal form I |
| | isopropyl ether | crystal form II | crystal form I |
| | methyl *tert-*butyl ether | crystal form I | crystal form I |
| | isopropyl acetate | / | crystal form I |
| | isopropanol | / | crystal form V |
| | toluene | crystal form I | crystal form I |
| | 2-butanone | / | crystal form III |
| | water | crystal form I | / |
| | cyclohexane | crystal form V | crystal form V |

### 8. Detection of moisture content in crystal form

### 8.1 Elemental analysis

C, H and N contents in the compound were determined by Elementar Vario EL III elemental analyzer.

**Table 25 Elemental analysis data of samples**

| **Element** | **C%** | **H%** | **N%** | **Note** |
|---|---|---|---|---|
| Calculated value | 67.27 | 5.46 | 18.31 | Calculated value based on anhydrous C₃₀H₂₉N₇O₃ |
| Crystal form I | 67.19 | 5.61 | 18.39 | |
| Crystal form I | 67.21 | 5.59 | 18.45 | |
| Mean value | 67.20 | 5.60 | 18.42 | |

The results of moisture determination further show that the samples do not contain crystal water.

## Claims

1. A crystal form of a compound represented by general formula (I), 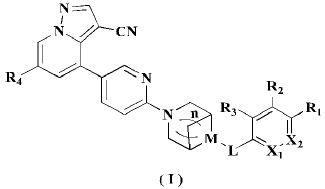 wherein:
L is selected from -CH₂- or -NHC(O)-;
M is selected from CR or N;
R is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
X₁ is selected from CRₐ or N;
X₂ is selected from CR_{b} or N;
Rₐ and R_{b} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
R₁, R₂ and R₃ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl;
R₄ is selected from C₂₋₆ alkenyl, C₂₋₆ alkynyl, 3-8-membered heterocyclyl, -O(CH₂)ₘR₅, - (CH₂)ₘR₅, -NR₆(CH₂)ₘR₅, -NR₆C(O)R₅, -CH=CH-(CH₂)ₘR₅, -O(CH₂)ₘS(O)R₅, - O(CH₂)ₘS(O)₂R₅ or -O(CH₂)ₘS(O)(=NH)R₅, and the C₂₋₆ alkenyl, the C₂₋₆ alkynyl and the 3-8-membered heterocyclyl are optionally further substituted by one or more substituents selected from deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-12-membered heterocyclyl, C₆₋₁₀ aryl and 5-12-membered heteroaryl;
R₅ and R₆ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl, and the C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl are optionally further substituted by one or more substituents selected from deuterium, halogen, amino, nitro, hydroxyl, cyano, oxo, thio, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl, 5-12-membered heteroaryl, =NH, -(CH₂)ₜR₇, -(CH₂)ₜOR₇ and -(CH₂)ₜC(O)NR₇R₈;
R₇ and R₈ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl or 5-12-membered heteroaryl, and the C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl are optionally further substituted by one or more substituents selected from deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-8-membered heterocyclyl, C₆₋₁₀ aryl and 5-12-membered heteroaryl;
n is 0, 1, 2 or 3;
m is 0, 1, 2 or 3; and
t is 0, 1, 2 or 3.

2. The crystal form of the compound as claimed in claim 1, wherein, M is selected from CR or N;
R is selected from hydrogen, amino or C₁₋₃ alkyl, preferably hydrogen, amino or methyl;
X₁ is selected from CRₐ or N;
X₂ is selected from CR_{b} or N;
Rₐ and R_{b} are each independently selected from hydrogen, deuterium, halogen, C₁₋₃ alkyl or C₁₋₃ alkoxy, preferably hydrogen, fluorine, chlorine, methyl or methoxy;
R₁, R₂ and R₃ are each independently selected from hydrogen, deuterium, halogen, C₁₋₃ alkyl or C₁₋₃ alkoxy, preferably hydrogen, fluorine, chlorine, methyl or methoxy.

3. The crystal form of the compound as claimed in any one of claims 1-2, wherein, R₄ is selected from C₂₋₃ alkynyl, C₂₋₃ alkenyl, 3-8-membered heterocyclyl containing a nitrogen or oxygen atom, -O(CH₂)ₘR₅, -(CH₂)ₘR₅, -NR₆(CH₂)ₘR₅, -NHC(O)R₅, -CH=CH-(CH₂)ₘR₅, - O(CH₂)ₘS(O)R₅, -O(CH₂)ₘS(O)₂R₅ or -O(CH₂)ₘS(O)(=NH)R₅, and the 3-8-membered heterocyclyl containing the nitrogen or oxygen atom is optionally further substituted by one or more substituents selected from deuterium, fluorine, chlorine, bromine, amino, hydroxyl, cyano, C₁₋₃ alkyl, C₁₋₃ hydroxyalkyl or C₁₋₃ alkoxy;
preferably, R₄ is selected from vinyl, ethynyl, oxetanyl, azetidinyl, pyrrolidinyl, piperidinyl, 2-azaspiro[3.3]heptanyl, -OCH₂R₅, -O(CH₂)₂R₅, -(CH₂)₂R₅, -NR₆(CH₂)ₘR₅, - NHC(O)R₅, -CH=CH-(CH₂)ₘR₅, -O(CH₂)ₘS(O)R₅, -O(CH₂)ₘS(O)₂R₅ or - O(CH₂)ₘS(O)(=NH)R₅, and the vinyl, ethynyl, oxetanyl, azetidinyl, pyrrolidinyl, piperidinyl and 2-azaspiro[3.3]heptanyl are optionally further substituted by one or more substituents selected from deuterium, hydroxyl, cyano, methyl, hydroxyethyl, 2-hydroxyisopropyl, 2-aminoisopropyl;
R₅ is selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₆ cycloalkyl or 3-8-membered heterocyclyl, and the C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₆ cycloalkyl and 3-8-membered heterocyclyl are optionally further substituted by one or more substituents selected from deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl, C₂₋₃ alkenyl, C₂₋₃ alkynyl, C₃₋₆ cycloalkyl, 3-8-membered heterocyclyl, -CH₂R₇, -CH₂OR₇ and -C(O)NR₇R₈;
preferably, R₅ is selected from methyl, ethyl, isopropyl, cyclopropyl, cyclobutyl, cyclopentyl, ethynyl, oxetanyl, thietanyl, azetidinyl, tetrahydropyranyl, bicyclo[1.1.1]pentanyl or tetrahydro-2H-thiopyran, which are optionally further substituted by one or more substituents selected from deuterium, hydroxyl, amino, cyano, fluorine, chlorine, bromine, methoxy, ethynyl, cyclopropyl, hydroxyethyl, oxo, =NH, -CH₂OCH₃, -C(O)NH₂ and - C(O)NHCH₃;
R₆ is selected from hydrogen, deuterium, halogen, amino, hydroxyl or C₁₋₃ alkyl, preferably hydrogen, deuterium or methyl;
R₇ and R₈ are each independently selected from hydrogen, deuterium, halogen, amino, hydroxyl or C₁₋₃ alkyl, preferably hydrogen, deuterium or methyl;
n is 0, 1, 2 or 3; and
m is 0, 1, 2 or 3.

4. The crystal form of the compound as claimed in any one of claims 1-3, wherein, the compound is further represented by general formula (II) 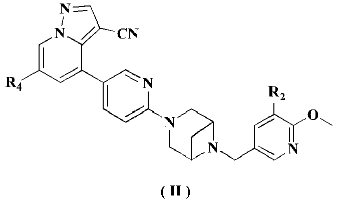
R₂ is selected from hydrogen, fluorine or chlorine;
R₄ is selected from -O(CH₂)ₘR₅, -(CH₂)ₘR₅ or -NR₆(CH₂)ₘR₅,
R₅ is selected from C₂₋₃ alkynyl, optionally further substituted by one or more substituents selected from deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₁₋₃ hydroxyalkyl or C₃₋₆ cycloalkyl;
R₆ is selected from hydrogen, deuterium, halogen, amino, hydroxyl or C₁₋₃ alkyl, preferably hydrogen, deuterium or methyl;
m is 0, 1, 2 or 3.

5. The crystal form of the compound as claimed in any one of claims 1-4, wherein, the compound has a specific structure as follows:

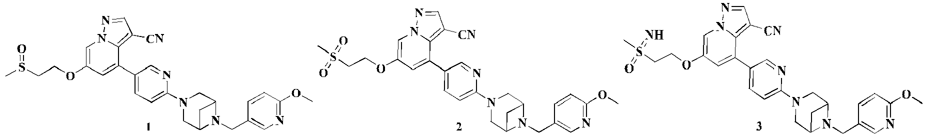

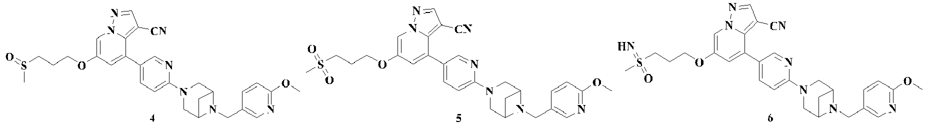

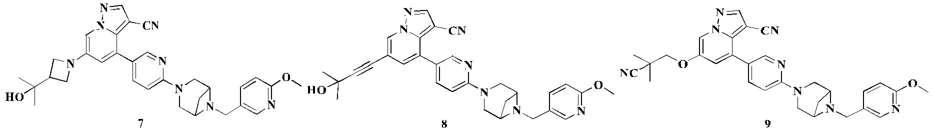

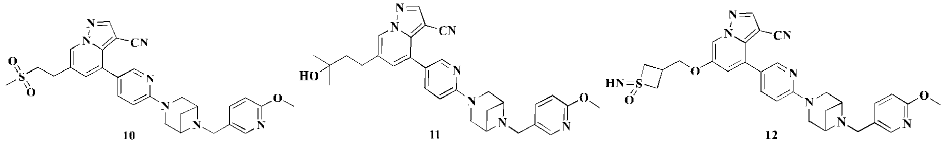

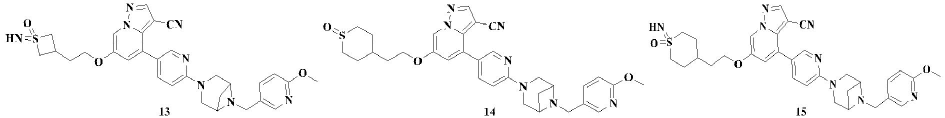

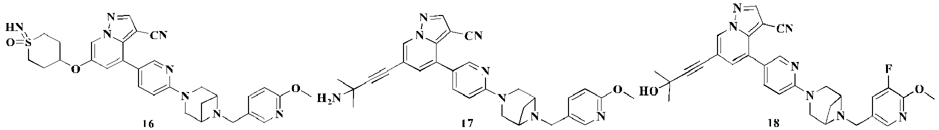

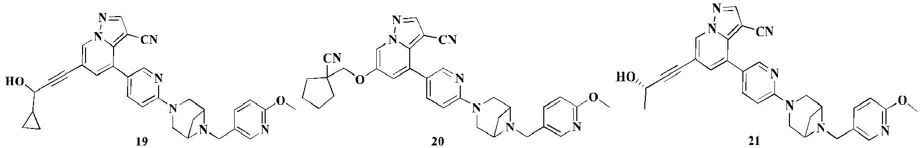

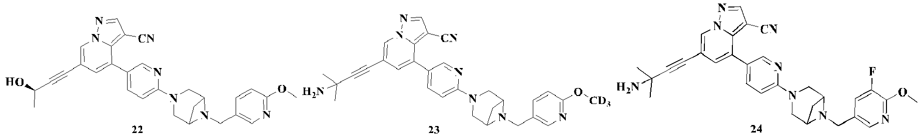

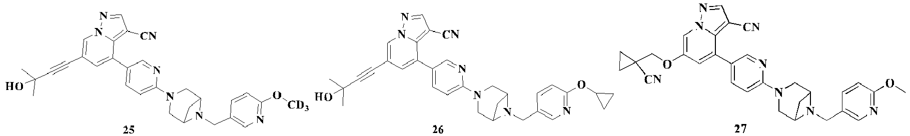

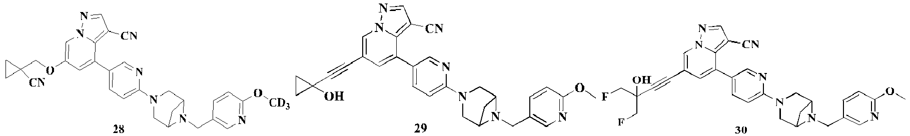

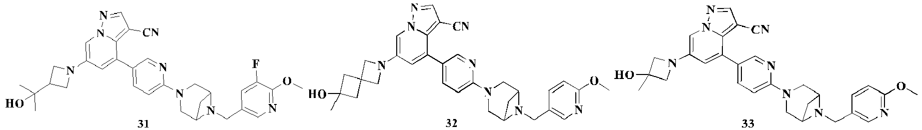

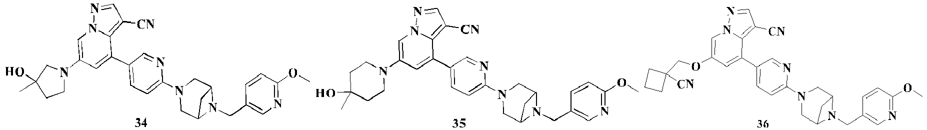

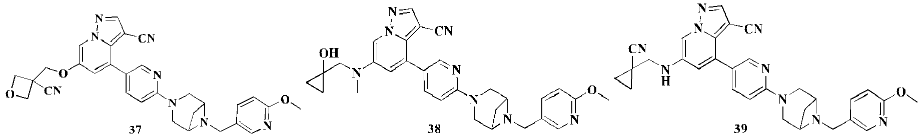

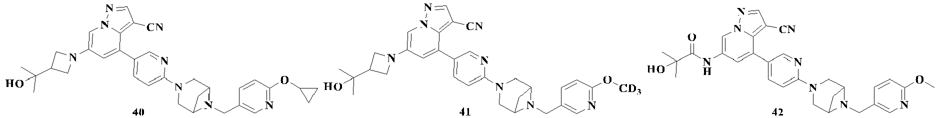

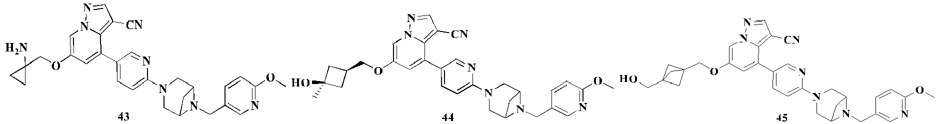

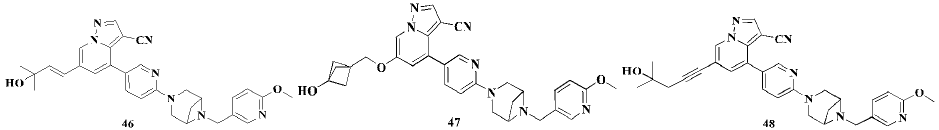

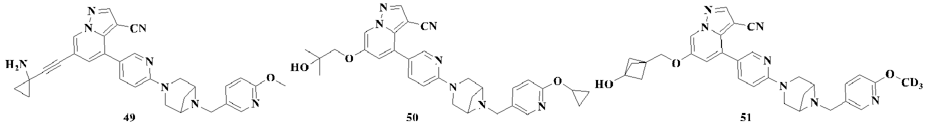

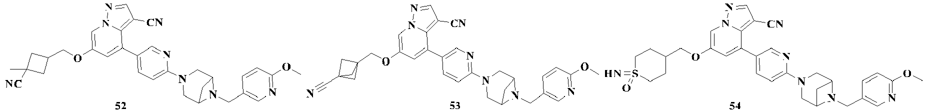

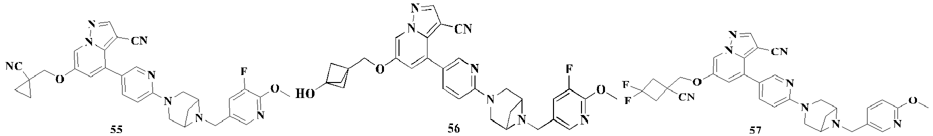

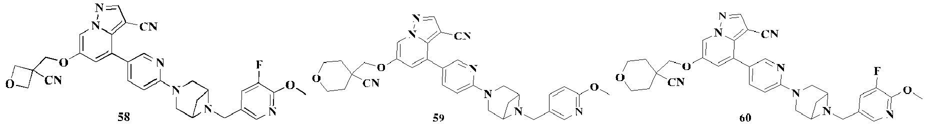

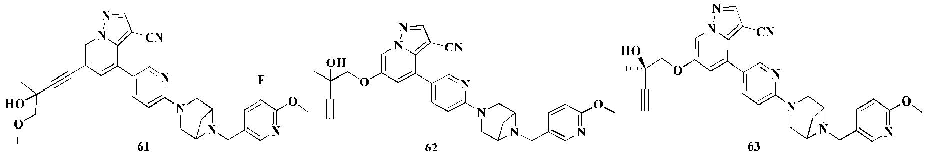

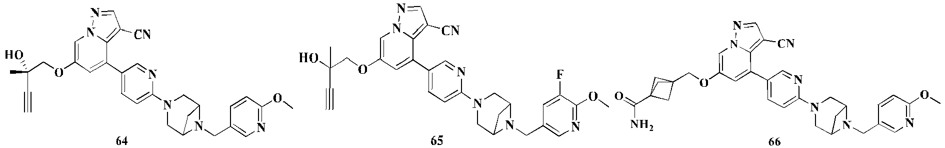

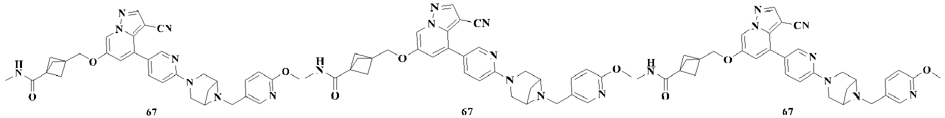

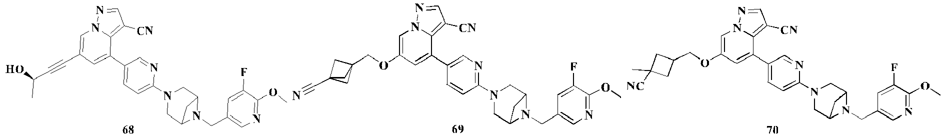

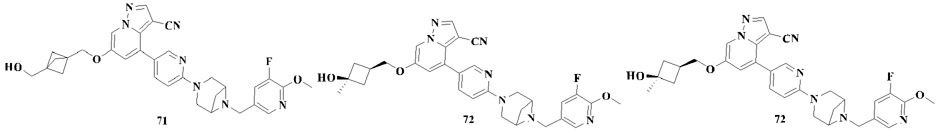

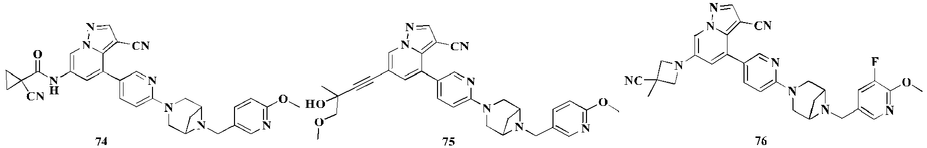

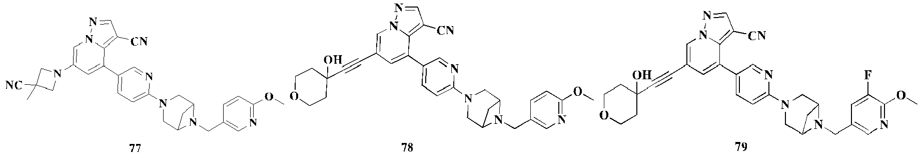

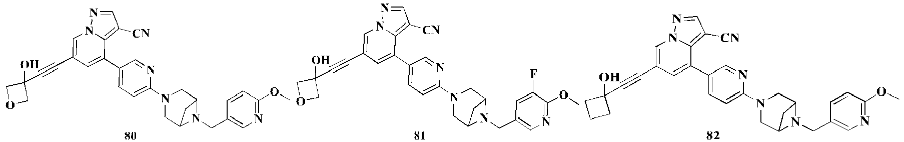

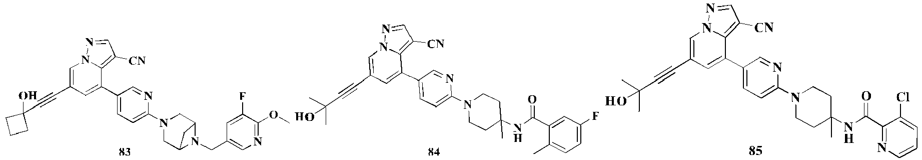

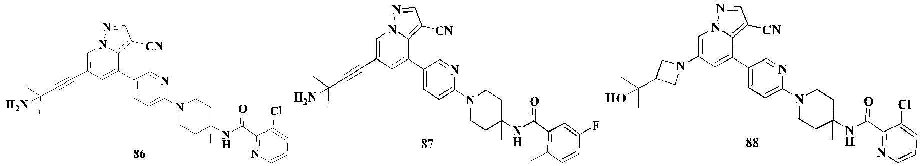

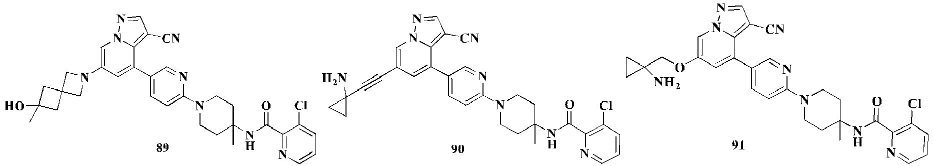

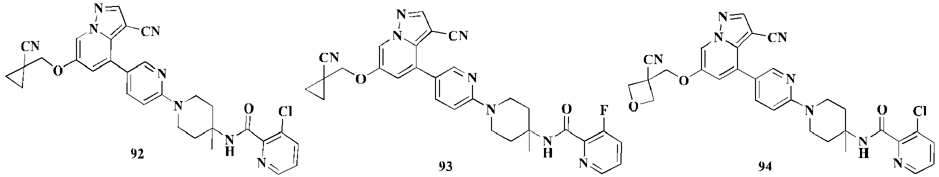

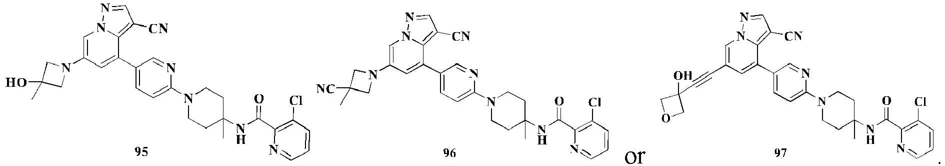

6. The crystal form of the compound as claimed in any one of claims 1-5, wherein the crystal form is a crystal form I, a crystal form II, a crystal form III, a crystal form IV or a crystal form V of 6-(((*R*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile, wherein:
the crystal form I has an X-ray powder diffraction pattern having a diffraction peak at 2θ angle of 5.0±0.2°; or having a diffraction peak at 9.6±0.2°; or having a diffraction peak at 15.0±0.2°; or having a diffraction peak at 17.2±0.2°; or having a diffraction peak at 22.2±0.2°; or having a diffraction peak at 19.4±0.2°; or having a diffraction peak at 30.2±0.2°; or having a diffraction peak at 8.2±0.2°; or having a diffraction peak at 25.1±0.2°; or having a diffraction peak at 26.9±0.2°; preferably comprising any 2-5, or 3-5, or 3-6, or 3-8, or 5-8, or 6-8 of the above diffraction peaks; more preferably comprising any 6, 7, or 8 of the diffraction peaks; or
the crystal form I has an X-ray powder diffraction pattern having a diffraction peak at 2θ angle of 5.0±0.2°; or having a diffraction peak at 8.2±0.2°; or having a diffraction peak at 9.6±0.2°; or having a diffraction peak at 12.9±0.2°; or having a diffraction peak at 15.0±0.2°; or having a diffraction peak at 17.2±0.2°; or having a diffraction peak at 15.4±0.2°; or having a diffraction peak at 16.6±0.2°; or having a diffraction peak at 17.7±0.2°; or having a diffraction peak at 18.5±0.2°; preferably comprising any 2-5, or 3-5, or 3-6, or 3-8, or 5-8, or 6-8 of the above diffraction peaks; more preferably comprising any 6, 7, or 8 of the diffraction peaks;
the crystal form II has an X-ray powder diffraction pattern having a diffraction peak at 2θ angles of 4.8±0.2°; or having a diffraction peak at 17.7±0.2°; or having a diffraction peak at 16.6±0.2°; or having a diffraction peak at 9.4±0.2°; or having a diffraction peak at 18.4±0.2°; or having a diffraction peak at 19.1±0.2°; or having a diffraction peak at 17.0±0.2°; or having a diffraction peak at 18.6±0.2°; or having a diffraction peak at 4.5±0.2°; or having a diffraction peak at 15.4±0.2°; preferably comprising any 2-5, or 3-5, or 3-6, or 3-8, or 5-8, or 6-8 of the above diffraction peaks; more preferably comprising any 6, 7, or 8 of the diffraction peaks;
the crystal form III has an X-ray powder diffraction pattern having a diffraction peak at 2θ angle of 4.6±0.2°; or having a diffraction peak at 15.2±0.2°; or having a diffraction peak at 9.9±0.2°; or having a diffraction peak at 16.7±0.2°; or having a diffraction peak at 18.2±0.2°; or having a diffraction peak at 17.9±0.2°; or having a diffraction peak at 25.5±0.2°; or having a diffraction peak at 15.0±0.2°; or having a diffraction peak at 19.5±0.2°; or having a diffraction peak at 23.6±0.2°; preferably comprising any 2-5, or 3-5, or 3-6, or 3-8, or 5-8, or 6-8 of the above diffraction peaks; more preferably comprising any 6, 7, or 8 of the diffraction peaks;
the crystal form IV has an X-ray powder diffraction pattern having a diffraction peak at 2θ angle of 5.0±0.2°; or having a diffraction peak at 4.8±0.2°; or having a diffraction peak at 15.0±0.2°; or having a diffraction peak at 19.1±0.2°; or having a diffraction peak at 14.3±0.2°; or having a diffraction peak at 10.0±0.2°; or having a diffraction peak at 23.9±0.2°; or having a diffraction peak at 25.1±0.2°; or having a diffraction peak at 30.3±0.2°; or having a diffraction peak at 9.6±0.2°; preferably comprising any 2-5, or 3-5, or 3-6, or 3-8, or 5-8, or 6-8 of the above diffraction peaks; more preferably comprising any 6, 7, or 8 of the diffraction peaks;
the crystal form V has an X-ray powder diffraction pattern having a diffraction peak at 2θ angle of 4.7±0.2°; or having a diffraction peak at 18.2±0.2°; or having a diffraction peak at 10.1±0.2°; or having a diffraction peak at 18.8±0.2°; or having a diffraction peak at 15.6±0.2°; or having a diffraction peak at 17.0±0.2°; or having a diffraction peak at 21.8±0.2°; or having a diffraction peak at 14.7±0.2°; or having a diffraction peak at 19.3±0.2°; or having a diffraction peak at 25.8±0.2°; preferably comprising any 2-5, or 3-5, or 3-6, or 3-8, or 5-8, or 6-8 of the above diffraction peaks; more preferably comprising any 6, 7, or 8 of the diffraction peaks.

7. The crystal form of the compound as claimed in claim 6, wherein, the X-ray powder diffraction pattern of the crystal form I comprises at least one or more diffraction peaks at 2θ angles of 5.0±0.2°, 9.6±0.2°, 15.0±0.2°, preferably comprises two of the above diffraction peaks, more preferably comprises three of the diffraction peaks; optionally, further comprises at least one diffraction peak at 2θ angle of 17.2±0.2°, 22.2±0.2°, 19.4±0.2°, 30.2±0.2° or 8.2±0.2°, preferably comprises 2, 3, 4 or 5 of the above diffraction peaks;
or, comprises diffraction peaks at 2θ angles of 5.0±0.2°, 9.6±0.2°, 15.0±0.2°, 17.2±0.2°, 22.2±0.2° and 19.4±0.2°;
or, comprises diffraction peaks at 2θ angles of 5.0±0.2°, 9.6±0.2°, 15.0±0.2°, 17.2±0.2°, 22.2±0.2° and 8.2±0.2°;
the X-ray powder diffraction pattern of the crystal form I comprises at least one or more diffraction peaks at 2θ angles of 5.0±0.2°, 8.2±0.2°, 9.6±0.2°, preferably comprises two of the above diffraction peaks, more preferably comprises three of the diffraction peaks; optionally, further comprises at least one diffraction peak at 2θ angle of 12.9±0.2°, 15.0±0.2°, 17.2±0.2°, 15.4±0.2° or 16.6±0.2°, preferably comprises 2, 3, 4 or 5 of the above diffraction peaks;
or, comprises diffraction peaks at 2θ angles of 5.0±0.2°, 8.2±0.2°, 9.6±0.2°, 12.9±0.2°, 15.0±0.2° and 17.2±0.2°;
or, comprises diffraction peaks at 2θ angles of 5.0±0.2°, 9.6±0.2°, 12.9±0.2°, 15.0±0.2°, 17.2±0.2° and 15.4±0.2°;
the X-ray powder diffraction pattern of the crystal form II comprises at least one or more diffraction peaks at 2θ angles of 4.8±0.2°, 17.7±0.2°, 16.6±0.2°, preferably comprises two of the above diffraction peaks, more preferably comprises three of the diffraction peaks; optionally, further comprises at least one diffraction peak at 2θ angle of 9.4±0.2°, 18.4±0.2°, 19.1±0.2°, 17.0±0.2° or 18.6±0.2°, preferably comprises 2, 3, 4 or 5 of the above diffraction peaks;
or, comprises diffraction peaks at 2θ angles of 4.8±0.2°, 17.7±0.2°, 16.6±0.2°, 9.4±0.2°, 18.4±0.2° and 19.1±0.2°;
or, comprises diffraction peaks at 2θ angles of 4.8±0.2°, 17.7±0.2°, 16.6±0.2°, 9.4±0.2°, 18.4±0.2° and 17.0±0.2°;
or, comprises diffraction peaks at 2θ angles of 4.8±0.2°, 17.7±0.2°, 16.6±0.2°, 9.4±0.2°, 18.4±0.2° and 18.6±0.2°;
or, comprises diffraction peaks at 2θ angles of 4.8±0.2°, 17.7±0.2°, 9.4±0.2°, 18.4±0.2°, 19.1±0.2° and 17.0±0.2°;
or, comprises diffraction peaks at 2θ angles of 4.8±0.2°, 16.6±0.2°, 9.4±0.2°, 18.4±0.2°, 19.1±0.2° and 17.0±0.2°;
the X-ray powder diffraction pattern of the crystal form III comprises at least one or more diffraction peaks at 2θ angles of 4.6±0.2°, 15.2±0.2° or 9.9±0.2°; preferably comprises two of the above diffraction peaks, more preferably comprises three of the diffraction peaks; optionally, further also comprises at least one diffraction peak at 2θ angle of 16.7±0.2°, 18.2±0.2°, 17.9±0.2°, 25.5±0.2° or 15.0±0.2°, preferably comprises 2, 3, 4 or 5 of the above diffraction peaks;
or, comprises diffraction peaks at 2θ angles of 4.6±0.2°, 15.2±0.2°, 9.9±0.2°, 16.7±0.2°, 18.2±0.2° and 17.9±9.2°;
or, comprises diffraction peaks at 2θ angles of 4.6±0.2°, 15.2±0.2°, 9.9±0.2°, 16.7±0.2°, 18.2±0.2° and 25.5±0.2°;
or, comprises diffraction peaks at 2θ angles of 4.6±0.2°, 15.2±0.2°, 9.9±0.2°, 16.7±0.2°, 18.2±0.2° and 15.0±0.2°;
or, comprises diffraction peaks at 2θ angles of 4.6±0.2°, 15.2±0.2°, 9.9±0.2°, 16.7±0.2°, 18.2±0.2° and 25.5±0.2°;
or, comprises diffraction peaks at 2θ angles of 4.6±0.2°, 15.2±0.2°, 9.9±0.2°, 18.2±0.2° 17.9±0.2° and 15.0±0.2°;
the X-ray powder diffraction pattern of the crystal form IV comprises at least one or more diffraction peaks at 2θ angles of 5.0±0.2°, 4.8±0.2° or 15.0±0.2°; preferably comprises two of the above diffraction peaks, more preferably comprises three of the diffraction peaks; optionally, further also comprises at least one diffraction peak at 2θ angle of 19.1±0.2°, 14.3±0.2°, 10.0±0.2°, 23.9±0.2° or 25.1±0.2°, preferably comprises 2, 3, 4 or 5 of the above diffraction peaks;
or, comprises diffraction peaks at 2θ angles of 5.0±0.2°, 4.8±0.2°, 15.0±0.2°, 19.1±0.2°, 14.3±0.2° and 10.0±0.2°;
or, comprises diffraction peaks at 2θ angles of 5.0±0.2°, 4.8±0.2°, 15.0±0.2°, 19.1±0.2°, 14.3±0.2° and 23.9±0.2°;
the X-ray powder diffraction pattern of the crystal form V comprises at least one or more diffraction peaks at 2θ angles of 4.7±0.2°, 18.2±0.2° or 10.1±0.2°; preferably comprises two of the above diffraction peaks, more preferably comprises three of the diffraction peaks; optionally, further also comprises at least one diffraction peak at 2θ angle of 18.8±0.2°, 15.6±0.2°, 17.0±0.2°, 21.8±0.2° or 14.7±0.2°, preferably comprises 2, 3, 4 or 5 of the above diffraction peaks;
or, comprises diffraction peaks at 2θ angles of 4.7±0.2°, 18.2±0.2°, 10.1±0.2°, 18.8±0.2, 15.6±0.2° and 17.0±0.2°;
or, comprises diffraction peaks at 2θ angles of 4.7±0.2°, 18.2±0.2°, 10.1±0.2°, 18.8±0.2, 15.6±0.2° and 21.8±0.2°;
or, comprises diffraction peaks at 2θ angles of 4.7±0.2°, 18.2±0.2°, 10.1±0.2°, 18.8±0.2, 15.6±0.2° and 14.7±0.2°.

8. The crystal form of the compound as claimed in any one of claims 6-7, wherein, the X-ray powder diffraction pattern of the crystal form I optionally also comprises one or more diffraction peaks at 2θ angles of 10.0±0.2°, 25.4±0.2°, 26.9±0.2°, 13.0±0.2°, 12.9±0.2° or 20.0±0.2°; preferably comprises at least any 2-3, or 4-5, or 6 of the above diffraction peaks; more preferably comprises any 2, 3, 4, 5 or 6 of the diffraction peaks;
or, comprises diffraction peaks at 2θ angles of 5.0±0.2°, 9.6±0.2°, 15.0±0.2°, 17.2±0.2°, 22.2±0.2°, 19.4±0.2°, 26.9±0.2° and 10.0±0.2°;
or, comprises diffraction peaks at 2θ angles of 5.0±0.2°, 9.6±0.2°, 15.0±0.2°, 17.2±0.2°, 22.2±0.2°, 19.4±0.2°, 26.9±0.2° and 25.4±0.2°;
the X-ray powder diffraction pattern of the crystal form I optionally also comprises one or more diffraction peaks at 2θ angles of 15.4±0.2°, 16.6±0.2°, 17.7±0.2°, 18.5±0.2°, 19.3±0.2° or 24.0±0.2°; preferably comprises at least any 2-3, or 4-5, or 6 of the above diffraction peaks; further preferably, comprises any 2, 3, 4, 5, 6 of the diffraction peaks;
or, comprises diffraction peaks at 2θ angles of 5.0±0.2°, 8.2±0.2°, 9.6±0.2°, 15.4±0.2°, 16.6±0.2°, 17.7±0.2°, 18.5±0.2° and 19.3±0.2°;
or, comprises diffraction peaks at 2θ angles of 5.0±0.2°, 8.2±0.2°, 9.6±0.2°, 16.6±0.2°, 17.7±0.2°, 18.5±0.2°, 19.3±0.2° and 24.0±0.2°;
the X-ray powder diffraction pattern of the crystal form II optionally also comprises one or more diffraction peaks at 2θ angles of 4.5±0.2°, 15.4±0.2°, 14.6±0.2°, 24.0±0.2°, 21.5±0.2°, 20.5±0.2° or 18.0±0.2°; preferably comprises at least any 2-3, or 4-5, or 6-7 of the above diffraction peaks; further preferably, comprises any 2, 3, 4, 5, 6, 7 of the diffraction peaks;
or, comprises diffraction peaks at 2θ angles of 4.8±0.2°, 17.7±0.2°, 16.6±0.2°, 9.4±0.2°, 18.4±0.2°, 19.1±0.2°, 4.5±0.2° and 15.4±0.2°;
or, comprises diffraction peaks at 2θ angles of 4.8±0.2°, 17.7±0.2°, 16.6±0.2°, 9.4±0.2°, 18.4±0.2°, 19.1±0.2°, 4.5±0.2° and 14.6±0.2°;
the X-ray powder diffraction pattern of the crystal form III optionally also comprises one or more diffraction peaks at 2θ angles of 19.5±0.2°, 23.6±0.2°, 26.1±0.2°, 22.0±0.2°, 20.2±0.2°, 21.1±0.2° or 27.4±0.2°; preferably comprises at least any 2-3, or 4-5, or 6-7 of the above diffraction peaks; further preferably, comprises any 2, 3, 4, 5, 6, 7 of the diffraction peaks;
or, comprises diffraction peaks at 2θ angles of 4.6±0.2°, 15.2±0.2°, 9.9±0.2°, 16.7±0.2°, 18.2±0.2°, 17.9±0.2°, 19.5±0.2° and 23.6±0.2°;
or, comprises diffraction peaks at 2θ angles of 4.6±0.2°, 15.2±0.2°, 9.9±0.2°, 16.7±0.2°, 18.2±0.2°, 17.9±0.2°, 19.5±0.2° and 26.1±0.2°;
the X-ray powder diffraction pattern of the crystal form IV optionally also comprises one or more diffraction peaks at 2θ angles of 30.3±0.2° or 9.6±0.2°; preferably comprises at least any 1-2 of the above diffraction peaks;
or, comprises diffraction peaks at 2θ angles of 5.0±0.2°, 4.8±0.2°, 15.0±0.2°, 19.1±0.2°, 14.3±0.2°, 10.0±0.2°, 23.9±0.2° and 30.3±0.2°;
or, comprises diffraction peaks at 2θ angles of 5.0±0.2°, 4.8±0.2°, 15.0±0.2°, 19.1±0.2°, 14.3±0.2°, 10.0±0.2°, 23.9±0.2° and 9.6±0.2°;
the X-ray powder diffraction pattern of the crystal form V optionally also comprises one or more diffraction peaks at 2θ angles of 19.3±0.2°, 25.8±0.2°, 15.2±0.2°, 17.8±0.2°, 20.4±0.2°, 23.5±0.2° or 25.6±0.2°; preferably comprises at least any 2-3, or 4-5, or 6-7 of the above diffraction peaks; further preferably, comprises any 2, 3, 4, 5, 6, 7 of the diffraction peaks;
or, comprises diffraction peaks at 2θ angles of 4.7±0.2°, 18.2±0.2°, 10.1±0.2°, 18.8±0.2°, 15.6±0.2°, 17.0±0.2°, 19.3±0.2° and 25.8±0.2°;
or, comprises diffraction peaks at 2θ angles of 4.7±0.2°, 18.2±0.2°, 10.1±0.2°, 18.8±0.2°, 15.6±0.2°, 17.0±0.2°, 19.3±0.2° and 15.2±0.2°.

9. The crystal form of the compound as claimed in claim 6, wherein, the X-ray powder diffraction pattern of the crystal form I comprises one or more diffraction peaks at 2θ angles of 5.0±0.2°, 9.6±0.2°, 15.0±0.2°, 17.2±0.2°, 22.2±0.2°, 19.4±0.2°, 30.2±0.2°, 25.1±0.2°, 10.0±0.2°, 25.4±0.2°, 26.9±0.2°, 13.0±0.2°, 12.9±0.2° or 20.0±0.2°; preferably, comprises any 4, 5, 6, 8 or 10 of the above diffraction peaks;
the X-ray powder diffraction pattern of the crystal form I has diffraction peaks at following 2θ angles of:
9.6±0.2°, 15.0±0.2° and 17.2±0.2°;
or, 5.0±0.2°, 9.6±0.2°, 15.0±0.2° and 17.2±0.2°;
or, 5.0±0.2°, 9.6±0.2°, 15.0±0.2° and 22.2±0.2°;
or, 5.0±0.2°, 9.6±0.2°, 15.0±0.2°, 17.2±0.2°, 22.2±0.2° and 25.1±0.2°;
the X-ray powder diffraction pattern of the crystal form I comprises one or more diffraction peaks at 2θ angles of 5.0±0.2°, 8.2±0.2°, 9.6±0.2°, 12.9±0.2°, 15.0±0.2°, 17.2±0.2°, 15.4±0.2°, 16.6±0.2°, 17.7±0.2°, 18.5±0.2°, 19.3±0.2° or 24.0±0.2°; preferably, comprises any 4, 5, 6, 8 or 10 of the above diffraction peaks;
the X-ray powder diffraction pattern of the crystal form I has diffraction peaks at following 2θ angles of:
8.2±0.2°, 9.6±0.2°, 12.9±0.2°, 15.0±0.2°, 17.2±0.2°, 15.4±0.2°, 16.6±0.2° and 17.7±0.2°;
or, 5.0±0.2°, 8.2±0.2°, 9.6±0.2°, 12.9±0.2°, 15.0±0.2°, 17.2±0.2°, 15.4±0.2°, 16.6±0.2°, 17.7±0.2° and 18.5±0.2°;
or, 8.2±0.2°, 9.6±0.2°, 12.9±0.2°, 15.0±0.2°, 17.2±0.2°, 15.4±0.2°, 16.6±0.2°, 17.7±0.2°, 18.5±0.2° and 19.3±0.2°;
the X-ray powder diffraction pattern of the crystal form II comprises one or more diffraction peaks at 2θ angles of 4.8±0.2°, 17.7±0.2°, 16.6±0.2°, 9.4±0.2°, 18.4±0.2°, 19.1±0.2°, 17.0±0.2°, 18.6±0.2°, 4.5±0.2°, 15.4±0.2°, 14.6±0.2°, 24.0±0.2°, 21.5±0.2°, 20.5±0.2° or 18.0±0.2°; preferably, comprises any 4, 5, 6, 8 or 10 of the above diffraction peaks;
the X-ray powder diffraction pattern of the crystal form II has diffraction peaks at following 2θ angles of:
4.8±0.2°, 17.7±0.2°, 16.6±0.2° and 9.4±0.2°;
or, 4.8±0.2°, 17.7±0.2°, 16.6±0.2° and 18.4±0.2°;
or, 4.8±0.2°, 17.7±0.2°, 16.6±0.2°, 9.4±0.2°, 18.4±0.2° and 20.5±0.2°;
or, 4.8±0.2°, 17.7±0.2°, 16.6±0.2°, 9.4±0.2°, 18.4±0.2°, 19.1±0.2°, 17.0±0.2° and 18.6±0.2°;
or, 4.8±0.2°, 17.7±0.2°, 16.6±0.2°, 9.4±0.2°, 18.4±0.2°, 19.1±0.2°, 17.0±0.2°, 18.6±0.2°, 4.5±0.2°, 14.6±0.2°;
the X-ray powder diffraction pattern of the crystal form III comprises one or more diffraction peaks at 2θ angles of 4.6±0.2°, 15.2±0.2°, 9.9±0.2°, 16.7±0.2°, 18.2±0.2°, 17.9±0.2°, 25.5±0.2°, 15.0±0.2°, 19.5±0.2°, 23.6±0.2°, 26.1±0.2°, 22.0±0.2°, 20.2±0.2°, 21.1±0.2° or 27.4±0.2°; preferably, comprises any 4, 5, 6, 8 or 10 of the above diffraction peaks;
the X-ray powder diffraction pattern of the crystal form III has diffraction peaks at following 2θ angles of:
4.6±0.2°, 15.2±0.2°, 9.9±0.2° and 16.7±0.2°;
or, 4.6±0.2°, 15.2±0.2°, 9.9±0.2° and 18.2±0.2°;
or, 4.6±0.2°, 15.2±0.2°, 16.7±0.2° and 18.2±0.2°;
or, 4.6±0.2°, 15.2±0.2°, 9.9±0.2°, 16.7±0.2°, 18.2±0.2° and 17.9±0.2°;
or, 4.6±0.2°, 15.2±0.2°, 9.9±0.2°, 16.7±0.2°, 18.2±0.2°, 17.9±0.2°, 26.1±0.2° and 22.0±0.2°;
or, 4.6±0.2°, 15.2±0.2°, 9.9±0.2°, 16.7±0.2°, 18.2±0.2°, 17.9±0.2°, 25.5±0.2°, 15.0±0.2°, 19.5±0.2° and 23.6±0.2°;
the X-ray powder diffraction pattern of the crystal form IV comprises one or more diffraction peaks at 2θ angles of 5.0±0.2°, 4.8±0.2°, 15.0±0.2°, 19.1±0.2°, 14.3±0.2°, 10.0±0.2°, 23.9±0.2°, 25.1±0.2°, 30.3±0.2° or 9.6±0.2°; preferably, comprises any 4, 5, 6, 8 or 10 of the above diffraction peaks;
the X-ray powder diffraction pattern of the crystal form IV has diffraction peaks at following 2θ angles of:
comprises diffraction peaks at 2θ angles of 5.0±0.2°, 4.8±0.2°, 15.0±0.2° and 19.1±0.2°;
or, 5.0±0.2°, 4.8±0.2°, 15.0±0.2° and 14.3±0.2°;
or, 5.0±0.2°, 4.8±0.2°, 15.0±0.2°, 19.1±0.2°, 23.9±0.2° and 25.1±0.2°;
or, 5.0±0.2°, 4.8±0.2°, 15.0±0.2°, 19.1±0.2°, 14.3±0.2°, 10.0±0.2°, 23.9±0.2° and 25.1±0.2°;
or, 5.0±0.2°, 4.8±0.2°, 15.0±0.2°, 19.1±0.2°, 14.3±0.2°, 10.0±0.2°, 23.9±0.2°, 25.1±0.2° and 30.3±0.2°;
or, 5.0±0.2°, 4.8±0.2°, 15.0±0.2°, 19.1±0.2°, 14.3±0.2°, 10.0±0.2°, 23.9±0.2°, 25.1±0.2°, 30.3±0.2° and 9.6±0.2°;
the X-ray powder diffraction pattern of the crystal form V comprises one or more diffraction peaks at 2θ angles of 4.7±0.2°, 18.2±0.2°, 10.1±0.2°, 18.8±0.2°, 15.6±0.2°, 17.0±0.2°, 21.8±0.2°, 14.7±0.2°, 19.3±0.2°, 25.8±0.2°, 15.2±0.2°, 17.8±0.2°, 20.4±0.2°, 23.5±0.2° or 25.6±0.2°; preferably, comprises any 4, 5, 6, 8 or 10 of the above diffraction peaks;
the X-ray powder diffraction pattern of the crystal form V has diffraction peaks at following 2θ angles of:
4.7±0.2°, 18.2±0.2°, 10.1±0.2° and 18.8±0.2°;
or, 4.7±0.2°, 10.1±0.2°, 18.8±0.2°, 15.6±0.2°, 17.0±0.2° and 21.8±0.2°;
or, 4.7±0.2°, 18.2±0.2°, 10.1±0.2°, 18.8±0.2°, 15.6±0.2°, 17.0±0.2°, 21.8±0.2° and
14.7±0.2°;
or, 4.7±0.2°, 18.2±0.2°, 10.1±0.2°, 18.8±0.2°, 15.6±0.2°, 17.0±0.2°, 21.8±0.2°, 14.7±0.2°, 19.3±0.2°, and 15.2±0.2°.

10. The crystal form of the compound as claimed in any one of claims 1-9, wherein, the X-ray powder diffraction pattern of the crystal form I is shown in FIG. 1; the X-ray powder diffraction pattern of the crystal form II is shown in FIG. 4; the X-ray powder diffraction pattern of the crystal form III is shown in FIG. 7; the X-ray powder diffraction pattern of the crystal form IV is shown in FIG. 10; the X-ray powder diffraction pattern of the crystal form V is shown in FIG. 13.

11. The crystal form of the compound as claimed in any one of claims 1-9, wherein, positions of diffraction peaks with relative peak intensity of top ten in the X-ray powder diffraction patterns of the crystal form I, crystal form II, crystal form III, crystal form IV, crystal form V and diffraction peaks at corresponding positions in the FIG. 1, FIG. 4, FIG. 7, FIG. 10, FIG. 13 have a 2θ error of ±0.2° to ±0.5°, preferably ±0.2° to ±0.3°, most preferably ±0.2°.

12. The crystal form of the compound as claimed in any one of claims 1-9, wherein,
the crystal form I has a DSC pattern as shown in FIG. 2; or has a TGA pattern as shown in FIG. 3;
the crystal form II has a DSC pattern as shown in FIG. 5; or has a TGA pattern as shown in FIG. 6;
the crystal form III has a DSC pattern as shown in FIG. 8; or has a TGA pattern as shown in FIG. 9;
the crystal form IV has a DSC pattern as shown in FIG. 11; or has a TGA pattern as shown in FIG. 12;
the crystal form V has a DSC pattern as shown in FIG. 14; or has a TGA pattern as shown in FIG. 15.

13. The crystal form of the compound as claimed in any one of claims 1-12, wherein, the crystal form is a solvent-containing or solvent-free crystal form, wherein the solvent is selected from one or more of water, methanol, acetone, ethyl acetate, acetonitrile, ethanol, 88% acetone, tetrahydrofuran, 2-methyl-tetrahydrofuran, dichloromethane, 1,4-dioxane, benzene, toluene, isopropanol, *n*-butanol, isobutanol, *N*,*N*-dimethylformamide, *N*,*N*-dimethylacetamide, *N-*methylpyrrolidone, dimethyl sulfoxide, *n*-propanol, *tert*-butanol, 2-butanone, 3-pentanone, *n-*heptane, heptane, ethyl formate, isopropyl acetate, cyclohexane, methyl *tert*-butyl ether or diisopropyl ether, preferably, the crystal form is an anhydrous crystal form.

14. The crystal form of the compound as claimed in claim 13, wherein, the number of the solvent is 0-3, preferably 0, 0.2, 0.5, 1, 1.5, 2, 2.5 or 3, more preferably 0, 0.5, 1, 2 or 3.

15. A method for preparing the crystal form of the compound as claimed in any one of claims 1-14, specifically comprising the following steps:
1) weighing an appropriate amount of a free base, and suspending with a poor solvent, preferably with a suspension density of 50 to 200 mg/mL;
2) shaking the suspension obtained above, preferably at a temperature of 0 to 50°C;
3) centrifuging the above suspension rapidly, removing the supernatant, and drying the remaining solid to obtain a target product;
wherein,
the poor solvent is selected from one or more of methanol, acetone, ethyl acetate, tetrahydrofuran, acetonitrile, ethanol, 88% acetone, 2-methyl-tetrahydrofuran, dichloromethane, 1,4-dioxane, methyl *tert*-butyl ether, *n*-heptane, benzene, toluene, chlorobenzene, isopropanol, *n*-butanol, isobutanol, *N*,*N*-dimethylformamide, *N,N-*dimethylacetamide, dimethyl sulfoxide, *n*-propanol, ethyl formate, isopropyl acetate, *tert-*butanol, 2-butanone or 3-pentanone;
preferably one or more of acetonitrile, acetone, ethyl acetate, methanol or ethanol.

16. A method for preparing the crystal form of the compound as claimed in any one of claims 1-14, specifically comprising the following steps:
1) weighing an appropriate amount of a free base and dissolving with a good solvent;
2) adding, optionally, a poor solvent to the solution obtained above;
3) stirring until the solid is precipitated, with a crystallization temperature selected from 0 to 50°C;
4) optionally, centrifuging the above suspension rapidly;
5) removing the supernatant, and drying the remaining solid to obtain a target product;
wherein,
the good solvent is selected from one or more of methanol, acetone, ethyl acetate, tetrahydrofuran, acetonitrile, ethanol, 88% acetone, 2-methyl-tetrahydrofuran, dichloromethane, 1,4-dioxane, benzene, toluene, chlorobenzene, isopropanol, n-butanol, isobutanol, *N*,*N*-dimethylformamide, *N*,*N*-dimethylacetamide, dimethyl sulfoxide, n-propanol, ethyl formate, isopropyl acetate, *tert*-butanol, 2-butanone or 3-pentanone;
preferably one or more of dichloromethane, tetrahydrofuran, 2-methyl-tetrahydrofuran, 1,4-dioxane, dimethyl sulfoxide, acetonitrile or 2-butanone;
the poor solvent is selected from one or more of methanol, ethanol, ethyl acetate, acetone, isopropanol, toluene, *n*-heptane, water, isopropyl acetate, cyclohexane, methyl *tert*-butyl ether or diisopropyl ether;
preferably one or more of water, *n*-heptane, cyclohexane or methyl *tert*-butyl ether.

17. A pharmaceutical composition comprising a therapeutically effective amount of each of the crystal forms of the compound of general formula as claimed in any one of claims 1-14, and one or more pharmaceutically acceptable carriers, excipients.

18. A use of the crystal form of the compound as claimed in any one of claims 1-14, or the pharmaceutical composition as claimed in claim 17 in the manufacture of a medicament of a RET inhibitor.

19. A use of the crystal form of the compound as claimed in any one of claims 1-14, or the pharmaceutical composition as claimed in claim 17 in the manufacture of a medicament for the treatment and/or prevention of a tumor, preferably, the tumor is selected from non-small cell lung cancer, fibrosarcoma, pancreatic tumor, medullary thyroid carcinoma, papillary thyroid tumor, soft tissue sarcoma, highly solid tumor, breast tumor and colon tumor.
